# EUROPÄISCHE PATENTANMELDUNG

(11) **EP 1 994 923 A2**
(43) Veröffentlichungstag der Anmeldung: **26.11.2008**
(21) Anmeldenummer: 08009496.4
(22) Anmeldetag: 23.05.2008
(51) Int. Cl.: A61K 8/49, A61K 8/73, A61K 8/97, A61K 36/03, A61Q 19/00

(54) **Kosmetische und dermatologische Zusammensetzungen gegen trockene Haut**

(30) Priorität: 23.05.2007 DE 102007024384
(71) Anmelder: Henkel AG & Co. KGaA, 40589 Düsseldorf (DE)
(72) Erfinder: Janssen, Frank, 41470 Neuss (DE); Anderheggen, Bernd, 42781 Haan (DE); Döring, Thomas, 41542 Dormagen (DE); Waldmann-Laue, Marianne, 40789 Monheim (DE)

(57) **Zusammenfassung**

Zusammensetzungen, die sich zur Vorbeugung vor und Behandlung von trockener Haut eignen und einen Wassertransport aus tieferen Hautschichten in die Oberhaut ermöglichen, enthalten in einem geeigneten kosmetischen oder dermatologischen Träger - jeweils bezogen auf das Gewicht der gesamten Zusammensetzung - 0,001 bis 10 Gew.-% mindestens eines Extraktes aus Laminaria Digitata und/oder 0,001 bis 10 Gew.-% mindestens eines 2-Mercapto-N^{α}N^{α}N^{α}-trialkyl-L-histidinium-Betains der Formel (I) 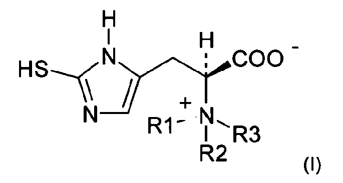 in der R1, R2 und R3 unabhängig voneinander ausgewählt sind aus -CH₃; -CH₂CH₃, -CH₂CH₂CH₃, - CH(CH₃)₂, -(CH₂)₃CH₃, -CH₂-CH(CH₃)₂, -CH(CH₃)CH₂CH₃, -C(CH₃)₃.

## Beschreibung

Die Haut ist das größte Organ des Menschen. Unter ihren vielen Funktionen (beispielsweise zur Wärmeregulation und als Sinnesorgan) ist die Barrierefunktion, diejenige die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, die als äußerste Schicht die eigentliche Schutzhülle gegenüber der Umwelt bildet. Mit etwa einem Zehntel der Gesamtdicke ist sie gleichzeitig die dünnste Schicht der Haut.
Die Epidermis ist ein stratifiziertes Gewebe, in dem die äußere Schicht, die Hornschicht (stratum corneum) den für die Barrierefunktion bedeutenden Teil darstellt.
Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell-und Lipidmaterial nachproduziert wird.
Das heute in der Fachwelt anerkannte Hautmodell von Elias beschreibt die Hornschicht als ZweiKomponenten-System, ähnlich einer Ziegelsteinmauer. In diesem Modell entsprechen die Hornzellen (Corneozyten) den Ziegelsteinen, die komplex zusammengesetzte Lipidmembran in den Interzellularräumen entspricht dem Mörtel. Dieses System stellt im Wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.
Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben. Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.
Die Lipide der Hornschicht bestehen im Wesentlichen aus Ceramiden, freien Fettsäuren, Cholesterin sowie Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.
Bereits bei einer Reinigung der Haut mit Hilfe eines einfachen Wasserbads- ohne Zusatz von Tensiden kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.
Bei gesunder Haut sind diese Vorgänge im Allgemeinen belanglos, da die Schutzmechanismen der Haut solche leichten Störungen der oberen Hautschichten ohne weiteres kompensieren können. Aber bereits im Fall nicht-pathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört. Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen. Trockene Haut weist einen Mangel an Barrierelipiden (Ceramide, Cholesterol und freie Fettsäuren) in der Hornschicht auf. Die verminderte Integrität und Reparaturleistung der Hautbarriere führt zu einem gesteigerten transepidermalen Wasserverlust. Die Haut wird rau, trocken, schuppig und neigt zu Spannungsgefühlen sowie Juckreiz.
Bei pathologisch trockener und empfindlicher Haut oder bei von atopischer Dermatitis betroffener Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.
Im Stand der Technik sind diverse Hautpflegeprodukte bekannt, die in der Lage sind, die epidermale Barrierefunktion zu verbessern. Diese Hautpflegeprodukte sind jedoch in der Regel okklusiver Natur, z. B. Vaseline und Vaseline-haltige Cremes, und weisen häufig ein klebriges Hautgefühl und/oder einen unangenehmen Tragekomfort auf. Die Salbe oder Creme stellt sozusagen eine (zweite) künstliche Barriere dar, die den Wasserverlust der Haut verhindern soll. Entsprechend leicht kann diese physikalische Barriere- beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Eine nachhaltige Produktwirkung wird in der Regel also nicht oder nur in einem eingeschränkten Masse erreicht. Um die Haut bei ihrer natürlichen Regeneration zu unterstützen und ihre physiologische Funktion zu stärken, werden den topischen Präparaten in neuerer Zeit zunehmend Interzellularlipidmischungen, wie Ceramide oder Ceramidanaloga, zugesetzt, die von der Haut zum Wiederaufbau der natürlichen Barriere verwendet werden sollen. Allerdings handelt es sich bei diesen Lipiden zumeist um sehr teure Rohstoffe, die außerdem aufgrund ihres hohen Schmelzpunktes schwierig zu formulieren sind. Zudem ist ihre Wirkung begrenzt, da die topisch applizierten Lipide nur in geringem Umfang in die barriererelevanten Lipidmembranen im mittleren Stratum corneum penetrieren und nur zu einem geringen Teil in diese Strukturen integriert werden.
Neben einer äußerlichen Versorgung der Haut mit Feuchtigkeit und/oder Feuchthaltemitteln kann auch der Wassertransport aus tieferen Hautschichten genutzt werden, um trockene Haut zu verhindern oder zu verringern.
Dabei kann Wasser nur in begrenztem Maße durch die Bilipidschicht der Zellmembran diffundieren. Sogenannte Aquaporine (abgekürzt AQP) sind Proteine, die Kanäle in der Zellmembran bilden, um den Durchtritt von Wassermolekülen zu erleichtern.
Die Aquaporine sind keine Pumpen oder Austauscher, und zum Transport wird keine Energie verbraucht. Aquaporine sind hoch selektiv. Vor allem muss verhindert werden, dass während des Wasserdurchtritts auch Protonen weitergeleitet werden und damit der für jede Zelle lebenswichtige Protonengradient zerstört wird. Wasser liegt in der flüssigen Phase nicht als Einzelmolekül vor, sondern als ein über Wasserstoffbrückenbindungen zusammenhängendes Netzwerk. Entlang dieser Wasserstoffbrücken können Protonen über den Grotthuss-Mechanismus transportiert werden.
Um diese Protonenwanderung beim Durchtritt von Wasser zu verhindern, ist die Pore des Kanals sanduhrförmig. Die engste Stelle ist mit 2,8 nm gerade breit genug, um einem einzelnen Wassermolekül den Durchtritt zu ermöglichen. Eine positive Ladung dieser Stelle der Kanalwand stößt weiterhin Protonen und kleinere Kationen zurück. Im weiteren Verlauf der Pore erzwingen zwei versetzt gegenüberliegende positive Ladungen die Drehung des durchtretenden Wassermoleküls und machen damit die Protonenweiterleitung unmöglich. Nur wenige Interaktionsstellen von H₂O und der Kanalwand ermöglichen einen schnellen Transport (bis zu 3x10⁹ Moleküle pro Sekunde). Der Kanal arbeitet bidirektional, d. h. Wasser kann in beiden Richtungen durch den Kanal wandern.
Der vorliegenden Erfindung lag die Aufgabe zugrunde, Zusammensetzungen bereitzustellen, die sich zur Vorbeugung vor und Behandlung von trockener Haut eignen. Dabei sollte auf den Einsatz herkömmlicher Feuchthaltemittel von außen verzichtet werden können. Durch Stimulierung der Aquaporinsynthese sollte zudem ein Wassertransport aus tieferen Hautschichten in die Oberhaut ermöglicht werden.

Es wurde überraschend gefunden, dass bestimmte Histidinium-Betaine und Extrakte von Laminaria Digitata die Aquaporinsynthese stimulieren und bei topischer Anwendung gegen trockene Haut wirken. Mischungen der beiden Substanzen sind dabei besonders effektiv.

### Stand der Technik

Ergothionein ist im bislang nicht im Zusammenhang mit der Regulierung des Wasserhaushalts oder gar einer Stimulierung der Aquaporinsynthese bekannt. Gemäß CTFA-Katalog ist Ergothionein ein typischer antioxidativer Wirkstoff. US 7354956 offenbart Ergothionein als Antiglycationswirkstoff. US 7268148 offenbart Ergothionein als Inhibitor von Matrixmetalloproteinasen, also als Wirkstoff, der den Abbau von Collagen und anderen Proteinen verlangsamt. Gemäß US 7122211 wird Ergothionein zur Reparatur UV-geschädigter Haut eingesetzt. Gemäß FR 2865398 dient eine Kombination ausgewählter Aminosäuren, darunter Ergothionein, mit Mannitol zum Zellschutz gegen UV-Schäden, bedingt durch die bekannte antioxidative Wirkung dieser Aminosäuren. Gemäß DE 4139639 ist Ergothionein als Bestandteil eines synthetischen Organextrakts offenbart, der zur Wundheilung und zur Stärkung des Immunsystems eingesetzt wird. DE 10113446 und DE 10243626 offenbaren Betaine, darunter Ergothionein, als Haarwuchs-fördernden Wirkstoff. JP 61155302 A offenbart die Kombination von Ergothionein und Plazentaextrakt zur Hautaufhellung.

Gegenstand der vorliegenden Erfindung ist in einer ersten Ausführungsform die Verwendung mindestens eines 2-Mercapto-N^{α}N^{α}N^{α}-trialkyl-L-histidinium-Betains der Formel (I) 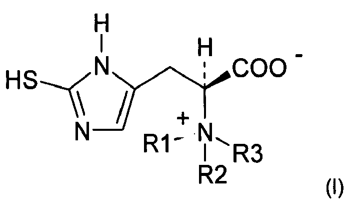 in der R1, R2 und R3 unabhängig voneinander ausgewählt sind aus -CH₃; -CH₂CH₃, -CH₂CH₂CH₃, - CH(CH₃)₂, -(CH₂)₃CH₃, -CH₂-CH(CH₃)₂, -CH(CH₃)CH₂CH₃, -C(CH₃)₃, wobei bevorzugt 2-Mercapto-N^{α}N^{α}N^{α}-trimethyl-L-histidinium-Betain der Formel (la) 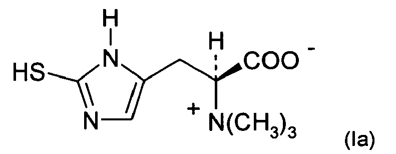 verwendet wird,
und/oder die Verwendung mindestens eines Extraktes aus Laminaria Digitata zur Regulierung und/oder Verbesserung des Wasserhaushalts der Haut.
Ein weiterer Gegenstand der vorliegenden Anmeldung ist die Verwendung mindestens eines 2-Mercapto-N^{α}N^{α}N^{α}-trialkyl-L-histidinium-Betains der Formel (I) 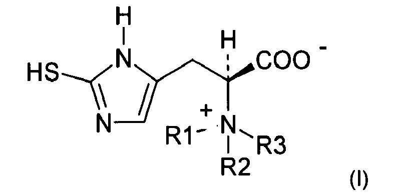 in der R1, R2 und R3 unabhängig voneinander ausgewählt sind aus -CH₃; -CH₂CH₃, -CH₂CH₂CH₃, - CH(CH₃)₂, -(CH₂)₃CH₃, -CH₂-CH(CH₃)₂, -CH(CH₃)CH₂CH₃, -C(CH₃)₃, wobei bevorzugt 2-Mercapto-N^{α}N^{α}N^{α}-trimethyl-L-histidinium-Betain der Formel (la) 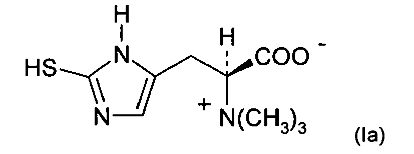 verwendet wird,
und/oder die Verwendung mindestens eines Extraktes aus Laminaria Digitata zur Stimulierung der Aquaporinsynthese.
Ein weiterer Gegenstand der vorliegenden Anmeldung ist die Verwendung mindestens eines Laminarins der Formel (II), 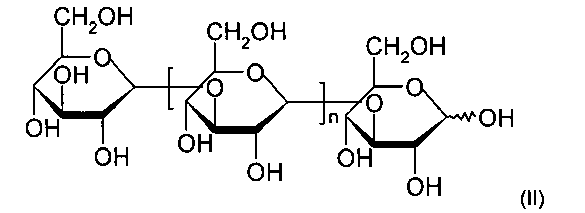 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind, zur Regulierung und/oder Verbesserung des Wasserhaushalts der Haut.
Ein weiterer Gegenstand der vorliegenden Anmeldung ist die Verwendung mindestens eines Laminarins der Formel (II), 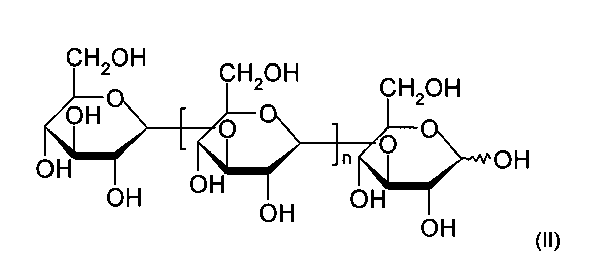 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind, zur Stimulierung der Aquaporinsynthese.

Bevorzugt dienen die vorgenannten Verwendungen zur nicht-therapeutischen, kosmetischen Behandlung trockener Haut.

Ein weiterer Gegenstand der vorliegenden Anmeldung ist ein nicht-therapeutisches Verfahren zur Regulierung und/oder zur Verbesserung des Wasserhaushalts der Haut und/oder zur Stimulierung der Aquaporinsynthese, bei dem mindestens ein 2-Mercapto-N^{α}N^{α}N^{α}-trialkyl-L-histidinium-Betain der Formel (I),

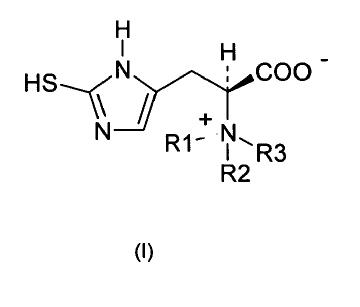

in der R1, R2 und R3 unabhängig voneinander ausgewählt sind aus -CH₃; -CH₂CH₃, -CH₂CH₂CH₃, - CH(CH₃)₂, -(CH₂)₃CH₃, -CH₂-CH(CH₃)₂, -CH(CH₃)CH₂CH₃, -C(CH₃)₃, bevorzugt 2-Mercapto-N^{α}N^{α}N^{α}-trimethyl-L-histidinium-Betain der Formel (la), 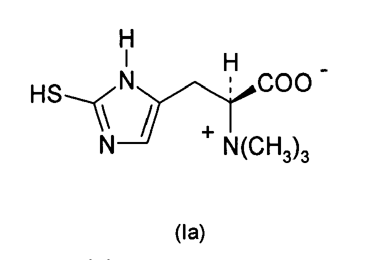 auf die Haut aufgetragen wird.

Ein weiterer Gegenstand der vorliegenden Anmeldung ist ein nicht-therapeutisches Verfahren zur Regulierung und/oder zur Verbesserung des Wasserhaushalts der Haut und/oder zur Stimulierung der Aquaporinsynthese, bei dem mindestens ein Extrakt aus Laminaria digitata auf die Haut aufgetragen wird.

Ein weiterer Gegenstand der vorliegenden Anmeldung ist ein nicht-therapeutisches Verfahren zur Regulierung und/oder zur Verbesserung des Wasserhaushalts der Haut und/oder zur Stimulierung der Aquaporinsynthese, bei dem mindestens ein Laminarin der Formel (II), 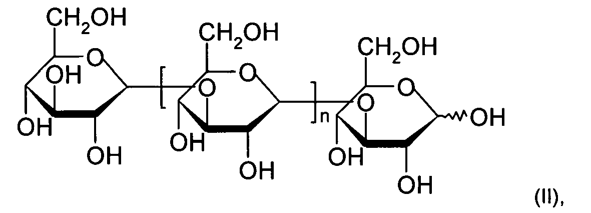 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
auf die Haut aufgetragen wird.

Bevorzugt dienen die vorstehend genannten nicht-therapeutischen Verfahren zur nicht-therapeutischen, kosmetischen Behandlung trockener Haut.

Ein weiterer bevorzugter Gegenstand der vorliegenden Anmeldung ist ein nicht-therapeutisches Verfahren wobei das mindestens eine 2-Mercapto-N^{α}N^{α}N^{α}-trialkyl-L-histidinium-Betain der Formel (I), 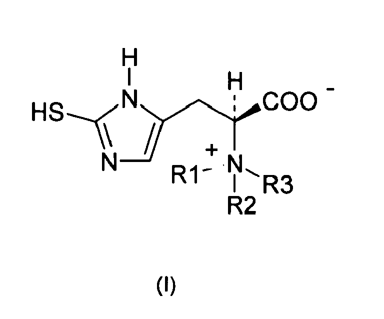 in der R1, R2 und R3 unabhängig voneinander ausgewählt sind aus -CH₃; -CH₂CH₃, -CH₂CH₂CH₃, - CH(CH₃)₂, -(CH₂)₃CH₃, -CH₂-CH(CH₃)₂, -CH(CH₃)CH₂CH₃, -C(CH₃)₃, bevorzugt 2-Mercapto-N^{α}N^{α}N^{α}-trimethyl-L-histidinium-Betain der Formel (la), 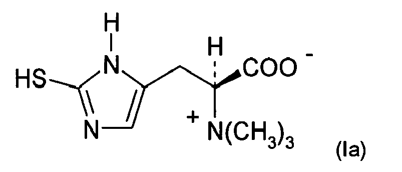 und/oder der mindestens eine Extrakt aus Laminaria Digitata
jeweils in einer Gesamtmenge von 0,001 bis 10 Gew.-%, bezogen auf das Gewicht der gesamten Zusammensetzung, in einem geeigneten kosmetischen oder dermatologischen Träger enthalten ist/sind.

Ein weiterer bevorzugter Gegenstand der vorliegenden Anmeldung ist ein nicht-therapeutisches Verfahren bei dem das mindestens eine Laminarin der Formel (II), 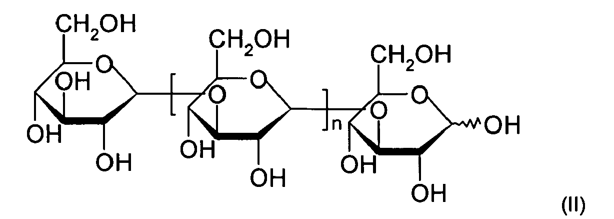 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
in einer Gesamtmenge von 00005 bis 0,5 Gew.-%, vorzugsweise 0,0005 bis 0,25 Gew.-% und insbesondere 0,005 bis 0,1 Gew.-%, jeweils bezogen auf das Gewicht der gesamten Zusammensetzung, in einem geeigneten kosmetischen oder dermatologischen Träger enthalten ist.

Als wesentlichen Inhaltsstoffe enthalten die erfindungsgemäß verwendeten Zubereitungen - jeweils bezogen auf ihr Gewicht - 0,001 bis 10 Gew.-% mindestens eines Extraktes aus Laminaria Digitata und/oder 0,001 bis 10 Gew.-% mindestens eines 2-Mercapto-N^{α}N^{α}N^{α}-trialkyl-L-histidinium-Betains der Formel (I), 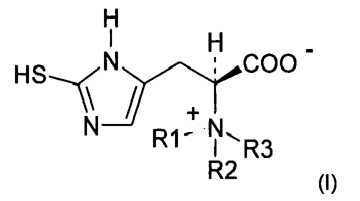 in der R1, R2 und R3 unabhängig voneinander ausgewählt sind aus -CH₃; -CH₂CH₃, -CH₂CH₂CH₃, - CH(CH₃)₂, -(CH₂)₃CH₃, -CH₂-CH(CH₃)₂, -CH(CH₃)CH₂CH₃, -C(CH₃)₃.

Die erfindungsgemäß verwendeten Zusammensetzungen können mindestens einen Extrakt aus Laminaria Digitata enthalten. Die Laminariales sind eine Ordnung der Braunalgen, deren Mitglieder unter Wasser im klaren, flachen Meer Wälder bilden. Mehrere Gattungen werden als Kelp bezeichnet, der Rest mit dem allgemeinen Wort "Tang". Die Art Laminaria Digitata wird auch als Fingertang, Blatt- oder Riementang bezeichnet.

Die Extraktion von Laminaria Digitata liefert Laminarin als Hauptbestandteil (ca. 40 g pro kg Pflanzenmaterial). Laminarin ist ein lineares Glucan mit beta-1,3-glycosidischen und einzelnen beta-1,6-glycosidischen Bindungen. Erfindungsgemäß bevorzugt verwendete Zusammensetzungen enthalten Laminarin. Erfindungsgemäß besonders bevorzugt verwendete Zusammensetzungen sind dadurch gekennzeichnet, dass sie 0,00005 bis 0,5 Gew.-%, vorzugsweise 0,0005 bis 0,25 Gew.-% und insbesondere 0,005 bis 0,1 Gew.-% Laminarin der Formel (II) enthalten 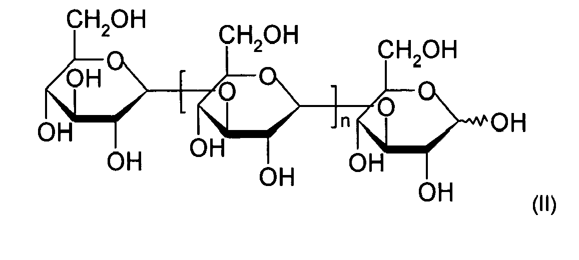 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind.

Zusätzlich zu dem bzw. den Extrakt(en) aus Laminaria Digitata und/oder zu Laminarin können die erfindungsgemäß verwendeten Mittel - bezogen auf ihr Gewicht - 0,001 bis 10 Gew.-% mindestens eines 2-Mercapto-N^{α}N^{α}N^{α}-trialkyl-L-histidinium-Betains der Formel (I), 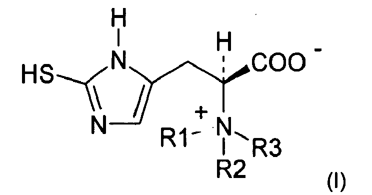 in der R1, R2 und R3 unabhängig voneinander ausgewählt sind aus -CH₃; -CH₂CH₃, -CH₂CH₂CH₃, -CH(CH₃)₂, -(CH₂)₃CH₃, -CH₂-CH(CH₃)₂, -CH(CH₃)CH₂CH₃, -C(CH₃)₃, enthalten.

Bevorzugte Verbindungen der Formel (I) sind in der nachfolgenden Tabelle wiedergegeben:

| **Nr.** | **R1** | **R2** | **R3** |
|---|---|---|---|
| 1. | -CH₃ | -CH₃ | -CH₃ |
| 2. | -CH₃ | -CH₃ | -CH₂CH₃ |
| 3. | -CH₃ | -CH₃ | -CH₂CH₂CH₃ |
| 4. | -CH₃ | -CH₃ | -CH(CH₃)₂ |
| 5. | -CH₃ | -CH₃ | -(CH₂)₃CH₃ |
| 6. | -CH₃ | -CH₃ | -CH₂-CH(CH₃)₂ |
| 7. | -CH₃ | -CH₃ | -CH(CH₃)CH₂CH₃ |
| 8. | -CH₃ | -CH₃ | -C(CH₃)₃ |
| 9. | -CH₃ | -CH₂CH₃ | -CH₂CH₃ |
| 10. | -CH₃ | -CH₂CH₃ | -CH₂CH₂CH₃ |
| 11. | -CH₃ | -CH₂CH₃ | -CH(CH₃)₂ |
| 12. | -CH₃ | -CH₂CH₃ | -(CH₂)₃CH₃ |
| 13. | -CH₃ | -CH₂CH₃ | -CH₂-CH(CH₃)₂ |
| 14. | -CH₃ | -CH₂CH₃ | -CH(CH₃)CH₂CH₃ |
| 15. | -CH₃ | -CH₂CH₃ | -C(CH₃)₃ |
| 16. | -CH₃ | -CH₂CH₂CH₃ | -CH₂CH₂CH₃ |
| 17. | -CH₃ | -CH₂CH₂CH₃ | -CH(CH₃)₂ |
| 18. | -CH₃ | -CH₂CH₂CH₃ | -(CH₂)₃CH₃ |
| 19. | -CH₃ | -CH₂CH₂CH₃ | -CH₂-CH(CH₃)₂ |
| 20. | -CH₃ | -CH₂CH₂CH₃ | -CH(CH₃)CH₂CH₃ |
| 21. | -CH₃ | -CH₂CH₂CH₃ | -C(CH₃)₃ |
| 22. | -CH₃ | -CH(CH₃)₂ | -CH(CH₃)₂ |
| 23. | -CH₃ | -CH(CH₃)₂ | -(CH₂)₃CH₃ |
| 24. | -CH₃ | -CH(CH₃)₂ | -CH₂-CH(CH₃)₂ |
| 25. | -CH₃ | -CH(CH₃)₂ | -CH(CH₃)CH₂CH₃ |
| 26. | -CH₃ | -CH(CH₃)₂ | -C(CH₃)₃ |
| 27. | -CH₃ | -(CH₂)₃CH₃ | -(CH₂)₃CH₃ |
| 28. | -CH₃ | -(CH₂)₃CH₃ | -CH₂-CH(CH₃)₂ |
| 29. | -CH₃ | -(CH₂)₃CH₃ | -CH(CH₃)CH₂CH₃ |
| 30. | -CH₃ | -(CH₂)₃CH₃ | -C(CH₃)₃ |
| 31. | -CH₃ | -CH₂-CH(CH₃)₂ | -CH₂-CH(CH₃)₂ |
| 32. | -CH₃ | -CH₂-CH(CH₃)₂ | -CH(CH₃)CH₂CH₃ |
| 33. | -CH₃ | -CH₂-CH(CH₃)₂ | -C(CH₃)₃ |
| 34. | -CH₃ | -CH(CH₃)CH₂CH₃ | -CH(CH₃)CH₂CH₃ |
| 35. | -CH₃ | -CH(CH₃)CH₂CH₃ | -C(CH₃)₃ |
| 36. | -CH₃ | -C(CH₃)₃ | -C(CH₃)₃ |
| 37. | -CH₂CH₃ | -CH₂CH₃ | -CH₂CH₃ |
| 38. | -CH₂CH₃ | -CH₂CH₃ | -CH₂CH₂CH₃ |
| 39. | -CH₂CH₃ | -CH₂CH₃ | -CH(CH₃)₂ |
| 40. | -CH₂CH₃ | -CH₂CH₃ | -(CH₂)₃CH₃ |
| 41. | -CH₂CH₃ | -CH₂CH₃ | -CH₂-CH(CH₃)₂ |
| 42. | -CH₂CH₃ | -CH₂CH₃ | -CH(CH₃)CH₂CH₃ |
| 43. | -CH₂CH₃ | -CH₂CH₃ | -C(CH₃)₃ |
| 44. | -CH₂CH₃ | -CH₂CH₂CH₃ | -CH₂CH₂CH₃ |
| 45. | -CH₂CH₃ | -CH₂CH₂CH₃ | -CH(CH₃)₂ |
| 46. | -CH₂CH₃ | -CH₂CH₂CH₃ | -(CH₂)₃CH₃ |
| 47. | -CH₂CH₃ | -CH₂CH₂CH₃ | -CH₂-CH(CH₃)₂ |
| 48. | -CH₂CH₃ | -CH₂CH₂CH₃ | -CH(CH₃)CH₂CH₃ |
| 49. | -CH₂CH₃ | -CH₂CH₂CH₃ | -C(CH₃)₃ |
| 50. | -CH₂CH₃ | -CH(CH₃)₂ | -CH(CH₃)₂ |
| 51. | -CH₂CH₃ | -CH(CH₃)₂ | -(CH₂)₃CH₃ |
| 52. | -CH₂CH₃ | -CH(CH₃)₂ | -CH₂-CH(CH₃)₂ |
| 53. | -CH₂CH₃ | -CH(CH₃)₂ | -CH(CH₃)CH₂CH₃ |
| 54. | -CH₂CH₃ | -CH(CH₃)₂ | -C(CH₃)₃ |
| 55. | -CH₂CH₃ | -(CH₂)₃CH₃ | -(CH₂)₃CH₃ |
| 56. | -CH₂CH₃ | -(CH₂)₃CH₃ | -CH₂-CH(CH₃)₂ |
| 57. | -CH₂CH₃ | -(CH₂)₃CH₃ | -CH(CH₃)CH₂CH₃ |
| 58. | -CH₂CH₃ | -(CH₂)₃CH₃ | -C(CH₃)₃ |
| 59. | -CH₂CH₃ | -CH₂-CH(CH₃)₂ | -CH₂-CH(CH₃)₂ |
| 60. | -CH₂CH₃ | -CH₂-CH(CH₃)₂ | -CH(CH₃)CH₂CH₃ |
| 61. | -CH₂CH₃ | -CH₂-CH(CH₃)₂ | -C(CH₃)₃ |
| 62. | -CH₂CH₃ | -CH(CH₃)CH₂CH₃ | -CH(CH₃)CH₂CH₃ |
| 63. | -CH₂CH₃ | -CH(CH₃)CH₂CH₃ | -C(CH₃)₃ |
| 64. | -CH₂CH₃ | -C(CH₃)₃ | -C(CH₃)₃ |
| 65. | -CH₂CH₂CH₃ | -CH₂CH₂CH₃ | -CH₂CH₂CH₃ |
| 66. | -CH₂CH₂CH₃ | -CH₂CH₂CH₃ | -CH(CH₃)₂ |
| 67. | -CH₂CH₂CH₃ | -CH₂CH₂CH₃ | -(CH₂)₃CH₃ |
| 68. | -CH₂CH₂CH₃ | -CH₂CH₂CH₃ | -CH₂-CH(CH₃)₂ |
| 69. | -CH₂CH₂CH₃ | -CH₂CH₂CH₃ | -CH(CH₃)CH₂CH₃ |
| 70. | -CH₂CH₂CH₃ | -CH₂CH₂CH₃ | -C(CH₃)₃ |
| 71. | -CH₂CH₂CH₃ | -CH(CH₃)₂ | -CH(CH₃)₂ |
| 72. | -CH₂CH₂CH₃ | -CH(CH₃)₂ | -(CH₂)₃CH₃ |
| 73. | -CH₂CH₂CH₃ | -CH(CH₃)₂ | -CH₂-CH(CH₃)₂ |
| 74. | -CH₂CH₂CH₃ | -CH(CH₃)₂ | -CH(CH₃)CH₂CH₃ |
| 75. | -CH₂CH₂CH₃ | -CH(CH₃)₂ | -C(CH₃)₃ |
| 76. | -CH₂CH₂CH₃ | -(CH₂)₃CH₃ | -(CH₂)₃CH₃ |
| 77. | -CH₂CH₂CH₃ | -(CH₂)₃CH₃ | -CH₂-CH(CH₃)₂ |
| 78. | -CH₂CH₂CH₃ | -(CH₂)₃CH₃ | -CH(CH₃)CH₂CH₃ |
| 79. | -CH₂CH₂CH₃ | -(CH₂)₃CH₃ | -C(CH₃)₃ |
| 80. | -CH₂CH₂CH₃ | -CH₂-CH(CH₃)₂ | -CH₂-CH(CH₃)₂ |
| 81. | -CH₂CH₂CH₃ | -CH₂-CH(CH₃)₂ | -CH(CH₃)CH₂CH₃ |
| 82. | -CH₂CH₂CH₃ | -CH₂-CH(CH₃)₂ | -C(CH₃)₃ |
| 83. | -CH₂CH₂CH₃ | -CH(CH₃)CH₂CH₃ | -CH(CH₃)CH₂CH₃ |
| 84. | -CH₂CH₂CH₃ | -CH(CH₃)CH₂CH₃ | -C(CH₃)₃ |
| 85. | -CH₂CH₂CH₃ | -C(CH₃)₃ | -C(CH₃)₃ |
| 86. | -CH(CH₃)₂ | -CH₂-CH(CH₃)₂ | -CH₂-CH(CH₃)₂ |
| 87. | -CH(CH₃)₂ | -CH₂-CH(CH₃)₂ | -CH(CH₃)CH₂CH₃ |
| 88. | -CH(CH₃)₂ | -CH₂-CH(CH₃)₂ | -C(CH₃)₃ |
| 89. | -CH(CH₃)₂ | -CH(CH₃)CH₂CH₃ | -CH(CH₃)CH₂CH₃ |
| 90. | -CH(CH₃)₂ | -CH(CH₃)CH₂CH₃ | -C(CH₃)₃ |
| 91. | -CH(CH₃)₂ | -C(CH₃)₃ | -C(CH₃)₃ |
| 92. | -(CH₂)₃CH₃ | -(CH₂)₃CH₃ | -(CH₂)₃CH₃ |
| 93. | -(CH₂)₃CH₃ | -(CH₂)₃CH₃ | -CH₂-CH(CH₃)₂ |
| 94. | -(CH₂)₃CH₃ | -(CH₂)₃CH₃ | -CH(CH₃)CH₂CH₃ |
| 95. | -(CH₂)₃CH₃ | -(CH₂)₃CH₃ | -C(CH₃)₃ |
| 96. | -(CH₂)₃CH₃ | -CH₂-CH(CH₃)₂ | -CH₂-CH(CH₃)₂ |
| 97. | -(CH₂)₃CH₃ | -CH₂-CH(CH₃)₂ | -CH(CH₃)CH₂CH₃ |
| 98. | -(CH₂)₃CH₃ | -CH₂-CH(CH₃)₂ | -C(CH₃)₃ |
| 99. | -(CH₂)₃CH₃ | -CH(CH₃)CH₂CH₃ | -CH(CH₃)CH₂CH₃ |
| 100. | -(CH₂)₃CH₃ | -CH(CH₃)CH₂CH₃ | -C(CH₃)₃ |
| 101. | -(CH₂)₃CH₃ | -C(CH₃)₃ | -C(CH₃)₃ |
| 102. | -CH₂-CH(CH₃)₂ | -CH₂-CH(CH₃)₂ | -CH₂-CH(CH₃)₂ |
| 103. | -CH₂-CH(CH₃)₂ | -CH₂-CH(CH₃)₂ | -CH(CH₃)CH₂CH₃ |
| 104. | -CH₂-CH(CH₃)₂ | -CH₂-CH(CH₃)₂ | -C(CH₃)₃ |
| 105. | -CH₂-CH(CH₃)₂ | -CH(CH₃)CH₂CH₃ | -CH(CH₃)CH₂CH₃ |
| 106. | -CH₂-CH(CH₃)₂ | -CH(CH₃)CH₂CH₃ | -C(CH₃)₃ |
| 107. | -CH₂-CH(CH₃)₂ | -C(CH₃)₃ | -C(CH₃)₃ |
| 108. | -CH(CH₃)CH₂CH₃ | -CH(CH₃)CH₂CH₃ | -CH(CH₃)CH₂CH₃ |
| 109. | -CH(CH₃)CH₂CH₃ | -CH(CH₃)CH₂CH₃ | -C(CH₃)₃ |
| 110. | -CH(CH₃)CH₂CH₃ | -C(CH₃)₃ | -C(CH₃)₃ |
| 111. | -C(CH₃)₃ | -CH(CH₃)CH₂CH₃ | -C(CH₃)₃ |
| 112. | -C(CH₃)₃ | -C(CH₃)₃ | -C(CH₃)₃ |

Besonders bevorzugte erfindungsgemäß verwendete Zusammensetzungen sind dadurch gekennzeichnet, dass sie 0,001 bis 10 Gew.-%, vorzugsweise 0,01 bis 5 Gew.-% und insbesondere 0,1 bis 2 Gew.-% mindestens eines 2-Mercapto-N^{α}N^{α}N^{α}-trialkyl-L-histidinium-Betains der Formel (I) enthalten, 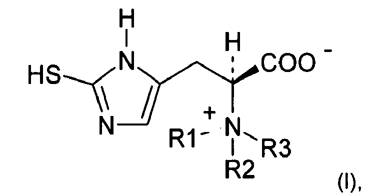 in der R1, R2 und R3 unabhängig voneinander ausgewählt sind aus -CH₃; -CH₂CH₃, -CH₂CH₂CH₃, -CH(CH₃)₂, wobei bevorzugte Zusammensetzungen 2-Mercapto-N^{α}N^{α}N^{α}-trimethyl-L-histidinium-Betain der Formel (la) 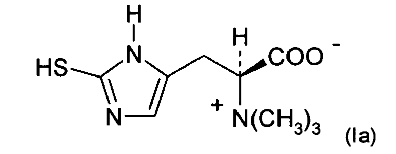 enthalten.

Wie bereits erwähnt, enthalten die erfindungsgemäß verwendeten Zusammensetzungen vorzugsweise weitere Inhaltstoffe. Im Hinblick auf die Behandlung trockener, rissiger Haut haben sich bestimmte weitere Wirksubstanzen als besonders bevorzugt erwiesen, da sie hautpflegende und hautberuhigende Eigenschaften haben und in ihrer Wirkung durch die erfindungsgemäß eingesetzten Hauptwirksubstanzen verstärkt werden.

Erfindungsgemäß besonders bevorzugte Zusammensetzungen enthalten zusätzlich zu mindestens einer der vorstehend erläuterten Komponenten (I), (la) oder (II) mindestens einen Wirkstoff, ausgewählt aus:
a) Monomeren, Oligomeren und Polymeren von Aminosäuren, N-C₂-C₂₄-Acylaminosäuren, den Estern und/oder den physiologisch verträglichen Metallsalzen dieser Substanzen,
b) DNA- oder RNA-Oligonucleotiden,
c) natürlichen Betainverbindungen (ohne Ergothionein),
d) Vitaminen, Provitaminen und Vitaminvorstufen der Gruppen A, B, C, E, H und K und den Estern der vorgenannten Substanzen,
e) α-Hydroxycarbonsäuren, α-Ketocarbonsäuren, β-Hydroxycarbonsäuren und deren Ester-, Lacton- oder Salzform,
f) den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
g) Isoflavonoiden und Isoflavonoid-reichen Pflanzenextrakten,
h) Ubichinon und Ubichinol sowie deren Derivaten,
i) Silymarin,
j) natürlich vorkommenden Xanthin-Derivaten, ausgewählt aus Coffein, Theophyllin, Theobromin und Aminophyllin,
k) Ectoin,
l) Kreatin,
m) Olivenblattextrakten, Ursolsäure, Oleanol und/oder Oleanolsäure,
n) Mono- und Polyhydroxystilbenen und deren Estern,
o) Derivaten von methyliertem Silanol,
p) Phytinsäure,
q) Extrakten aus Maiskörnern (Zea Mays (Corn) Kernel Extract),
r) Extrakten aus Haferkörnern (Avena Sativa (Oat) Kernel Extract),
s) Saccharomyces/Xylinum/Black Tea Ferment,
t) Apfelkernextrakten (Pyrus Malus (Apple) Fruit Extract),
u) Lotuskeim-Extrakten (Nelumbo Nucifera Germ Extract),
v) Rotweinextrakten,
w) Traubenkernextrakten (Vitis Vinifera (Grape) Seed Extract), die bevorzugt aus der Chardonnay-Traube stammen,
x) Extrakten aus Schwarzen Holunderblüten (Sambucus Nigra Flower Extract),
y) Wirkstoffen, die die beta-Endorphinsynthese in Keratinozyten stimulieren,
z) anorganischen und organischen UV-Filtersubstanzen,
aa) selbstbräunenden Wirkstoffen,
bb) hautaufhellenden Wirkstoffen,
cc) Wirkstoffen, die die Prostaglandinsynthese und/oder die Leukotrien-Synthese inhibieren,
dd) sebumregulierenden Wirkstoffen,
ee) sowie Desoxyzuckern oder Desoxyzucker-Bausteine enthaltenden Polysacchariden. enthält.

In einer ersten bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein Monomer, Oligomer oder Polymer von Aminosäuren, N-C₂-C₂₄-Acylaminosäuren und/oder den Estern und/oder den physiologisch verträglichen Metallsalzen dieser Substanzen.
Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Glutamin, Glutaminsäure, Glycin, Histidin, Hydroxylysin, Hydroxyprolin, Isodesmosin, Isoleucin, Leucin, Lysin, Methionin, Ornithin, Phenylalanin, Prolin, Pyroglutaminsäure, Sarcosin, Serin, Taurin, Threonin, Thyroxin, Tryptophan, Tyrosin und Valin. Besonders bevorzugt sind Taurin, Arginin, Hydroxyprolin, Prolin und Glutaminsäure.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Bevorzugte Beispiele sind Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Zinkpyroglutamat und Natriumlauroylglutamat.
Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Die Oligomere der Aminosäuren und/oder der N-C₂-C₂₄-Acylaminosäuren sind ausgewählt aus Di-, Tri- , Tetra-, Penta-, Hexa-, Hepta-, Octa-, Nona- und Decapeptiden, die acyliert und/oder verestert sein können. Besonders bevorzugt sind die Di-, Tri-, Tetra-, Penta- und Hexapeptide, die acyliert und/oder verestert sein können. Erfindungsgemäß bevorzugte, gegebenfalls acylierte und/oder veresterte Dipeptide sind Tyr-Arg, Val-Trp, Asn-Phe, Asp-Phe, N-Palmitoyl-β-Ala-His, N-Acetyl-Tyr-Arghexyldecylester (z.B. Calmosensine von Sederma), Carnosin (β-Ala-His) und N-Palmitoyl-Pro-Arg. Erfindungsgemäß bevorzugte, gegebenfalls acylierte und/oder veresterte Tripeptide sind Gly-His-Lys, N-Palmitoyl-Gly-His-Lys, Gly-Lys-His, His-Ala-Orn, Lys-Phe-Lys, N-Elaidoyl-Lys-Phe-Lys und N-Acetyl-Arg-Lys-Arg-NH₂. Erfindungsgemäß bevorzugte, gegebenfalls acylierte und/oder veresterte Tetrapeptide sind Gly-Gln-Pro-Arg, Gly-Gln-Arg-Pro und N-Palmitoyl-Gly-Gln-Pro-Arg. Erfindungsgemäß bevorzugte, gegebenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met und N-Palmitoyl-Tyr-Gly-Gly-Phe-Leu. Ein erfindungsgemäß bevorzugtes Hexapeptid ist Palmitoyl-Val-Gly-Val-Ala-Pro-Gly (Biopeptide EL von Sederma).

Es kann erfindungsgemäß besonders bevorzugt sein, ein Gemisch aus mindestens zwei Oligopeptiden einzusetzen. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z.B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich.

Die Polymere der Aminosäuren und/oder der N-C₂-C₂₄-Acylaminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine^{®} von Coletica oder Ridulisse C^{®} von Silab.
Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI-Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans*, einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome^{™}, liposomenverkapselte T4N5 z. B. unter der Bezeichnung Ultrasome^{™} von der Firma AGI Dermatics, USA, erhältlich.
In den erfindungsgemäßen Zusammensetzungen sind die Photosome^{™} oder Ultrasome^{™} in Mengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.
In den erfindungsgemäßen Zusammensetzungen sind die Monomere, Oligomere oder Polymere von Aminosäuren, N-C₂-C₂₄-Acylaminosäuren und/oder den Estern und/oder den physiologisch verträglichen Metallsalzen dieser Substanzen in Mengen von 0,0001 - 10 Gew.%, bevorzugt 0,01 - 5 Gew.-% und besonders bevorzugt 0,1 - 3 Gew.%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein DNA-Oligonucleotid oder mindestens ein RNA-Oligonucleotid.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).
Ein erfindungsgemäß besonders bevorzugtes Oligonucleotid ist das Thymidin-Dinucleotid.
In den erfindungsgemäßen Zusammensetzungen sind die DNA-Oligonucleotide oder RNA-Oligonucleotide vorzugsweise in Mengen von 0,0001 - 5 Gew.-%, bevorzugt 0,001 - 1,0 Gew.-% und besonders bevorzugt 0,01 - 0,5 Gew.-%, bezogen auf das gesamte Mittel, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens eine natürliche Betainverbindung (abgesehen von Ergothionein). Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).
Die Betainverbindungen sind in den erfindungsgemäßen Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zubereitungen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf das gesamte Mittel, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.%, bezogen auf das gesamte Mittel, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

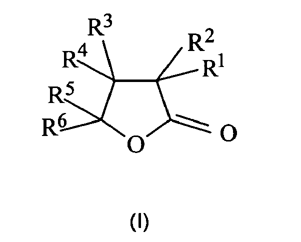

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugt verwendete Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugt verwendete Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Unter Vitamin F werden üblicherweise essentielle Fettsäuren, insbesondere Linolsäure, Linolensäure und Arachidonsäure, verstanden.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten. Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl- , Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind vorzugsweise in Mengen von 0,1 - 10 Gew.-%, bevorzugt 0,5 - 5 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein Flavonoid oder mindestens einen Flavonoid-reichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Penta-hydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-gluco-pyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.
Erfindungsgemäß werden die Flavonoide vorzugsweise in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.
Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß werden die Isoflavonoide vorzugsweise in Mengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, eingesetzt.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Erfindungsgemäß werden die Polyphenole vorzugsweise in Mengen von 0,001 bis 10 Gew.-%, bevorzugt 0,005 bis 5 Gew.-% und besonders bevorzugt 0,01 bis 3 Gew.-%, jeweils bezogen auf die gesamte kosmetische Zusammensetzung, eingesetzt.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 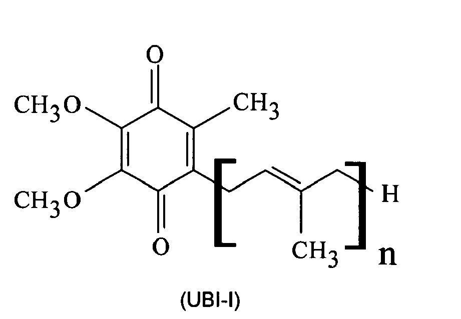 mit n = 6, 7, 8, 9 oder 10.
Besonders bevorzugt ist das Ubichinon der Formel (II) mit n = 10, auch bekannt als Coenzym Q10.
Erfindungsgemäß werden die Ubichinone, Ubichinole oder deren Derivate vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß wird Silymarin vorzugsweise in Mengen von 0,0001 bis 1 Gew.-%, bevorzugt 0,001 bis 1,0 Gew.-% und besonders bevorzugt 0,005 bis 0,5 Gew.-%, jeweils bezogen auf die gesamte Zusammensetzung, eingesetzt.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{□}N^{□}N^{□}-trialkyl-L-histidinium-Betain(en) mindestens ein natürlich vorkommendes Xanthin-Derivat, ausgewählt aus Coffein, Theophyllin, Theobromin und Aminophyllin.
Erfindungsgemäß sind die natürlich vorkommenden Xanthin-Derivate vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) Ectoin. Ectoin ist der Trivialname für 2-Methyl-1,4,5,6-tetrahydropyrimidin-4-carboxylat. Erfindungsgemäß ist Ectoin vorzugsweise in Mengen von 0,0001 bis 1 Gew.%, bevorzugt 0,001 bis 0,5 Gew.-% und besonders bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens eine anorganische und/oder mindestens eine organische UV-Filtersubstanz.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt.
Die erfindungsgemäß verwendeten organischen UV-Filter sind ausgewählt aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Meth-oxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(di- methylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten.
Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid. Erfindungsgemäß sind die organischen UV-Filtersubstanzen in Mengen von 0,1 - 30 Gew.-%, bevorzugt 0,5 - 20 Gew.-%, besonders bevorzugt 1,0 - 15 Gew.-% und außerordentlich bevorzugt 3,0 -10 Gew.-%, jeweils bezogen auf die gesamte Zusammensetzung, enthalten.
Erfindungsgemäß sind die anorganischen UV-Filtersubstanzen vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 -4,0 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens einen selbstbräunenden Wirkstoff. Erfindungsgemäß bevorzugte selbstbräunende Wirkstoffe sind ausgewählt aus Dihydroxyaceton und Erythrulose.
Erfindungsgemäß sind die selbstbräunenden Wirkstoffe vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsäure und/oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.
Die hautaufhellenden Wirkstoffe sind vorzugsweise in einer Menge von 0,05 bis 5 Gew.-%, bevorzugt von 0,1 - 2 Gew-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens einen hautberuhigenden Wirkstoff. Erfindungsgemäß bevorzugte hautberuhigende Wirkstoffe sind ausgewählt aus Allantoin, α-Bisabolol und α-Liponsäure.
Die hautberuhigenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens einen sebumregulierenden Wirkstoff. Erfindungsgemäß bevorzugte sebumregulierende Wirkstoffe sind ausgewählt aus Acnacidol, Azelainsäure, Azeloglycina und einem Extrakt aus Spiraea Ulmaria. Der Extrakt ist z.B. im Produkt Seburegul der Firma Silab enthalten. Die sebumregulierenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben dem bzw. den Extrakt(en) aus Laminaria Digitata und/oder Laminarin und/oder dem bzw. den 2-Mercapto-N^{α}N^{α}N^{α}-trialkyl-L-histidinium-Betain(en) mindestens einen Desoxyzucker oder ein mindestens einen Desoxyzucker-Baustein enthaltendes Polysaccharid.
Erfindungsgemäß bevorzugte Desoxyzucker sind ausgewählt aus Rhamnose und Fucose. Erfindungsgemäß besonders bevorzugte, Desoxyzucker-Bausteine enthaltende Polysaccharide sind Biosaccharide Gum-1, enthalten beispielsweise in dem Handelsprodukt Fucogel^{®} von Solabia, Biosaccharide Gum-2, enthalten beispielsweise in dem Handelsprodukt Rhamnosoft^{®} von Solabia, Biosaccharide Gum-3, enthalten beispielsweise in dem Handelsprodukt Fucogenol^{®} von Solabia, Biosaccharide Gum-4, enthalten beispielsweise in dem Handelsprodukt Glycofilm^{®} von Solabia, sowie Mischungen aus Biosaccharide Gum-2 und Biosaccharide Gum-3, beispielsweise erhältlich als Handelsprodukt Elastinol plus^{®} von Solabia.
Die Desoxyzucker oder Desoxyzucker-Bausteine enthaltenden Polysaccharide sind vorzugsweise in Mengen von 0,001 bis 5 Gew.-%, bevorzugt 0,01 bis 2 Gew.-% und besonders bevorzugt 0,1 bis 1 Gew.%, jeweils bezogen auf die gesamte Zusammensetzung, enthalten.

Je nach Darreichungsform enthalten die erfindungsgemäßen bzw. erfindungsgemäß verwendeten Zusammensetzungen weitere Wirk- und/oder Hilfsstoffe.

Bevorzugte optionale Inhaltsstoffe der erfindungsgemäßen bzw. erfindungsgemäß verwendeten Zusammensetzungen können über ihre Funktion definiert werden. Natürlich können manche Inhaltsstoffe auch multifunktionell sein.
Bevorzugte Inhaltsstoffe der erfindungsgemäßen Mittel, vorzugsweise Kosmetikprodukte können sein:
a) Absorptionsmittel
   Diese haben vorzugsweise die Aufgabe, wasser- und/oder öllösliche aufgelöste oder feinverteilte Substanzen aufzunehmen.
b) Antimikrobielle Stoffe
   Diese können den Produkten zugesetzt werden, um ganz allgemein die Aktivitäten von Mikroorganismen, insbesondere den Mikroorganismen, die für die Bildung von Akne verantwortlich sind, zu verringern. Zu diesen Keimen zählen unter anderem verschiedene Spezies aus der Gruppe der Staphylokokken, der Gruppe der Corynebakterien, Anaerokokken und Mikrokokken, insbesondere Corynebacterium acnes, Corynebacterium granulosum, Propionibacterium acnes, Propionibacterium avidum und Propionibacterium granulosum. Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzen extrakte (z. B. grüner Tee und Bestandteile des Lindenblütenöls) einsetzbar. Weitere bevorzugte Wirkstoffe gegen Akne und unreine Haut sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nichtgeruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.
c) Antiperspirantien
   Diese werden vorzugsweise in Antitranspirantien eingesetzt und verringern die Schweißabgabe. Schweißhemmende Wirkstoffe haben eine porenadstringierende Wirkung und werden daher häufig auch in Hautbehandlungsmitteln zur Sebumsuppression eingesetzt. Die zur Sebumsuppression verwendeten Einsatzmengen sind dabei üblicherweise geringer als die zur Schweißhemmung verwendeten Mengen und liegen im Bereich von 0,1 - 10 Gew.-%, bevorzugt 2 - 3 Gew.-%. Bevorzugte erfindungsgemäße Mittel enthalten - zur Unterstützung der Gesamtleistung des Produktes - mindestens einen adstringierenden Antitranspirant-Wirkstoff, ausgewählt aus den wasserlöslichen adstringierenden anorganischen und organischen Salzen des Aluminiums, Zirkoniums und Zinks bzw. beliebigen Mischungen dieser Salze, enthalten ist. Besonders bevorzugte Antitranspirant-Wirkstoffe sind ausgewählt aus den Aluminiumchlorhydraten, insbesondere den Aluminiumchlorhydraten mit der allgemeinen Formel [Al₂(OH)₅Cl · 2-3 H₂O]ₙ, die in nicht-aktivierter oder in aktivierter (depolymerisierter) Form vorliegen können, weiterhin Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAI(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. Die Antitranspirant-Wirkstoffe können als wässrige Lösung eingesetzt werden. In bevorzugten wasserfreien erfindungsgemäßen Mitteln ist bevorzugt mindestens ein aktiviertes Aluminiumchlorhydrat enthalten, bevorzugt ein Rohstoff aus der Serie REACH^{®} von Reheis.
d) Antischaummittel
   Diese können zugesetzt werden, z.B. um Schaum während der Herstellung zu beseitigen oder um die Neigung von Fertigprodukten zur überschießenden Schaumbildung zu verringern.
e) Bindemittel
   Sie gewährleisten z.B. den Zusammenhalt pulver- und puderhaltiger kosmetischer Produkte.
f) Stoffe biologischen Ursprungs
   Das sind z.B. bestimmte Pflanzeninhaltsstoffe, z.B. Grüner-Tee-Extrakt. Sie sollen einem Produkt bestimmte, gewünschte Eigenschaften verleihen, die mit dem entsprechenden biologischen Materialen in Zusammenhang stehen, oder aber bereits bestehende Eigenschaften weiter verbessern oder unerwünschte Eigenschaften unterdrücken bzw. sie soweit wie möglich verringern.
g) Bleichmittel
   Diese können z.B. dazu dienen, den Farbton des Haares oder der Haut aufzuhellen.
h) Chelatbildner
   Diese werden z.B. kosmetischen Mitteln zugesetzt, damit sie Komplexe mit Metallionen bilden, um so beispielsweise die Lagerstabilität der erfindungsgemäßen Mittel zu verbessern. Als komplexbildende Substanz besonders bevorzugt ist Ethylendiamintetraessigsäure (EDTA) und ihre Natriumsalze, wie sie beispielsweise unter dem Handelsnamen Trilon B von der Firma BASF erhältlich ist, weiterhin Nitrilotriessigsäure (NTA) und ihre Natriumsalze, β-Alanindiessigsäure und ihre Salze und Phosphonsäuren und deren Salze. Die mindestens eine komplexbildende Substanz ist bevorzugt in einer Gesamtmenge von 0,01 - 0,5 Gew.-%, besonders bevorzugt 0,08 - 0,2 Gew.-%, bezogen auf das Gesamtgewicht der erfindungsgemäßen Zusammensetzung, enthalten.
i) Deodorant-Wirkstoffe,
   in einer weiteren bevorzugten Ausführungsform können die erfindungsgemäßen Mittel mindestens einen Deodorant-Wirkstoff enthalten. Dieser kann bevorzugt aus den bereits genannten antimikrobiellen Wirkstoffen ausgewählt sein. Weiterhin kann es bevorzugt sein, einen Wirkstoff einzusetzen, der die für die Schweißzersetzung verantwortlichen Enzyme, insbesondere die Arylsulfatase, β-Glucuronidase, Aminoacylase, Esterasen, Lipasen und/oder Lipoxigenase, hemmt, z. B. Trialkylcitronensäureester, insbesondere Triethylcitrat, oder Zinkglycinat. Weitere Inhibitorsubstanzen der für die Schweißzersetzung verantwortlichen Enzyme und Keime, sind offenbart in WO 03/039505 A2, WO 01/99376 A2, EP 1430879 A2, EP 1428520 A2, EP 1738803 A1, EP 1576946 A1 und DE 10216368 A1. Weitere eindungsgemäß bevorzugte Deodorant-Wirkstoffe sind Geruchsabsorber und desodorierend wirkende Ionenaustauscher. Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.
j) Emollientien
   Diese haben die Aufgabe, die Haut geschmeidig zu machen und zu glätten.
   Bevorzugte erfindungsgemäße Zusammensetzungen, die als Emulsion, Suspension, Spray oder Stift vorliegen, enthalten als Emollient mindestens ein bei 20 ° C flüssiges Öl, das keine Duftstoffkomponente und kein etherisches Öl darstellt. Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24. Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat. Weitere erfindungsgemäß bevorzugte Emollient-Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten. Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat. Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM). Weitere erfindungsgemäß bevorzugte Ölkomponenten sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat. Weitere erfindungsgemäß bevorzugte Ölkomponenten sind ausgewählt aus den Anlagerungsprodukten von mindestens 6 Ethylenoxid und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57) und PPG-15-Stearylether (Arlamol^{®} E). Weitere erfindungsgemäß bevorzugte Ölkomponenten sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI. Weitere erfindungsgemäß bevorzugte Ölkomponenten sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1. Weitere erfindungsgemäß bevorzugte Ölkomponenten sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Es kann erfindungsgemäß außerordentlich bevorzugt sein, Mischungen der vorgenannten Öle einzusetzen. Weitere erfindungsgemäß bevorzugte Ölkomponenten sind ausgewählt aus Siliconölen und Kohlenwasserstoffölen. Erfindungsgemäß bevorzugte Siliconöle sind ausgewählt aus Dialkyl- und Alkylarylsiloxanen, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Weitere erfindungsgemäß bevorzugte Siliconöle sind ausgewählt aus flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind. Weitere erfindungsgemäß bevorzugte Siliconöle sind ausgewählt aus nichtflüchtigen höhermolekularen linearen Dimethylpolysiloxanen, im Handel erhältlich z. B. unter der Bezeichnung Dow Corning^{®} 190, Dow Corning^{®} 200 Fluid mit Viskositäten im Bereich von 5 - 100 cSt, bevorzugt 5 - 50 cSt oder auch 5 - 10 cSt, und Baysilon^{®} 350 M. Erfindungsgemäß bevorzugte natürliche und synthetische Kohlenwasserstoffe sind ausgewählt aus Paraffinölen, Isohexadecan, Isoeicosan, (ggf. hydrierten) Polyisobutenen und Polydecenen, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (Cetiol^{®}S). Besonders bevorzugte erfindungsgemäße Hautbehandlungsmittel sind dadurch gekennzeichnet, dass das/die bei 20 °C flüssige/n Öl/e in einer Gesamtmenge von 0,1 - 80 Gew.-%, bevorzugt 2 - 20 Gew.-%, besonders bevorzugt 3 - 15 Gew.-%, jeweils bezogen auf das Gesamtgewicht der Zusammensetzung, enthalten ist/sind. In einer weiteren bevorzugten Ausführungsform der Erfindung weist ein Anteil der Ölkomponenten von mindestens 80 Gew.-% einen Brechungsindex n_{D} von 1,39 - 1,51 auf. Besonders bevorzugt ist es, wenn 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen. Neben den Ölen, das heißt, den Substanzen, die unter Normalbedingungen flüssig vorliegen, können auch bei Normalbedingungen fest vorliegende Lipid- oder Wachskomponenten als Emollient verwendet werden. Besonders bevorzugte Lipid- oder Wachskomponenten sind ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung des Produktes und eine Minimierung der sichtbaren Rückstände auf der Haut. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol^{®} MM). Erfindungsgemäß bevorzugte Emiollients sind weiterhin natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren. Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie den erfindungsgemäßen Hautbehandlungsmitteln ausgezeichnete sensorische Eigenschaften und (für Stifte) eine hohe Stabilität verleihen. Die Ester setzen sich aus gesättigten verzweigten oder unverzweigten Monocarbonsäuren und gesättigten verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten verzweigten oder unverzweigten Alkoholen sind erfindungsgemäß einsetzbar. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkoholen einer Kettenlänge von 16 bis 50 C-Atomen, die einen Schmelzpunkt > 50°C haben. Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf. Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Kesterwachs bietet den Vorteil, dass es auch bei geringen Konzentrationen eine exzellente Ölgelierfähigkeit aufweist und so die Stiftmasse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben. Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C). Erfindungsgemäß ist als Wachskomponente hydriertes Rizinusöl, erhältlich z.B. als Handelsprodukt Cutina^{®} HR, besonders bevorzugt. Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Palmitin- und Stearinsäure).
k) Emulgatoren /Dispergatoren
   Hierbei handelt es sich um oberflächenaktive Substanzen, die vorzugsweise im Stande sind, nicht mischbare Flüssigkeiten wie Öl und Wasser stabil ineinander zu verteilen bzw. die das Dispergieren eines pulverförmigen Feststoffes in einem flüssigen Medium verbessern.
   Erfindungsgemäß besonders bevorzugte Zusammensetzungen, die als Wasser-in-Öl-Emulsion konfektioniert sind, enthalten bevorzugt mindestens einen Wasser-in-Öl-Emulgator. Der mindestens eine Wasser-in-Öl-Emulgator ist bevorzugt in einer Menge von 0,5 - 5 Gew.-%, besonders bevorzugt 1,0 - 1,5 - 2,5 Gew.-%, jeweils bezogen auf das Gesamtgewicht der Emulsion, enthalten. Eine erfindungsgemäß besonders bevorzugte Gruppe von Wasser-in-Öl-Emulgatoren sind die Poly-(C₂-C₃)alkylenglycol-modifizierten Silicone, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, PEG/PPG-4/12 Dimethicone, das unter der Bezeichnung Abil B 8852 erhältlich ist, sowie Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440). Weitere erfindungsgemäß bevorzugte W/O-Emulgatoren sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyole und Alkyl Dimethicone Ethoxy Glucoside. Die erfindungsgemäßen Zusammensetzungen, die als Emulsion, insbesondere als Öl-in-Wasser-Emulsion oder Polyol-in-Wasser-Emulsion, formuliert sind, enthalten bevorzugt mindestens einen nichtionischen Öl-in-Wasser-Emulgator mit einem HLB-Wert von mehr als 7. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. Bei der Auswahl erfindungsgemäß geeigneter nichtionischer ÖI-in-Wasser-Emulgatoren ist es besonders bevorzugt, ein Gemisch von nichtionischen ÖI-in-Wasser-Emulgatoren einzusetzen, um die Stabilität der erfindungsgemäßen Stiftzusammensetzungen optimal einstellen zu können. Die einzelnen Emulgatorkomponenten liefern dabei einen Anteil zum Gesamt-HLB-Wert oder mittleren HLB-Wert des ÖI-in-Wasser-Emulgatorgemisches gemäß ihrem Mengenanteil an der Gesamtmenge der öl-in-Wasser-Emulgatoren. Erfindungsgemäß beträgt der mittlere HLB-Wert des ÖI-in-Wasser- Emulgatorgemisches 10 - 19, bevorzugt 12 - 18 und besonders bevorzugt 14 - 17. Um derartige mittlere HLB-Werte zu erzielen, werden bevorzugt Öl-in-Wasser-Emulgatoren aus den HLB-Wertbereichen 10 - 14, 14 - 16 und gegebenenfalls 16 - 19 miteinander kombiniert. Selbstverständlich können die Öl-in-Wasser-Emulgatorgemische auch nichtionische Emulgatoren mit HLB-Werten im Bereich von > 7 - 10 und 19 - 20 enthalten; derartige Emulgatorgemische können erfindungsgemäß ebenfalls bevorzugt sein. Die erfindungsgemäßen Hautbehandlungsmittel können aber in einer anderen bevorzugten Ausführungsform auch nur einen einzigen Öl-in-Wasser-Emulgator mit einem HLB-Wert im Bereich von 10 - 19 enthalten. Bevorzugte erfindungsgemäße Hautbehandlungsmittel sind dadurch gekennzeichnet, dass die nichtionischen Öl-in-Wasser-Emulgatoren ausgewählt sind aus ethoxylierten C₈-C₂₄-Alkanolen mit durchschnittlich 10 - 100 Mol Ethylenoxid pro Mol, ethoxylierten C₈-C₂₄-Carbonsäuren mit durchschnittlich 10 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen. Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat. Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20. Weiterhin werden vorzugsweise C₈ - C₂₂-Alkylmono- und -oligoglycoside eingesetzt. C₈-C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere bei 1,1 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet. Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol. Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexa glycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat. Besonders bevorzugte erfindungsgemäße Hautbehandlungsmittel sind dadurch gekennzeichnet, dass der nichtionische Öl-in-Wasser-Emulgator in einer Gesamtmenge von 0,5 - 10 Gew.-%, besonders bevorzugt 1 - 4 Gew.-% und außerordentlich bevorzugt 1,5 - 3 Gew.-%, bezogen auf die Gesamtzusammensetzung, enthalten ist. Erfindungsgemäß bevorzugte Zusammensetzungen, die als Emulsion oder Stift formuliert sind, enthalten bevorzugt weiterhin mindestens einen nichtionischen Wasser-in-ÖI-Emulgator mit einem HLB-Wert größer 1,0 und kleiner/gleich 7,0, ausgewählt aus den Mono- und Diestern von Ethylenglycol und den Mono-, Di-, Tri- und Tetraestern von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-Öl-Emulgatoren mit einem HLB-Wert größer 1,0 und kleiner/gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon. Erfindungsgemäß besonders bevorzugte Wasser-in-Öl-Emulgatoren mit einem HLB-Wert größer 1,0 und kleiner/ gleich 7,0 sind zum Beispiel als Handelsprodukte Cutina® PES (INCI: Pentaerythrityl distearate), Cutina® AGS (INCI: Glycol distearate) oder Cutina® EGMS (INCI: Glycol stearate) erhältlich. Diese Handelsprodukte stellen bereits Mischungen aus Mono- und Diestern (bei den Pentaerythritylestern sind auch Tri- und Tetraester enthalten) dar. Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-Öl-Emulgatoren. Unter einer technischen Mischung wird beispielsweise ein Handelsprodukt wie Cutina® PES verstanden. Außer den genannten Wasser-in-Öl-Emulgatoren auf Basis der Ethylenglycol- oder Pentaerythritylester kann in einer bevorzugten Ausführungsform auch noch mindestens ein weiterer nichtionischer Wasser-in-Öl-Emulgator mit einem HLB-Wert größer 1,0 und kleiner/gleich 7,0 enthalten sein, dessen Anteil an dem Gesamtgewicht an nichtionischen Wasser-in-Öl-Emulgatoren mit einem HLB-Wert größer 1,0 und kleiner/gleich 7,0 bevorzugt allerdings nicht größer als 80 % sein sollte. In einer besonders bevorzugten Ausführungsform enthalten die erfindungsgemäßen Zusammensetzungen den mindestens einen zusätzlichen Wasser-in-ÖI-Emulgator mit einem HLB-Wert größer 1,0 und kleiner/ gleich 7,0 nur in einem Gewichtsanteil von maximal 10 % beziehungsweise sind frei von zusätzlichen Wasser-in-Öl-Emulgatoren. Einige dieser zusätzlichen geeigneten Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen. Als Wasser-in-Öl-Emulgator bevorzugt geeignet sind: lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind, Ester und insbesondere Partialester aus einem Polyol mit 3 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃ₒ-Carbonsäuren, die hydroxyliert sein können, insbesondere diejenigen von Myristinsäure, Palmitinsäure, Stearinsäure oder von Mischungen dieser Fettsäuren und die Methylglucosemono- und -diester von linearen gesättigten und ungesättigten C₁₂ - C₃₀-Carbonsäuren, die hydroxyliert sein können; Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1 - 5 EO) sein können; Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen; Glycerinmonoether gesättigter und/oder ungesättigter, verzweigter und/oder unverzweigter Alkohole einer Kettenlänge von 8 - 30, insbesondere 12 - 18 Kohlenstoffatomen; Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen, sowie Mischungen der vorgenannten Substanzen. Erfindungsgemäß kann es bevorzugt sein, nur einen einzigen zusätzlichen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei zusätzlichen Wasser-in-Öl-Emulgatoren. Unter einer technischen Mischung wird beispielsweise ein Handelsprodukt wie Cutina® GMS verstanden, das eine Mischung aus Glycerylmonostearat und Glyceryldistearat darstellt. Besonders vorteilhaft einsetzbare zusätzliche Wasser-in-Öl-Emulgatoren sind Stearylalkohol, Cetylalkohol, Glycerylmonostearat, insbesondere in Form der Handelsprodukte Cutina® GMS und Cutina® MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat. Besonders bevorzugte erfindungsgemäße Hautbehandlungsmittel sind dadurch gekennzeichnet, dass mindestens ein Wasser-in-Öl-Emulgator in einer Gesamtmenge von 0,1 - 15 Gew.%, bevorzugt 0,5 - 8,0 Gew.%, und besonders bevorzugt 1 - 4 Gew.%, bezogen auf die Gesamtzusammensetzung, enthalten sind. Weiterhin können auch Mengen von 2 - 3 Gew.%, bezogen auf das Gesamtgewicht der Zusammensetzung, erfindungsgemäß außerordentlich bevorzugt sein.
l) Emulsionsstabilisatoren
   Diese können den Prozeß der Emulgierung (vgl. Emulgatoren) noch weiter unterstützen und auf diese Weise die Stabilität und Haltbarkeit des Produktes weiter verbessern.
m) Enthaarungsmittel und Wirkstoffe mit Haarwuchs inhibierender Wirkung.
   Diese dienen der vorzugsweise selektiven Entfernung von Körperbehaarung beziehungsweise der Verhinderung des Nachwachsens von entferntem, beispielsweise rasiertem oder epiliertem, Haar. Bevorzugte Substanzen, die den Haarwuchs inhibieren, sind insbesondere ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, wie sie beispielsweise und bevorzugt in dem Rohstoff Pilinhib^{®} Veg LS 9109 von Laboratoires Serobiologiques mit der INCI-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (pumpkin, Zucchini) und den Früchten von Serenoa serrulata, wie sie beispielsweise und bevorzugt in den Rohstoffen ARP 100 von Greentech S.A./Rahn mit der INCI-Deklaration "Water, Alcohol, Serenoa Serrulata Fruit Extract, Epilobium Angustifolium Extract, Cucurbita Pepo (Pumpkin) Seed Extract" und ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag / Seporga mit der INCI-Deklaration "Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration "Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise und bevorzugt in dem Rohstoff Capislow von Sederma, der Lecithinvesikel mit einem hydroglycolischen Extrakt aus Larrea divaricata enthält, enthalten sind. Weitere bevorzugte Haarwuchs-inhibierende Wirkstoffe sind ausgewählt aus Substanzen, die die Protein-Tyrosinkinase hemmen, insbesondere aus Lavendustin-A, Erbstatin, Tyrphostin, Piceatannol, 4-Hydroxybenxylidenmalononitril, 3,5-Di-*tert*-butyl-4- hydroxybenzylidenmalononitril, α-Cyano-(3,4-di-hydroxy)-cinnamonitril, α-Cyano-(3,4,5-trihydroxy)cinnamonitril, α-Cyano-(3,4-dihydroxy)cinnamid, α-Cyano-(3,4-dihydroxy)thiocinnamid, 2- Amino-4-(4'-hydroxyphenyl)-1,1,3-tricyanobuta-1,3-dien, 2-Amino-4-(3,4,5'- trihydroxyphenyl)-1,1,3-tricyanobuta-1,3-dien, 2-Amino-4-(1H-alpha-indol-5-yl)-1,1,3-tricyanobuta-1,3-dien, 4-Hydroxy-3-methoxy-5-(benzothiazolylthiomethyl)benzylidencyano-acetamid, 4-Amino-N-(2,5-dihydroxybenzyl)methylbenzoat, α-Cyano-(3,4-dihydroxy)-cinnamonitril, 4-(3-Chloranilino)-6,7-dimethoxychinazolin, α-Cyano-(3,4-dihydroxy)-N-benzylcinnamid, (-)-R-N-(a-Methylbenzyl)-3,4-dihydroxybenzylidencyanoacetamid, α-Cyano-(3,4-dihydroxy)-N-(3-phenylpropyl)-cinnamid, α-Cyano-(3,4-dihydroxy)-N-phenylcinnamid, α-Cyano-(+)-(S)-N-(alpha-phenethyl)-(3,4-dihydroxy)cinnamid, α-Cyano-(3,4-dihydroxy)-N-(phenylbutyl)cinnamid, Herbimycin A, Thiazolidindion, Phenazocin, 2,3-Dihydro-2-thioxo-1H-indol-3-Alkansäuren, 2,2'-Dithiobis-(1H-indol-3-Alkansäuren), Sulfonylbenzoylnitrostyrol, Methylcaffeat, HNMPA(AM)₃(hydroxy-2-naphthalenyl-methylphosphonsäure-*tris*-acetoxymethylester) und N-Acetyl-Asp-Tyr-(2-malonyl)-Val-Pro-Met-Leu-NH₂. Weitere bevorzugte Haarwuchs-inhibierende Wirkstoffe sind ausgewählt aus den in WO 2006/ 130330 A2 offenbarten Substanzen, nämlich Agonisten des Farnesoid X-Rezeptors, bevorzugt ausgewählt aus Gallensäuren, wie insbesondere Lithocholsäure, Cholsäure, Deoxycholsäure, Chenodeoxycholsäure, Ursodeoxycholsäure und 6-alpha-Ethylchenodeoxycholsäure, weiterhin aus Farnesoiden, insbesondere Farnesol (3,7,11-Trimethyl-2,6,10-dodecatrien-1-ol), Farnesal, Farnesylacetat, 3,7,11-Trimethyl-2,6,10-dodecatrien-1-carbonsäure, Methylfarnesylether, Methylfarnesoat, Ethylfarnesylether, Ethylfarnesoat, weiterhin aus 7-Methyl-9-(3,3-dimethyloxivanyl)-3-methyl-2,6-nonadiensäuremethylester (Juvenilhormon III), 7-Methyl-9-(3,3-dimethyloxivanyl)-3-methyl-2,6-nonadiensäureethylester, 3-alpha,7-alpha-Dihydroxy-6-alpha-ethyl-5p-cholan-24-säure, 7-alpha-Dihydroxy-6-alpha-propyl-5p-cholan-24-säure, 7-alpha-Dihydroxy-6-alpha-allyl-5p-cholan-24-säure, N-(2,2,2-trifluoroethyl)-N-[4-[2,2,2-trifluoro-1-hydroxy-1-(trifluoromethyl)-ethyl]phenyl]-benzolsulfonanilid, 3-[2-[2-Chloro-4-[3-(2,6-dichlorophenyl)-5-(1-methylethyl)-4-isoxazolyl]-methoxy]-phenylethenyl]-benzoesäure, [3,5-bis(1,1-dimethylethyl)-4-hydroxyphenyl]ethenyliden]-bisphosphonsäuretetraethylester, [2-[3,5-bis(1,1-dimethylethyl)-4-hydroxyphenyl]ethyliden]bisphos-phonsäuretetrakis(1-methylethyl)ester, [2-[3,5-bis(1,1-dimethylethyl)-4-hydroxyphenyl]ethyliden]-bisphosphonsäuretetraethylester und [3,5-bis(1,1-dimethylethyl-4-hydroxyphenyl]ethenyliden]bis-phosphonsäuretetrakis(1-methylethyl)ester. Bevorzugte erfindungsgemäße Zusammensetzungen enthalten mindestens eine den Haarwuchs inhibierende Substanz in einer Gesamtmenge von 0,0001 - 5 Gew.-%, bevorzugt 0,001 - 2 Gew.-%, besonders bevorzugt 0,01 - 1 Gew.-%, und außerordentlich bevorzugt 0,1 - 0,5 Gew.-%, jeweils bezogen auf das Gewicht an Aktivsubstanz des/der Haarwuchs inhibierenden Wirkstoffs/e und das Gesamtgewicht der erfindungsgemäßen Zusammensetzung.
n) Feuchtigkeitsspender
   Diese können einen Beitrag zur Erhaltung oder Wiederherstellung der Hautfeuchtigkeit leisten und dem Austrocknen der Haut entgegenwirken. Neben den oben erwähnten Emollients eignen sich hierzu vor wasserlösliche mehrwertige C₂ - C₉-Alkanole mit 2 - 6 Hydroxylgruppen und/oder wasserlösliche Polyethylenglycole mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fucose, Rhamnose, Fructose, Glucose, Maltose, Maltitol, Mannit, Inosit, Sucrose, Trehalose und Xylose sind erfindungsgemäß geeignet. Bevorzugte erfindungsgemäße Hautbehandlungsmittel sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Besonders bevorzugte erfindungsgemäße Hautbehandlungsmittel sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist.
o) Filmbildner
   Diese sind dazu im Stande, einen schützenden, stabilisierenden Film auf Oberflächen, vorzugsweise Haut, Haar oder Nägeln, zu erzeugen. Mit Bezug auf die erfindungsgemäßen Mittel wirkt dieser Film vorzugsweise feuchtigkeitsspeichernd. Bevorzugte feuchtigkeitsspeichernde Filmbildner sind ausgewählt aus Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, Glycosaminoglycanen, besonders bevorzugt Hyaluronsäure, Dextran, Dextransulfat, Chondroitin-4-sulfat und Chondroitin-6-sulfat.
p) Farbstoffe
   Diese werden kosmetischen Produkten zugesetzt, um eine Produktfärbung zu erzeugen,
q) Konservierungsstoffe
   Diese werden kosmetischen Mitteln zugesetzt, um sie vor der schadhaften Einwirkung von Mikroorganismen (Bakterien, Pilze, Hefen) zu bewahren und damit ihren Verderb zu vermeiden. Zahlreiche Konservierungsstoffe haben zwangsläufig auch desodorierende Eigenschaften, da sie auch gegen geruchsverursachende Bakterien antimikrobiell wirken, so dass einige Stoffe beiden Gruppen angehören. Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z. B. Methyl-, Ethyl-, Propyl-, Isopropyl-, Butyl-, Isobutyl-, Isodecyl-, Phenyl-, Phenoxyethyl- und Benzylparaben, Hexamidinparaben und -diparaben, Natrium- und Kaliumparaben, Natrium- und Kaliummethylparaben, Kaliumbutylparaben, Natrium- und Kaliumpropylparaben), Alkohole und deren Salze (z.B. Ethanol, Propanol, Isopropanol, Benzylalkohol, Phenethylalkohol, Phenol, Kaliumphenolat, Phenoxyethanol, Phenoxyisopropanol, o-Phenylphenol), Guajacol und dessen Derivate, Chlorhexidin und dessen Derivate (z. B. Chlorhexidindiacetat, -digluconat, und -dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylharnstoff, Imidazolidinylharnstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquaternium-42, Quaternium-8, Quaternium-14, Quaternium-15), Carbamate (z. B. lodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Triclosan, Zinkpyrithion sowie diverse Peptid-Antibiotika (z. B. Nisin). Erfindungsgemäß bevorzugte Konservierungsmittel sind Phenoxyethanol, die Ester der 4-Hydroxybenzoesäure, insbesondere Methyl-, Ethyl-, Propyl-, Isopropyl-, Butyl- und Isobutylparaben sowie lodopropynylbutylcarbamat. Die Menge der Konservierungsmittel in den bevorzugten erfindungsgemäßen Zusammensetzungen beträgt 0,001 - 10 Gew.-%, vorzugsweise 0,01 - 5 Gew.-% und insbesondere 0,1 - 3 Gew.-%, bezogen auf das Gesamtgewicht der Zusammensetzung.
r) Korrosionsschutzmittel
   Diese können z.B. dazu dienen, die Korrosion der Verpackung z.B. eines kosmetischen Mittels zu verhindern oder aber auch die Korrosion von Teilen, die mit dem Mittel ansonsten in Kontakt treten.
s) Hautkühlende Wirkstoffe
   Erfindungsgemäß besonders bevorzugte Zusammensetzungen enthalten weiterhin bevorzugt mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4- dioxaspiro (4.5)decan-2-methanol), Monomenthylsuccinat und 2-Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol. Erfindungsgemäß besonders bevorzugt ist, dass mindestens ein hautkühlender Wirkstoff in einer Gesamtmenge von 0,01 - 1 Gew.-%, besonders bevorzugt 0,02 - 0,5 Gew.% und außerordentlich bevorzugt 0,05 - 0,2 Gew.%, jeweils bezogen auf das Gesamtgewicht der Zusammensetzung, enthalten ist.
t) pH-Wert-Regler/Puffersubstanzen
   Diese können z.B. in Kosmetika dazu dienen, um einen gewünschten pH-Wert einzustellen bzw. zu stabilisieren.
u) Tenside/Waschaktive Substanzen
   Dies sind grenzflächenaktive Verbindungen, die Reinigungszwecken oder aber der besseren Abwaschbarkeit der erfindungsgemäßen Mittel dienen.
v) Treibgase
   Dies sind gasförmige Substanzen, mit denen die erfindungsgemäßen kosmetischen Mittel unter Druck in druckbeständigen Behältern gesetzt werden können, um den Inhalt dann bei Druckentlastung hervorzubringen. Erfindungsgemäß besonders bevorzugte Zusammensetzungen, die als treibgasgetriebenes Aerosol konfektioniert sind, enthalten mindestens ein Treibmittel. Bevorzugte Treibmittel (Treibgase) sind Propan, Propen, n-Butan, iso-Butan, iso-Buten, n-Pentan, Penten, isoPentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann. Die Menge der Treibmittel beträgt bevorzugt 20 - 80 Gew.%, besonders bevorzugt 30 - 70 Gew.% und außerordentlich bevorzugt 40 - 50 Gew.-%, jeweils bezogen auf das Gesamtgewicht der Zubereitung, bestehend aus der erfindungsgemäßen Zusammensetzung und dem Treibmittel
w) Trübungsmittel
   Diese kann man vorzugsweise durchsichtigen oder durchscheinenden Produkten beigeben, um sie undurchdringlicher für sichtbares Licht oder lichtnahe Strahlung zu machen. Dies kann zum Schutz des die AQP-Synthese-stimulierenden Wirkstoffs während der Lagerung in transparenten oder transluzenten Behältern gegebenenfalls von Vorteil sein; die Schichtdicke, die später auf die auf die Haut appliziert wird, ist so gering, dass der Zusatz eines Trübungsmittels die Wirksamkeit des die AQP-Synthese-stimulierenden Wirkstoffs, insbesondere Ergothionein, nicht beeinträchtigt.
x) UV-Absorber/Lichtfiltersubstanzen
   Sie sind im Stande, bestimmte UV-Strahlen zu filtern und können auf diese Weise z.B. die Haut vor vorzeitiger, lichtbedingter Alterung sowie vor Sonnenbrand schützen. UV-Absorber können in geringen Mengen aber auch als Produktschutz eingesetzt werden.
y) Vergällungsmittel
   Diese werden kosmetischen Mitteln, die Ethanol enthalten, zugesetzt, um sie ungenießbar zu machen.
z) Viskositätsregler
   Diese sind im Stande, die Zähflüssigkeit eines Produkts zu erhöhen oder auch zu verringern.

Vorgenannte Inhaltsstoffe können gemäß weiterer bevorzugter Ausführungsformen als einziger Zusatz oder in beliebigen Kombinationen in den erfindungsgemäßen Mitteln enthalten sein.

Im Folgenden werden bevorzugte Inhaltsstoffe der erfindungsgemäßen Mittel näher beschrieben.

Anionische Tenside sind bevorzugt in den erfindungsgemäßen Mitteln enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden insbesondere in Reinigungsmitteln aufgrund ihres hohen Schaumverhaltens vorzugsweise nur in relativ geringen Mengen, beispielsweise in Mengen von 1 bis 5 Gew.-%, eingesetzt. Vorzugsweise können die erfindungsgemäßen Mittel frei von Schwefelsäuremonoester sein.

Eine weitere Klasse von Aniontensiden ist die durch Umsetzung von Fettalkoholethoxylaten mit Natriumchloracetat in Gegenwart basischer Katalysatoren zugängliche Klasse der Ethercarbonsäuren. Sie haben die allgemeine Formel: R¹⁰ O-(CH₂-CH₂-O)*ₚ*-CH₂-COOH mit R¹⁰ = C₁-C₁₈ und *p* = 0,1 bis 20.

Ethercarbonsäuren sind wasserhärteunempfindlich und weisen ausgezeichnete Tensideigenschaften auf.

Geeignete anionische Tenside sind beispielsweise auch die Partialester von Di- oder Polyhydroxyalkanen, Mono- und Disacchariden, Polyethylenglycolen mit den EO-Addukten von Maleinsäureanhydrid an mindestens einfach ungesättigte Carbonsäuren mit einer Kettenlänge von 10 bis 25 Kohlenstoffatomen mit einer Säurezahl von 10 bis 140.

Bevorzugte anionische Tenside weisen neben einem unverzweigten oder verzweigten, gesättigten oder ungesättigten, aliphatischen oder aromatischen, acyclischen oder cyclischen, optional alkoxylierten Alkylrest mit 4 bis 28, vorzugsweise 6 bis 20, insbesondere 8 bis 18, besonders bevorzugt 10 bis 16, äußerst bevorzugt 12 bis 14 Kohlenstoffatomen, zwei oder mehr anionische, insbesondere zwei, Säuregruppen, vorzugsweise Carboxylat-, Sulfonat- und/oder Sulfatgruppen, insbesondere eine Carboxylat- und eine Sulfatgruppe, auf. Beispiele dieser Verbindungen sind die alpha-Sulfofettsäuresalze, die Acylglutamate, die Monoglyceriddisulfate und die Alkylether des Glycerindisulfats sowie insbesondere die nachfolgend beschriebenen monoveresterten Sulfosuccinate.

Besonders bevorzugte anionische Tenside sind die Sulfosuccinate, Sulfosuccinamate und Sulfosuccinamide, insbesondere Sulfosuccinate und Sulfosuccinamate, äußerst bevorzugt Sulfosuccinate. Bei den Sulfosuccinaten handelt es sich um die Salze der Mono- und Di-ester der Sulfobernsteinsäure HOOCCH(SO₃H)CH₂COOH, während man unter den Sulfosuccinamaten die Salze der Monoamide der Sulfobernsteinsäure und unter den Sulfosuccinamiden die Salze der Diamide der Sulfobernsteinsäure versteht.

Bei den Salzen handelt es sich bevorzugt um Alkalimetallsalze, Ammoniumsalze sowie Mono-, Di- bzw. Trialkanolammoniumsalze, beispielsweise Mono-, Di- bzw. Triethanolammoniumsalze, insbesondere um Lithium-, Natrium-, Kalium- oder Ammoniumsalze, besonders bevorzugt Natrium- oder Ammoniumsalze, äußerst bevorzugt Natriumsalze.

In den Sulfosuccinaten ist eine bzw. sind beide Carboxylgruppen der Sulfobernsteinsäure vorzugsweise mit einem bzw. zwei gleichen oder verschiedenen unverzweigten oder verzweigten, gesättigten oder ungesättigten, acyclischen oder cyclischen, optional alkoxylierten Alkoholen mit 4 bis 22, vorzugsweise 6 bis 20, insbesondere 8 bis 18, besonders bevorzugt 10 bis 16, äußerst bevorzugt 12 bis 14 Kohlenstoffatomen verestert. Besonders bevorzugt sind die Ester unverzweigter und/oder gesättigter und/oder acyclischer und/oder alkoxylierter Alkohole, insbesondere unverzweigter, gesättigter Fettalkohole und/oder unverzweigter, gesättigter, mit Ethylen- und/oder Propylenoxid, vorzugsweise Ethylenoxid, alkoxylierter Fettalkohole mit einem Alkoxylierungsgrad von 1 bis 20, vorzugsweise 1 bis 15, insbesondere 1 bis 10, besonders bevorzugt 1 bis 6, äußerst bevorzugt 1 bis 4. Die Monoester werden im Rahmen der vorliegenden Erfindung gegenüber den Diestern bevorzugt. Ein besonders bevorzugtes Sulfosuccinat ist Sulfobernsteinsäurelaurylpolyglycolester-di-Natrium-Salz (Lauryl-EO-sulfosuccinat, Di-Na-Salz; INCI Disodium Laureth Sulfosuccinate), das beispielsweise als Tego^{®} Sulfosuccinat F 30 (Goldschmidt) mit einem Sulfosuccinatgehalt von 30 Gew.-% kommerziell erhältlich ist.

In den Sulfosuccinamaten bzw. Sulfosuccinamiden bildet eine bzw. bilden beide Carboxylgruppen der Sulfobernsteinsäure vorzugsweise mit einem primären oder sekundären Amin, das einen oder zwei gleiche oder verschiedene, unverzweigte oder verzweigte, gesättigte oder ungesättigte, acyclische oder cyclische, optional alkoxylierte Alkylreste mit 4 bis 22, vorzugsweise 6 bis 20, insbesondere 8 bis 18, besonders bevorzugt 10 bis 16, äußerst bevorzugt 12 bis 14 Kohlenstoffatomen trägt, ein Carbonsäureamid. Besonders bevorzugt sind unverzweigte und/oder gesättigte und/oder acyclische Alkylreste, insbesondere unverzweigte, gesättigte Fettalkylreste.

Weiterhin geeignet sind beispielsweise die folgenden gemäß INCI bezeichneten Sulfosuccinate und Sulfosuccinamate, die im *International Cosmetic Ingredient Dictionary and Handbook* näher beschrieben sind: Ammonium Dinonyl Sulfosuccinate, Ammonium Lauryl Sulfosuccinate, Diammonium Dimethicone Copolyol Sulfosuccinate, Diammonium Lauramido-MEA Sulfosuccinate, Diammonium Lauryl Sulfosuccinate, Diammonium Oleamido PEG-2 Sulfosuccinate, Diamyl Sodium Sulfosuccinate, Dicapryl Sodium Sulfosuccinate, Dicyclohexyl Sodium Sulfosuccinate, Diheptyl Sodium Sulfosuccinate, Dihexyl Sodium Sulfosuccinate, Diisobutyl Sodium Sulfosuccinate, Dioctyl Sodium Sulfosuccinate, Disodium Cetearyl Sulfosuccinate, Disodium Cocamido MEA-Sulfosuccinate, Disodium Cocamido-Glucoside Sulfosuccinate, Disodium Cocoyl Butyl Gluceth-10 Sulfosuccinate, Disodium C12-15 Pareth Sulfosuccinate, Disodium Deceth-5 Sulfosuccinate, Disodium Deceth-6 Sulfosuccinate, Disodium Dihydroxyethyl Sulfosuccinylundecylenate, Disodium Dimethicone Copolyol Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Lauramido PEG-5 Sulfosuccinate, Disodium Laureth-6 Sulfosuccinate, Disodium Laureth-9 Sulfosuccinate, Disodium Laureth-12 Sulfosuccinate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PEG-5 Laurylcitrate Sulfosuccinate, Disodium PEG-8 Palm Glycerides Sulfosuccinate, Disodium Ricinoleamido MEA-Sulfosuccinate, Disodium Sitostereth-14 Sulfosuccinate, Disodium Stearamido MEA-Sulfosuccinate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Di-TEA-Oleamido PEG-2 Sulfosuccinate, Ditridecyl Sodium Sulfosuccinate, Sodium Bisglycol Ricinosulfosuccinate, Sodium/MEA Laureth-2 Sulfosuccinate und Tetrasodium Dicarboxyethyl Stearyl Sulfosuccinamate. Noch ein weiteres geeignetes Sulfosuccinamat ist Dinatrium-C₁₆₋₁₈-alkoxypropylensulfosuccinamat.

Eine weitere geeignete Verbindungsklasse sind Aniontensid-Kation-Komplexe, wobei das Kation selbst ursprünglich tensidische Eigenschaften aufweist. Beispiele sind Umesterungsprodukte von LAS-Säure mit Aminen, Aminderivaten, enthaltend eine C-Kette mit > 2 C-Atomen, vorzugsweiese C₁₂-C₁₆. Diese können z.B. am Stickstoff oxidiert sein, d.h. z.B. Umesterung von LAS-Säure mit Aminoxid C₁₂₋₁₄.

Der Gehalt des erfindungsgemäßen Mittels an anionischen Tensiden, vorzugsweise an den genannten anionischen Tensiden, kann in weiten Bereichen variieren, je nachdem welchem Zweck das betreffende Mittel dient. So kann ein erfindungsgemäßes Mittel sehr große Mengen Aniontensid enthalten, vorzugsweise bis zu einer Größenordnung von bis zu 40, 50 oder 60 Gew.- oder mehr. Ebenso kann ein erfindungsgemäßes Mittel nur sehr geringe Mengen Aniontensid enthalten, beispielsweise weniger als 15 oder 10 Gew.-% oder weniger als 5 Gew.-% oder noch weniger. Vorteilhafterweise sind in den erfindungsgemäßen Mitteln jedoch Aniontenside in Mengen von 0,1 bis 40 Gew.-% und insbesondere 5 bis 30 Gew.-% enthalten, wobei Konzentrationen oberhalb von 10 Gew.-% und sogar oberhalb von 15 Gew.-% besondere Bevorzugung finden. Nach einer bevorzugten Ausführungsform enthält das erfindungsgemäße Mittel anionische Tenside, vorzugsweise in Mengen von zumindest 0,01 Gew.-%, bezogen auf das gesamte Mittel. Nach einer anderen bevorzugten Ausführungsform kann das erfindungsgemäße Mittel frei von Aniontensid sein.

Zusätzlich zu den genannten anionischen Tensiden, aber auch unabhängig von diesen, können in den erfindungsgemäßen Mitteln Seifen enthalten sein. Geeignet sind insbesondere gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische. Der Gehalt des Mittels an Seifen beträgt, unabhängig von anderen Aniontensiden, vorzugsweise nicht mehr als 3 Gew.-% und insbesondere 0,5 bis 2,5 Gew.-%, bezogen auf das gesamte Mittel. Nach einer anderen bevorzugten Ausführungsform ist das erfindungsgemäße Mittel frei von Seife.

Die anionischen Tenside und Seifen können in Form ihrer Natrium-, Kalium- oder Ammoniumsalze sowie als lösliche Salze organischer Basen, wie Mono-, Di- oder Triethanol-amin, vorliegen. Vorzugsweise liegen sie in Form ihrer Natrium- oder Kaliumsalze, insbesondere in Form der Natriumsalze vor. Anionische Tenside und Seifen können auch in situ hergestellt werden, indem in die sprühzutrocknende Zusammensetzung die Aniontensidsäuren und gegebenenfalls Fettsäuren eingebracht werden, welche dann durch die Alkaliträger in der sprühzutrocknenden Zusammensetzung neutralisiert werden.

Vorteilhafterweise können nichtionische Tenside in den erfindungsgemäßen Mitteln ebenfalls enthalten sein, sowohl in festen wie in flüssigen Mitteln. Beispielsweise kann ihr Gehalt bis zu 2 oder 3 oder 5 Gew.-% betragen. Es können auch größere Mengen an nichtionischem Tensid enthalten sein, beispielsweise bis zu 5 Gew.-% oder 10 Gew.-% oder 15 Gew.-% oder 20 Gew.-%, 30 Gew.-%, 40 Gew.-%, 50 Gew.-% oder sogar darüber hinaus, falls es zweckmäßig ist. Sinnvolle Untergrenzen können bei Werten von 0,01, 0,1, 1, 2, 3 oder 4 Gew.-% liegen.

Vorzugsweise sind die nichtionischen Tenside aber in größeren Mengen, also bis zu 50 Gew.-%, vorteilhafterweise von 0,1 bis 40 Gew.-%, besonders bevorzugt von 0,5 bis 30 und insbesondere von 2 bis 25 Gew.-%, jeweils bezogen auf das gesamte Mittel, enthalten. Nach einer bevorzugten Ausführungsform enthält das erfindungsgemäße Mittel nichtionische Tenside, vorzugsweise in Mengen von zumindest 0,1 Gew.%, bezogen auf das gesamte Mittel. Nach einer anderen bevorzugten Ausführungsform kann das erfindungsgemäße Mittel frei von Niotensid sein.

Vorteilhafterweise können alle aus dem Stand der Technik bekannten nichtionischen Tenside in den erfindungsgemäßen Mitteln enthalten sein. Bevorzugte Niotenside werden weiter unten vorgestellt.

Die erfindungsgemäßen Hautbehandlungsmittel können vorzugsweise auch mindestens ein kationisches Tensid enthalten. Geeignete Kationtenside sind beispielsweise oberflächenaktive quaternäre Verbindungen, insbesondere mit einer Ammonium-, Sulfonium-, Phosphonium-, Jodonium- oder Arsoniumgruppe. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Besonders bevorzugte kationische Tenside sind die quaternären, z.T. antimikrobiell wirkenden Ammoniumverbindungen (QAV; *INCI* Quaternary Ammonium Compounds) gemäß der allgemeinen Formel (R^{I})(R^{II})(R^{III})(R^{IV})N⁺ X⁻, in der R^{I} bis R^{IV} gleiche oder verschiedene C₁₋₂₂-Alkylreste C₇₋₂₈-Aralkyl-reste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere 12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzylammonium-chlorid, CAS No. 8001-54-5), Benzalkon B (*m*,*p*-Dichlorbenzyl-dimethyl-C₁₂-alkylammoniumchlorid, CAS No. 58390-78-6), Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)phenoxy]ethoxy]ethyl]-benzylammo-niumchlorid, CAS No. 121-54-0), Dialkyldimethylammoniumchloride wie Di-*n*-decyl-dimethyl-ammo-niumchlorid (CAS No. 7173-51-5-5), Didecyldimethylammoniumbromid (CAS No. 2390-68-3), Dioctyl-dimethylammoniumchloric, 1-Cetylpyridiniumchlorid (CAS No. 123-03-5) und Thiazolinjodid (CAS No. 15764-48-1) sowie deren Mischungen. Bevorzugte QAV sind die Benzalkoniumchloride mit C₈-C₁₈-Alkylresten, insbesondere C₁₂-C₁₄-Aklyl-benzyl-dimethylammo-niumchlorid. Eine besonders bevorzugte QAV Kokospentaethoxymethylammoniummethosulfat (*INCI* PEG-5 Cocomonium Methosulfate; Rewoquat^{®} CPEM).

Zur Vermeidung möglicher Inkompatibilitäten der antimikrobiellen kationischen Tenside mit in dem erfindungsgemäßen Mittel enthaltenen anionischen Tensiden werden möglichst aniontensidverträgliches und/oder ggf. möglichst wenig kationisches Tensid eingesetzt oder in einer besonderen Ausführungsform der Erfindung gänzlich auf kationische Tenside verzichtet.

Weiter unten werden insbesondere im Zusammenhang mit Konditioniermitteln und Weichmachern weitere kationische Tenside, so auch quartäre Ammoniumverbindungen beschrieben. Auch diese können vorzugweise in den erfindungsgemäßen Mitteln enthalten sein.

Die erfindungsgemäßen Hautbehandlungsmittel können ein oder mehrere kationische Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Geeignete Mindestwerte können auch bei 0,5, 1, 2 oder 3 Gew.-% liegen. Nach einer bevorzugten Ausführungsform enthält das erfindungsgemäße Mittel kationische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Kationtensid sein.

Ebenso können die erfindungsgemäßen Hautbehandlungsmittel auch mindestens ein amphoteres Tensid enthalten. Diese werden weiter unten insbesondere im Zusammenhang mit Konditioniermitteln und Weichmachern noch näher beschrieben.

Die erfindungsgemäßen Hautbehandlungsmittel können ein oder mehrere amphotere Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Nach einer anderen bevorzugten Ausführungsform kann das erfindungsgemäße Mittel frei von amphoteren Tensiden sein.

Nach einer bestimmten Ausführungsform können die erfindungsgemäßen Mittel nur sehr wenig Gesamttensid enthalten, z.B. kann die Gesamttensidmenge unter 20 Gew.-%, 15 Gew.-%, 10 Gew.-% oder 5 Gew.-%, vorteilhafterweise sogar unter 3 Gew.-% oder unter 1 Gew.-%, insbesondere sogar unter 0,5 Gew.-% oder unter 0,1 Gew.-% liegen, Gew.-% jeweils bezogen auf das gesamte Mittel. Vorzugsweise beträgt der Gesamttensidgehalt aber zumindest 0,01 Gew.-%, 0,1 Gew-% oder 1 Gew.-%, bezogen auf das gesamte Mittel.

Lösliche Komplexbildner kann das erfindungsgemäße Mittel vorzugsweise in Mengen von 0,1 Gew.-% bis 30 Gew.-%, bevorzugt 5 Gew.-% bis 25 Gew.-% und besonders bevorzugt 10 Gew.-% bis 20 Gew.-%, bezogen auf das Gesamtgewicht des Mittel, enthalten, wobei Ethylendiamintetraeesigsäure und ihre Natriumsalze als lösliche Komplexbildner besonders bevorzugt sind. Es kann aber vorteilhafterweise auch vorgesehen sein, dass das erfindungsgemäße Mittel weniger als 10 Gew.-%, beispielsweise weniger als 5 Gew.-% lösliche Komplexbildner enthält. Geeignet sind beispielsweise Citrate, SKS-6, Citronensäure, MGDA (methyl glycine di-acetic acid), Triphosphate, Phosphonate, aliphatische Dicarbonsäuren (z.B. Adipin-, Glutar-, Bernsteinsäure).
Nach einer anderen bevorzugten Ausführungsform kann das erfindungsgemäße Mittel frei von löslichen Komplexbildnern sein.

Weitere geeignete organische Komplexbildner sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol. Ein bevorzugtes Dextrin ist in der britischen Patentanmeldung 94 19 091 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Cobuilder. Dabei wird Ethylendiamin-N,N'-di-succinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen beispielsweise bei 3 bis 15 Gew.-%, bezogen auf das gesamte Mittel.

Weitere brauchbare organische Co-Komplexbildner sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Co-Komplexbildner-Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-Komplexbildner. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Bevorzugte organische Komplexbildner sind beispielsweise die in Form ihrer Alkali- und insbesondere Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer komplexierenden Wirkung typischerweise auch die Eigenschaft einer Säuerungskomponente und dienen somit auch zur Einstellung eines niedrigeren und milderen pH-Wertes bevorzugter erfindungsgemäßer Hautbehandlungsmittel. Insbesondere sind hierbei Citronensäure, Bernsteinsäure, Glutarsäure, Adipinsäure, Gluconsäure und beliebige Mischungen aus diesen zu nennen.

Komplexbildner (INCI Chelating Agents), auch Sequestriermittel genannt, sind Inhaltsstoffe, die Metallionen zu komplexieren und inaktivieren vermögen, beispielsweise um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert die oxidative Zersetzung der fertigen Mittel.

Geeignet sind beispielsweise die folgenden gemäß INCI bezeichneten Komplexbildner, die beispielsweise im International Cosmetic Ingredient Dictionary and Handbook näher beschrieben sind: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphos-phate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Bevorzugte Komplexbildner sind tertiäre Amine, insbesondere tertiäre Alkanolamine (Ami-noalkohole). Die Alkanolamine besitzen sowohl Amino- als auch Hydroxy- und/oder Ether-gruppen als funktionelle Gruppen. Besonders bevorzugte tertiäre Alkanolamine sind Tri-ethanolamin und Tetra-2-hydroxypro-pylethylendiamin (N,N,N',N'-Tetrakis-(2-hydroxy-pro-pyl)ethylendiamin). Besonders bevorzugte Kombinationen tertiärer Amine mit Zinkricinoleat und einem oder mehreren ethoxylierten Fettalkoholen als nichtionische Lösungsvermittler sowie ggf. Lösungsmittel sind im Stand der Technik beschrieben.

Ein besonders bevorzugter Komplexbildner ist die Etidronsäure (1-Hydroxyethyliden-1,1-diphosphonsäure, 1-Hydroxyethyan-1,1-diphosphonsäure, HEDP, Acetophosphonsäure, INCI Etidronic Acid) einschließlich ihrer Salze. In einer bevorzugten Ausführungsform enthält das erfindungsgemäße Mittel demgemäß als Komplexbildner Etidronsäure und/oder eines oder mehrere ihrer Salze.

In einer besonders bevorzugten Ausführungsform enthält das erfindungsgemäße Mittel eine Komplexbildnerkombination aus einem oder mehreren tertiären Aminen und einer oder mehreren weiteren Komplexbildnern, vorzugsweise einer oder mehreren Komplexbildnersäuren oder deren Salzen, insbesondere aus Triethanolamin und/oder Tetra-2-hydroxypropylethylendiamin und Etidronsäure und/oder einem oder mehrerer ihrer Salze.

Das erfindungsgemäße Mittel enthält bevorzugt mindestens einen Komplexbildner in einer Gesamtmenge von üblicherweise 0 bis 20 Gew.-%, vorzugsweise 0,1 bis 15 Gew.-%, insbesondere 0,5 bis 10 Gew.-%, besonders bevorzugt 1 bis 8 Gew.-%, äußerst bevorzugt 1,5 bis 6 Gew.-%, bezogen auf das gesamte Mittel.

Es sei an dieser Stelle darauf hingewiesen, dass sich die Angabe Gew.-%, sofern es nicht anders angegeben ist, jeweils auf das gesamte Mittel bezieht.

Der Gehalt an Wasser in bevorzugten erfindungsgemäßen Mitteln richtet sich u.a. danach, ob das Mittel in flüssiger oder fester Form vorliegt, beträgt daher vorzugsweise 0 bis weniger als 100 Gew.-% und insbesondere 0,5 bis 95 Gew.-%, wobei Werte von maximal 5 Gew.-% insbesondere bei festen oder nichtwässrigen flüssigen Mitteln besondere Bevorzugung finden. Nicht miteingerechnet wurde hierbei das in einzelnen Rohstoffen, wie insbesondere den schweißhemmenden Aluminium- und/ode Zirconium-Verbindungen, vorhandene Kristallwasser.

Im Falle flüssiger Mittel enthält das erfindungsgemäße Mittel nach einer bevorzugten Ausführungsform Wasser in einer Menge von mehr als 20 Gew.-%, vorteilhafterweise mehr als 30 Gew.-%., in weiter vorteilhafter Weise mehr als 40 Gew.-%, noch vorteilhafter mehr als 50 Gew.-%, insbesondere 60 bis 99 Gew.-%, besonders bevorzugt 70 bis 98 Gew.-% und äußerst bevorzugt 80 bis 95 Gew.-%, bezogen auf das gesamte Mittel.

Die Obergrenze an Wasser kann auch bei 80 Gew.-%, 70 Gew.-%, 60 Gew.-%, 50 Gew.-%, 40 Gew.-%, 30 Gew.-%, 20 Gew.-% oder 10 Gew.-% Gew.-% liegen, bezogen auf das gesamte Mittel.

Die Untergrenze an Wasser kann z.B. auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel.

Die genannten Ober- und Untergrenzen können natürlich sinnvoll kombiniert werden, z.B. zu Wassergehalten von 60-80 Gew.-% oder 10-30 Gew.-% usw.

Liegt das erfindungsgemäße Mittel in partikulärer Form vor, beispielsweise als Anti-Akne-Puder oder als mattierender Puder, so können die Partikel nachbehandelt werden, beispielsweise indem man die Partikel des Mittels verrundet. Die Verrundung kann in einem üblichen Verrunder erfolgen. Vorzugsweise beträgt die Verrundungszeit dabei nicht länger als 4 Minuten, insbesondere nicht länger als 3,5 Minuten. Verrundungszeiten von maximal 1,5 Minuten oder darunter sind insbesondere bevorzugt. Durch die Verrundung wird eine weitere Vereinheitlichung des Kornspektrums erreicht, da gegebenenfalls entstandene Agglomerate zerkleinert werden.

Ein erfindungsgemäßes Mittel in Partikelform kann man insbesondere mit nichtionischen Tensiden, Parfümöl und/oder Schauminhibitoren bzw. Zubereitungsformen, welche diese Inhaltsstoffe enthalten, vorzugsweise mit Mengen bis zu 20 Gew.-% Aktivsubstanz, insbesondere mit Mengen von 2 bis 18 Gew.-% Aktivsubstanz, jeweils bezogen auf das nachbehandelte Produkt, in an sich üblicher Weise, vorzugsweise in einem Mischer oder ggf. einer Wirbelschicht, nachbehandeln.

Insbesondere kann ein erfindungsgemäßes Mittel ebenfalls mit Feststoffen, vorzugsweise in Mengen bis zu 15 Gew.-%, insbesondere in Mengen von 2 bis 15 Gew.-%, jeweils bezogen auf das Gesamtgewicht des nachbehandelten Mittels, nachbehandelt bzw. abgepudert werden.

Als Feststoffe zur Nachbehandlung lassen sich vorzugsweise Bicarbonat, Carbonat, Zeolith, Kieselsäure, Citrat, Harnstoff oder Mischungen aus diesen, insbesondere in Mengen von 2 bis 15 Gew.-%, bezogen auf das Gesamtgewicht des nachbehandelten Produkts, verwenden. Die Nachbehandlung lässt sich in vorteilhafter Weise in einem Mischer und/oder mittels Verrunder durchführen.

In einer weiteren bevorzugten Ausführungsform enthält das erfindungsgemäße Hautbehandlungsmittel mindestens eine UV-absorbierende Substanz enthalten. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzyliden)campher; 4-Aminobenzoesäure-derivate, vorzugsweise 4-(Di-methylamino)benzoesäure-2-ethylhexylester, 4-(Dimethyl-amino)benzoe-säure-2-octylester und 4-(Dimethylamino)benzoesäureamylester; Ester der Zimtsäure, vorzugsweise 4-Meth-oxyzimtsäure-2-ethylhexylester, 4-Methoxyzimtsäurepropylester, 4-Methoxyzimtsäureiso-amyl-ester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzyl-ester, Salicylsäurehomomenthylester; Derivate des Benzophenons, vorzugsweise 2-Hy-droxy-4-methoxybenzophenon, 2-Hydroxy-4-methoxy-4'-methylbenzophenon, 2,2'-Dihy-droxy-4-methoxybenzophenon; Ester der Benzalmalonsäure, vorzugsweise 4-Methoxy-benzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb^{®} HEB); Propan-1,3-dione, wie z.B. 1-(4-tert.Butylphenyl)-3-(4'methoxyphenyl)propan-1,3-dion; Ketotricyclo(5.2.1.0)decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hy-droxy-4-methoxybenzophenon-5-sulfon-säure und ihre Salze; Sulfonsäurederivate des 3-Benzylidencamphers, wie z.B. 4-(2-Oxo-3-bor-nylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zink-stearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise von 5 bis 50 nm und insbesondere von 15 bis 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Ebenfalls bevorzugt kann mikronisiertes Zinkoxid verwendet werden. Weitere geeignete UV-Lichtschutzfilter sind dem einschlägigen Stand der Technik zu entnehmen.

Die mindestens eine UV-absorbierende Substanz ist in bevorzugten erfindungsgemäßen Hautbehandlungsmitteln in einer Gesamtmenge von 0,01 Gew.-% bis 20 Gew.-%, besonders bevorzugt 1 - 10 Gew.%, außerordentlich bevorzugt 2 Gew.-% bis 7 Gew.-%, enthalten.

In einer weiteren bevorzugten Ausführungsform liegen die erfindungsgemäßen Mittel in flüssiger Form vor. Zum Erreichen einer flüssigen Konsistenz kann der Einsatz sowohl flüssiger organischer Lösungsmittel, wie auch der von Wasser angezeigt sein. Bevorzugte erfindungsgemäße Mittel enthalten daher gegebenenfalls mindestens ein Lösungsmittel.

Lösungsmittel, die in bevorzugten erfindungsgemäßen Mitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Glycol, Propan- oder Butandiol, Glycerin, Diglyol, Propyl- oder Butyldiglycol, Hexylenglycol, Ethylenglycolmethylether, Ethylenglycolethylether, Ethylen-glycolpropylether, Ethylenglycolmono-n-butylether, Diethylenglycol-methylether, Diethylenglycolethylether, Propylenglycolmethyl-, -ethyl- oder -propyl-ether, Butoxy-propoxy-propanol (BPP), Dipropylenglycolmonomethyl-, oder -ethylether, Di-isopropylenglycol-monomethyl-, oder -ethylether, Methoxy-, Ethoxy- oder Butoxytriglycol, 1-Butoxyethoxy-2-propanol, 3-Methyl-3-methoxybutanol, Propylen-glycol-t-butylether sowie Mischungen dieser Lösungsmittel.

Einige Glycolether sind unter den Handelsnamen Arcosolv^{®} (Arco Chemical Co.) oder Cellosolve^{®}, Carbitol^{®} oder Propasol^{®} (Union Carbide Corp.) erhältlich; dazu gehören auch z.B. ButylCarbitol^{®}, HexylCarbitol^{®}, MethylCarbitol^{®}, und Carbitol^{®} selbst, (2-(2-Ethoxy)ethoxy)ethanol. Die Wahl des Glycolethers kann vom Fachmann leicht auf der Basis seiner Flüchtigkeit, Wasserlöslichkeit, seines Gewichtsprozentanteils an der gesamten Dispersion und dergleichen getroffen werden. Pyrrolidon-Lösungsmittel, wie N-Alkyl-pyrrolidone, beispielsweise N-Methyl-2-pyrrolidon oder N-C₈-C₁₂-Alkyl-pyrrolidon, oder 2-Pyrrolidon, können ebenfalls eingesetzt werden. Weiterhin bevorzugt als alleinige Lösungsmittel oder als Bestandteil eines Lösungsmittelgemisches sind Glycerinderivate, insbesondere Glycerincarbonat.

Zu den Alkoholen, die in der vorliegenden Erfindung vorzugsweise als Cosolventien eingesetzt werden können, gehören flüssige Polyethylenglycole, mit niederem Molekulargewicht, beispielsweise Polyethylenglycole mit einem Molekulargewicht von 200, 300, 400 oder 600. Weitere geeignete Cosolventien sind andere Alkohole, zum Beispiel (a) niedere Alkohole wie Ethanol, Propanol, Isopropanol und n-Butanol, (b) Ketone wie Aceton und Methylethylketon, (c) C₂-C₄-Polyole wie ein Diol oder ein Triol, beispielsweise Ethylenglycol, Propylenglycol, Glycerin oder Gemische davon. Insbesondere bevorzugt ist aus der Klasse der Diole 1,2-Octandiol.

In einer bevorzugten Ausführungsform enthält das erfindungsgemäße Mittel ein oder mehrere Lösungsmittel aus der Gruppe, umfassend C₁- bis C₄-Monoalko-hole, C₂- bis C₆-Glycole, C₃- bis C₁₂-Glycolether und Glycerin, insbesondere Ethanol. Die erfindungsgemäßen C₃- bis C₁₂-Glycolether enthalten Alkyl- bzw. Alkenylgruppen mit weniger als 10 Kohlenstoffatomen, vorzugsweise bis zu 8, insbesondere bis zu 6, besonders bevorzugt 1 bis 4 und äußerst bevorzugt 2 bis 3 Kohlenstoffatomen.

Bevorzugte C₁- bis C₄-Monoalkohole sind Ethanol, *n*-Propanol, *iso*-Propanol und *tert*-Butanol. Bevorzugte C₂- bis C₆-Glycole sind Ethylenglycol, 1,2-Propylenglycol, 1,3-Propylenglycol, 1,5-Pentandiol, Neopentylglycol und 1,6-Hexandiol, insbesondere Ethylenglycol und 1,2-Propylenglycol. Bevorzugte C₃- bis C₁₂-Glycolether sind Di-, Tri-, Tetra- und Pentaethylenglycol, Di-, Tri-und Tetrapropylenglycol, Propylenglycolmonotertiärbutylether und Propylenglycolmonoethylether sowie die gemäß INCI bezeichneten Lösungsmittel Butoxydiglycol, Butoxyethanol, Butoxyisopropanol, Butoxypropanol, Butyloctanol, Ethoxydiglycol, Ethoxyethanol, Ethyl Hexanediol, Isobutoxypropanol, Isopentyldiol, 3-Methoxybutanol, Methoxyethanol, Methoxyisopropanol und Methoxymethylbutanol.

Bevorzugte erfindungsgemäße Mittel enthalten ein oder mehrere Lösungsmittel in einer Gesamtmenge von üblicherweise bis zu 90 Gew.-%, vorzugsweise 0,1 bis 30 Gew.-%, insbesondere 2 bis 20 Gew.-%, besonders bevorzugt 3 bis 15 Gew.-%, äußerst bevorzugt 5 bis 12 Gew.-%, beispielsweise 5,3 oder 10,6 Gew.-%, jeweils bezogen auf das gesamte Mittel.

In einer weiteren bevorzugten Ausführungsform enthält das erfindungsgemäße Mittel mindestens einen Viskositätsregulator, der vorzugsweise als Verdicker fungiert.

Die Viskosität der erfindungsgemäßen Mittel kann mit üblichen Standardmethoden (beispielsweise Brookfield-Viskosimeter RVD-VII bei 20 U/min und 20°C, Spindel 3) gemessen werden und liegt vorzugsweise im Bereich von 10 bis 5000 mPas. Bevorzugte flüssige bis gelförmige Mittel haben Viskositäten von 20 bis 4000 mPas, wobei Werte zwischen 40 und 2000 mPas besonders bevorzugt sind.

Geeignete Verdicker sind anorganische oder polymere organische Verbindungen. Es können auch Gemische aus mehreren Verdickern eingesetzt werden.

Zu den anorganischen Verdickern zählen beispielsweise Polykieselsäuren, Tonmineralien wie Montmorillonite, Zeolithe, Kieselsäuern, Aluminiumsilikate, Schichtsilikate und Bentonite.

Die organischen Verdicker stammen aus den Gruppen der natürlichen Polymere, der abgewandelten natürlichen Polymere und der vollsynthetischen Polymere.

Aus der Natur stammende Polymere, die als Verdicker Verwendung finden, sind beispiels-weise Xanthan, Agar-Agar, Carrageen, Tragant, Gummi arabicum, Alginate, Pektine, Polyosen, Guar-Mehl, Gellan-Gum, Johannisbrotbaumkernmehl, Stärke, Dextrine, Gelatine und Casein.

Abgewandelte Naturstoffe stammen vor allem aus der Gruppe der modifizierten Stärken und Cellulosen, beispielhaft seien hier Carboxymethylcellulose und andere Celluloseether, Hydroxyethyl- und - propylcellulose, hochveretherte Methylhydroxyethylcellulose sowie Kernmehlether genannt.

Eine große Gruppe von Verdickern, die breite Verwendung in den unterschiedlichsten Anwendungsgebieten finden, sind die vollsynthetischen Polymere wie Polyacryl- und Polymethacryl-Verbindungen, die vernetzt oder unvernetzt und ggf. kationisch modifiziert sein können, Vinylpolymere, Polycarbonsäuren, Polyether, aktivierte Polyamidderivate, Rizinusölderivate, Polyimine, Polyamide und Polyurethane. Beispiele für derartige Polymer sind Acrylharze, Ethylacrylat-Acrylamid-Copolymere, Acrylsäureester-Methacrylsäure-ester-Copolymere, Ethylacrylat-Acrylsäure-Methacrylsäure-Copolymere, N-Methylolmeth-acrylamid, Maleinsäureanhydrid-Methylvinylether-Copolymere, Polyether-Polyol-Copolymere sowie Butadien-Styrol-Copolymere.

Weitere geeignete Verdicker sind Derivate organischer Säuren sowie deren Alkoxid-Addukte, beispielsweise Arylpolyglycolether, carboxylierte Nonylphenolethoxylatderivate, Natriumalginat, Diglycerinmonoisostearat, Nichtionogene Ethylenoxid-Addukte, Kokosfettsäurediethanolamid, Isododecenylbernsteinsäureanhydrid sowie Galactomannan. Verdicker aus den genannten Substanzklassen sind kommerziell erhältlich und werden beispielsweise unter den Handelsnamen Acusol®-820 (Methacrylsäure(stearylalkohol-20-EO)ester-Acrylsäure-Copolymer, 30%ig in Wasser, Rohm & Haas), Dapral®-GT-282-S (Alkylpolyglycolether, Akzo), Deuterol®-Polymer-11 (Dicarbonsäure-Co-polymer, Schöner GmbH), Deuteron®-XG (anionisches Heteropolysaccharid auf Basis von β-D-Glucose, D-Mannose, D-Glucuronsäure, Schöner GmbH), Deuteron®-XN (nicht-ionogenes Polysaccharid, Schöner GmbH), Dicrylan®-Verdicker-O (Ethylenoxid-Addukt, 50%ig in Wasser/Isopropanol, Pfersse Chemie), EMA®-81 und EMA®-91 (Ethylen-Maleinsäureanhydrid-Copolymer, Monsanto), Verdicker-QR-1001 (Polyurethan-Emulsion, 19-21%ig in Wasser/Diglycolether, Rohm & Haas), Mirox®-AM (anionische Acrylsäure-Acrylsäureester-Copolymer-Dispersion, 25%ig in Wasser, Stockhausen), SER-AD-FX-1100 (hydrophobes Urethanpolymer, Servo Delden), Shellflo®-S (hoch-molekulares Polysaccharid, mit Formaldehyd stabilisiert, Shell), Shellflo®-XA (Xanthan-Biopolymer, mit Formaldehyd stabilisiert, Shell), Kelzan, Keltrol T (Kelco) angeboten.

Farbstoffe können optional im erfindungsgemäßen Mittel eingesetzt werden, wobei die Menge an einem oder mehreren Farbstoffen so gering zu wählen ist, dass nach der Anwendung des Mittels keine sichtbaren Rückstände verbleiben. Vorzugsweise ist das erfindungsgemäße Mittel frei von Farbstoffen.

Das erfindungsgemäße Mittel kann vorzugsweise einen oder mehrere antimikrobielle Wirkstoffe bzw. Konservierungsmittel in einer Menge von üblicherweise 0,0001 bis 3 Gew.%, vorzugsweise 0,0001 bis 2 Gew.-%, insbesondere 0,0002 bis 1 Gew.%, besonders bevorzugt 0,0002 bis 0,2 Gew.-%, äußerst bevorzugt 0,0003 bis 0,1 Gew.%, enthalten.

Antimikrobielle Wirkstoffe bzw. Konservierungsmittel unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung. Geeignete antimikrobielle Wirkstoffe sind vorzugsweise ausgewählt aus den Gruppen der Alkohole, Amine, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-di-cyanobutan, lodo-2-propyl-butyl-carbamat, Iod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Pro-panol, 1,3-Butandiol, Phenoxyethanol, 1,2-Propylenglycol, Glycerin, Undecylensäure, Benzoesäure, Salicylsäure, Dihydracetsäure, o-Phenylphenol, N-Methylmorpholin-aceto-nitril (MMA), 2-Benzyl-4-chlorphenol, 2,2'-Methylen-bis-(6-brom-4-chlorphenol), 4,4'-Di-chlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguani-dinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydro-chlorid, 1,6-Di-(N₁,N₁'-o-chlorophenyldiguanido- N₅,N₅')-hexan-dihydrochlorid, 1,6-Di-(N₁,N₁'-2,6-dichlorophe- nyldiguanido-N₅,N₅')hexan-dihydrochlorid, 1,6-Di-[N₁,N₁'-beta-(p-methoxyphenyl) diguanido-N₅,N₅']-hexane-dihy-drochlorid, 1,6-Di-(N₁,N₁'-alpha-methyl-.beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-( N₁,N₁'- phenyldiguanido-N₅,N₅')-di-n-propylether-dihydrochlorid, omega:omega'-Di-(N₁,N₁'-p-chlorophe-nyldguanido-N₅,N₅')-di-n-propylether-tetrahydrochlorid, 1,6-Di-(N₁,N₁'-2,4-dichlorophenyidiguanido-N₅,N₅')hexan-tetrahydrochlorid, 1,6-Di-(N₁,N₁'-p-methylphe-nyldiguanido-N₅,N₅')hexan-dihydrochlorid, 1,6-Di-(N₁,N₁'-2,4,5-trichlorophenyldi-guanido-N₅,N₅')hexan-tetrahydrochtorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophe-nyldiguanido-N₅,N₅')m-xylene-dihydrochlorid, 1,12-Di-(N₁,N₁'-p-chlorophenyldiguanido-N₅,N₅') dodecandihydro-chlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecan-tetrahydrochlorid, 1,6-Di-(N₁,N₁'-o-chlorophenyldi-guanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbi-guanid), Ethylen-bis-(N-butylphenylbi-guanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylenbis(o-tolylbiguanid), N-Butyl-trimethyle-bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormetaxylol, sowie natürliche antimikrobielle Wirkstoffe pflanzlicher Herkunft (z.B. aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/ oder mindestens ein natürlicher antimikrobieller Wirkstoff tierischer Herkunft aus der Gruppe, umfassend Enzyme wie Eiweiß aus Milch, Lysozym und Lactoperoxidase, und/ oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden. Bevorzugt werden Glycin, Glycinderivate, Formaldehyd, Verbindungen, die leicht Formaldehyd abspalten, Ameisensäure und Peroxide verwendet.

Die als antimikrobielle Wirkstoffe geeigneten quaternären Ammoniumverbindungen (QAV) sind oben schon beschrieben worden. Besonders geeignet ist beispielsweise Benzalkoniumchlorid etc. Benzalkoniumhalogenide und/ oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Weitere bevorzugte erfindungsgemäße Zusammensetzungen sind dadurch gekennzeichnet, dass zur Verbesserung der Hautfeuchtigkeit unter Verzicht auf komedogene Substanzen mindestens ein alkyl- oder hydroxyalkylsubstituierten Harnstoff der allgemeinen Formel (A) enthalten ist, 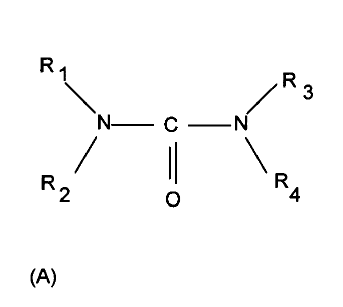 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.
Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-HydroxyalkylGruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-HydroxyalkylGruppen substituiert ist, darstellt.
Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (A) ausgewählt ist aus Bis-N,N'- (2-hydroxyethyl)harnstoff.
Erfindungsgemäß besonders bevorzugte Zusammensetzungen sind dadurch gekennzeichnet, dass sie mindestens einen alkyl- oder hydroxyalkylsubstituierten Harnstoff gemäß Formel (A), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance der National Starch and Chemical Company als ca. 45 - 55 %ige wässrige Lösung mit einem Gehalt an Harnstoff und Ammoniumlactat erhältlich.

In einer bevorzugten Ausführungsform enthalten die erfindungsgemäßen Zusammensetzungen mindestens einen weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.
Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.
Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.
ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).
Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8-18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.
Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist.

Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.
Proteinhydrolysate können naturgemäß auch monomere Aminosäuren und Oligopeptide enthalten; ihre Zusammensetzung ist normalerweise nicht definiert.
Ebenfalls bevorzugt ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Ein weiterer erfindungsgemäß bevorzugter Wirkstoff, dessen Wirkung in Gegenwart des erfindungsgemäß verwendeten, die AQP-Synthese-stimulierenden Wirkstoffs, insbesondere Ergothionein, verbessert wird, ist das Peptidderivat L-Glutamylaminoethyl-indol (erhältlich z. B. unter dem Handelsnamen Glistin von Exsymol).

Erfindungsgemäß bevorzugt sind auch kationisierte Proteinhydrolysate. Besondes bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome^{™}, liposomenverkapselte T4N5 z. B. unter der Bezeichnung Ultrasome^{™} von der Firma AGI Dermatics, USA, erhältlich.
Erfindungsgemäß besonders bevorzugte Zusammensetzungen sind dadurch gekennzeichnet, dass sie mindestens eines der Handelsprodukte Photosomes^{™} oder Ultrasomes^{™} in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung, enthalten.
Erfindungsgemäß besonders bevorzugte Zusammensetzungen sind dadurch gekennzeichnet, dass sie mindestens ein Monomer, Oligomer oder Polymer von Aminosäuren, N-C₂-C₂₄-Acylaminosäuren und/oder den Estern und/ oder den physiologisch verträglichen Metallsalzen dieser Substanzen in Gesamtmengen von 0,0000001 - 10 Gew.-%, bevorzugt 0,001 - 5 Gew.-% und besonders bevorzugt 0,01 - 1 - 2 - 3 Gew.-%, jeweils bezogen auf den Aktivsubstanzgehalt in der gesamten erfindungsgemäßen Zusammensetzung, enthalten.

In einer weiteren bevorzugten Ausführungsform enthalten die erfindungsgemäßen Zusammensetzungen mindestens ein DNA-Oligonucleotid oder mindestens ein RNA-Oligonucleotid.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).
Ein erfindungsgemäß besonders bevorzugtes Oligonucleotid ist das Thymidin-Dinucleotid. Erfindungsgemäß besonders bevorzugte Zusammensetzungen sind dadurch gekennzeichnet, dass sie mindestens ein DNA-Oligonucleotid und/oder ein RNA-Oligonucleotid in Gesamtmengen von 0,000001 - 5 Gew.-%, bevorzugt 0,0001 - 0,5 Gew.-% und besonders bevorzugt 0,001 - 0,05 Gew.-%, bezogen auf die gesamte Zusammensetzung, enthalten.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).
Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.%, bevorzugt 0,1 bis 3 Gew.-%, besonders bevorzugt 0,5 bis 2 Gew.%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt.

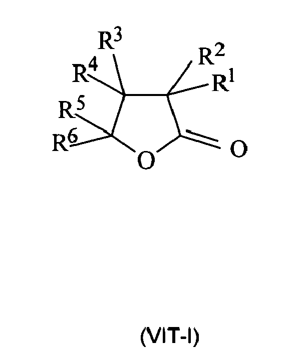

Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Erfindungsgemäß besonders bevorzugte Zusammensetzungen sind dadurch gekennzeichnet, dass sie mindestens eine der genannten Verbindungen des Vitamin B₅-Typs sowie der 2-Furanonderivate in einer Gesamtmenge von 0,05 bis 5 Gew.%, bevorzugt 0,1 bis 3 Gew.-%, besonders bevorzugt 0,5 bis 2 Gew.%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton.

Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.%, bevorzugt 0,1 bis 3 Gew.%, besonders bevorzugt 0,5 bis 1 - 2 Gew.-%, jeweils bezogen auf die gesamte Zusammensetzung, enthalten.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Erfindungsgemäß besonders bevorzugte Zusammensetzungen sind dadurch gekennzeichnet, dass sie mindestens ein Vitamin K in einer Gesamtmenge von 0001 bis 1,0 Gew.-%, bevorzugt 0,05 bis 0,01 Gew.-%, besonders bevorzugt 0,1 bis 0,5 Gew.-%, jeweils bezogen auf die gesamte Zusammensetzung, enthalten.

Vitamin A-palmitat (Retinylpalmitat), Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5-1-2 Gew.-%. jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.
Erfindungsgemäß besonders bevorzugte Zusammensetzungen sind dadurch gekennzeichnet, dass sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte Zusammensetzungen sind dadurch gekennzeichnet, dass sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 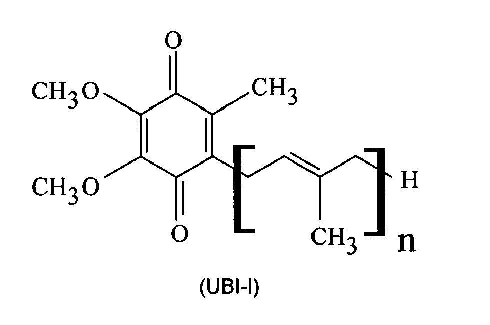 mit n = 6, 7, 8, 9 oder 10.
Besonders bevorzugt ist das Ubichinon der Formel (II) mit n = 10, auch bekannt als Coenzym Q10.
Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß besonders bevorzugte Zusammensetzungen sind dadurch gekennzeichnet, dass sie Silymarin in Mengen von 0,00001 bis 1 Gew.-%, bevorzugt 0,0001 bis 0,01 Gew.-% und besonders bevorzugt 0,005 bis 0,1 Gew.%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für *N*-Methyl-guanidino-essigsäure bzw. *N*-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.
Erfindungsgemäß besonders bevorzugte Zusammensetzungen sind dadurch gekennzeichnet, dass sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten.

Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure, Oleanol und/oder Oleuropein. Erfindungsgemäß besonders bevorzugte Zusammensetzungen sind dadurch gekennzeichnet, dass sie Oleanolsäure, Oleanol und/oder Oleuropein in einer Gesamtmenge von 0,00001 bis 2 Gew.%, bevorzugt 0,001 bis 1 Gew.-% und besonders bevorzugt 0,05 bis 0,1 Gew.%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.
Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®, Exsymol)
- ascorbylmethylsilanol pectinate (Ascorbosilane C®, Exsymol)
- dimethyl oxobenzodioxsilane (DSBC®, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsifanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C®, Exsymol).
Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Zusammensetzung.

In einer weiteren bevorzugten Ausführungsform enthalten die erfindungsgemäßen Zusammensetzungen Phytinsäure. Erfindungsgemäß besonders bevorzugte kosmetische oder dermatologische Zusammensetzungen sind dadurch gekennzeichnet, dass sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Zusammensetzung.

In einer weiteren bevorzugten Ausführungsform enthalten die erfindungsgemäßen Zusammensetzungen mindestens einen Extrakt aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich. Dieser Extrakt erhöht und/oder verbessert die Interaktion zwischen der extrazellulären Matrix und den Fibroblasten.
Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Maiskörnern (Zea Mays (Corn) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Maiskörnern (Zea Mays (Corn) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich. Dieser Extrakt erhöht und/oder verbessert die Interaktion zwischen der extrazellulären Matrix und den Fibroblasten.
Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Haferkörnern (Avena Sativa (Oat) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Haferkörnern (Avena Sativa (Oat) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Produkt, das durch Fermentation von gezuckertem Schwarztee mit den zwei symbiotischen Mikroorganismen Saccharomyces und Acetobacter ylinum gewonnen wird, als Kombucha bezeichnet wird und die INCI-Bezeichnung Saccharomyces/ Xylinum/Black Tea Ferment trägt. Derartige Produkte erhöhen und/oder verbessern die Interaktion zwischen der extrazellulären Matrix und den Fibroblasten. Ein besonders bevorzugtes Produkt ist unter dem Handelsnamen Kombuchka von der Firma Sederma erhältlich (INCI-Bezeichnung: Saccharomyces/ Xylinum/Black Tea Ferment, Glycerin, Hydroxyethylcellulose).
Erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Xylinum gewonnen werden und die INCI-Bezeichnung Saccharomyces/Xylinum/ Black Tea Ferment tragen, in einer Gesamtmenge von 0,01 - 5 Gew.-%, bevorzugt 0,1 - 3 Gew.-%, besonders bevorzugt 1-2 Gew.-% enthalten, jeweils bezogen auf den Gehalt an Produkt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Acetobacter Xylinum gewonnen werden und die INCI-Bezeichnung Saccharomyces/Xylinum/Black Tea Ferment tragen, in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Apfelkernextrakten (Pyrus Malus (Apple) Fruit Extract). Derartige Extrakte erhöhen und/oder verbessern die Interaktion zwischen der extrazellulären Matrix und den Fibroblasten. Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.
Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,00001 - 2 Gew.-%, bevorzugt 0,001 - 1,6 Gew.-% und besonders bevorzugt 0,03 - 1 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Lotuskeimen (Nelumbo Nucifera Germ Extract). Derartige Extrakte erhöhen und/oder verbessern die Interaktion zwischen der extrazellulären Matrix und den Fibroblasten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.
Erfindungsgemäß besonders bevorzugte kosmetische oder dermatologische Zusammensetzungen sind dadurch gekennzeichnet, dass sie mindestens einen Extrakt aus Lotuskeimen (Nelumbo Nucifera Germ Extract) in Mengen von 0,1-10 Gew.-%, bevorzugt 1-8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Lotuskeimen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Rotwein. Derartige Extrakte erhöhen und/ oder verbessern die Interaktion zwischen der extrazellulären Matrix und den Fibroblasten. Ein erfindungsgemäß besonders bevorzugter Rotweinextrakt ist unter dem Handelsnamen Sepivinol R von der Firma Seppic erhältlich.
Erfindungsgemäß besonders bevorzugte kosmetische oder dermatologische Zusammensetzungen sind dadurch gekennzeichnet, dass sie mindestens einen Extrakt aus Rotwein in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Rotwein in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Traubenkernen (Vitis Vinifera (Grape) Seed Extract). Derartige Extrakte erhöhen und/oder verbessern die Interaktion zwischen der extrazellulären Matrix und den Fibroblasten. Besonders bevorzugt stammen die Traubenkernextrakte aus der Chardonnay-Traube. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.
Erfindungsgemäß besonders bevorzugte kosmetische oder dermatologische Zusammensetzungen sind dadurch gekennzeichnet, dass sie mindestens einen Extrakt aus Traubenkernen in Mengen von 0,1 - 10 Gew.%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Traubenkernen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Schwarzen Holunderblüten (Sambucus Nigra Flower Extract). Derartige Extrakte erhöhen und/oder verbessern die Interaktion zwischen der extrazellulären Matrix und den Fibroblasten. Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.
Erfindungsgemäß besonders bevorzugte kosmetische oder dermatologische Zusammensetzungen sind dadurch gekennzeichnet, dass sie mindestens einen Extrakt aus Schwarzen Holunderblüten in Mengen von 0,1 - 10 Gew.-%, bevorzugt 1-5 Gew.-% und besonders bevorzugt 2-3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Schwarzen Holunderblüten in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Wirkstoff, der die beta-Endorphinsynthese in Keratinozyten stimuliert.
Erfindungsgemäß besonders bevorzugte Stimulatoren der beta-Endorphin-Synthese sind ausgewählt aus Mischungen aus mindestens einem Extrakt aus den Blättern der Mentha piperita und mindestens einem Extrakt aus Kakaobohnen, wobei wässrige, glycolische oder wässrig-glycolische Zubereitungen dieser Extraktmischungen, die unter den Handelsbezeichnungen Caomint, Caophenol, Caobromine, Caospice und Caoorange von der Firma Solabia erhältlich sind, besonders bevorzugt sind. Ein weiterer besonders bevorzugter Stimulator der beta-Endorphin-Synthese ist das Dipeptidderivat N-Acetyl-Tyr-Arg-hexyldecylester mit der INCI-Bezeichnung Acetyl Dipeptide-1 Cetyl Ester, das z. B. als wässrige Zubereitung unter der Handelsbezeichnung Calmosensine von der Firma Sederma erhältlich ist.
Weitere bevorzugte Stimulatoren der beta-Endorphin-Synthese sind Extrakte aus Helichrysum italicum, z. B. erhältlich unter der Handelsbezeichnung Areaumat Perpetua von der Firma Codif, Extrakte aus Crithmum Maritimum, z. B. erhältlich unter den Handelsbezeichnungen Areaumat Samphira und Aroleat Samphira von der Firma Codif, Extrakte aus Lavendula stoechas, z. B. erhältlich unter der Handelsbezeichnung Areaumat Lavanda von der Firma Codif, Extrakte aus Mentha piperita, wie sie z. B. unter den Handelsbezeichnungen Authenticals of Peppermint (Solabia) und Calmiskin (Silab) erhältlich sind, Glutamylamidoethyl Indole, z. B. erhältlich unter der Handelsbezeichnung Glistin von der Firma Exsymol, ein durch mikrobielle Fermentation gewonnenes verzweigtes Polysaccharid mit Rhamnose-, Galactose- und Glucuronsäure-Einheiten mit der INCI-Bezeichnung Biosaccharide Gum-2, z. B. erhältlich unter der Handelsbezeichnung Rhamnosoft von der Firma Solabia, Extrakte aus den Samen von Tephrosia Purpurea mit der INCI-Bezeichnung Tephrosia Purpurea Seed Extract, z. B. erhältlich unter der Handelsbezeichnung Tephroline von der Firma Vincience, Mischungen aus dem Öl von Mentha arvensis-Blättern, Limonenschalenöl, Zypressenöl, Lavendelöl und Cistus Ladaniferus-Öl mit der INCI-Bezeichnung Mentha Arvensis Leaf Oil and Citrus Medica Limonum (Lemon) Peel Oil and Cupressus Sempervirens Oil and Lavandula Hybrida Oil and Cistus Ladaniferus Oil, z. B. erhältlich unter der Handelsbezeichnung V-Tonic (Gattefossé), und Hexasaccharide gemäß FR 2842201 sowie beliebige Mischungen dieser Wirkstoffe.
Erfindungsgemäß besonders bevorzugte kosmetische oder dermatologische Zusammensetzungen sind dadurch gekennzeichnet, dass sie mindestens einen Wirkstoff zur Stimulation der beta-Endorphin-Synthese in Gesamtmengen von 0,01 - 10 Gew.-%, bevorzugt 0,1 - 5 Gew.-% und besonders bevorzugt 1 - 3 Gew.-%, jeweils bezogen auf das Handelsprodukt, das den Wirkstoff enthält, in der gesamten erfindungsgemäßen Zusammensetzung, enthalten. Erfindungsgemäß besonders bevorzugte kosmetische oder dermatologische Zusammensetzungen sind dadurch gekennzeichnet, dass sie mindestens einen Wirkstoff zur Stimulation der beta-Endorphin-Synthese in Gesamtmengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten erfindungsgemäßen Zusammensetzung, enthalten.

In einer weiteren bevorzugten Ausführungsform enthalten die erfindungsgemäßen Zusammensetzungen zur Unterstützung der Wirkung des erfindungsgemäß verwendeten Wirkstoffs, insbesondere Ergothionein, mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin(salzen), Gerbsäure(salzen) und/oder Gallotanninen und Naringin, wie sie zum Beispiel als Handelsprodukt BiSCos Glynarin PF (INCI: Aqua (Water), Alacohol, Ohenoxyethanol, Ammonium Glycyrrhizate, Tannic Acid, Naringine) von der Firma Biesterfeld erhältlich sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques), Hefeproteinhydrolysaten, wie sie z.B. in den Handelsprodukten der Asebiol^{®}-Serie von Laboratoires Sérobiologiques erhältlich sind, insbesondere Asebiol^{®} LS 2539 BT 2 (INCI: Aqua, Hydrolyzed Yeast Protein, Pyridoxine, Niacinamide, Glycerin, Panthenol, Allantoin, Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin) und PEG-8 Isolauryl Thioether, wie es z. B. in dem Handelsprodukt "Antifettfaktor^{®} COS-218/2-A" von Cosmetochem enthalten ist (INCI: Aqua, Cetyl-PCA, PEG-8 Isolauryl Thioether, PCA, Cetyl Alcohol), sowie Mischungen der vorgenannten Substanzen. Bevorzugte Mischungen sind beispielsweise erhältlich als Handelsprodukt Acnacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte kosmetische oder dermatologische Zusammensetzungen sind dadurch gekennzeichnet, dass sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Zur Unterstützung des erfindungsgemäß verwendeten, die AQP-Synthese-stimulierenden Wirkstoffs, insbesondere Ergothionein, enthalten die erfindungsgemäßen Hautbehandlungsmittel bevorzugt weiterhin mindestens einen partikelförmigen, sebumsorbierenden Wirkstoff, insbesondere Talkum, Stärke und andere Partikel.
Erfindungsgemäß bevorzugte kosmetische oder dermatologische Zusammensetzungen sind dadurch gekennzeichnet, dass der partikelförmige, sebumsorbierende Wirkstoff ausgewählt ist aus anorganischen und organischen kosmetischen Adsorbentien mit mittleren Partikeldurchmessern von 0,1 - 100 µm, bevorzugt 0,5 - 50 µm, besonders bevorzugt 5 - 30 µm und außerordentlich bevorzugt 10 - 25 µm. Besonders bevorzugte anorganische Adsorbentien sind ausgewählt aus Kieselsäuren, insbesondere Aerosil^{®}-Typen, Kieselgelen, Siliciumdioxid, Tonen und Schichtsilicaten, insbesondere Bentoniten oder Kaolin, Magnesiumaluminiumsilikaten, insbesondere Talkum, und Bornitrid, sowie Mischungen der genannten Substanzen. Besonders bevorzugte organische Adsorbentien sind ausgewählt aus Stärken und Stärkederivaten, die chemisch und/oder physikalisch modifiziert sein können, insbesondere modifizierten Stärkederivaten vom Typ DRY FLO^{®} der National Starch and Chemical Company, Cellulosepulvern, Lactoglobulinderivaten, insbesondere Natrium-C₈₋₁₆-Isoalkyl-succinyllactoglobulinsulfonat, z. B. als Handelsprodukt Biopol^{®} OE von Brooks Industries erhältlich, Polymerpulvern aus Polyolefinen, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die genannten Polymerpulver in einer besonders bevorzugten Ausführungsform der Erfindung vernetzt sein können, sowie Mischungen der genannten Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugte Polymerpulver sind vernetzte Polymethacrylate (Micropearl^{®} M von SEPPIC oder Plastic Powder A von NIKKOL), vernetzte Polymethylmethacrylate (Micropearl^{®} M 305 und Micropearl^{®} M 310 von SEPPIC), Styrol-Divinylbenzol-Copolymere (z. B. Plastic Powder FP von NIKKOL), Polyethylen- und Polypropylen-Pulver (z. B. ACCUREL^{®} EP 400 von AKZO) oder auch Siliconpolymere (z. B. Silicone Powder X2-1605 von Dow Corning).
In einer weiteren bevorzugten Ausführungsform der Erfindung weist der partikelförmige, sebumsorbierende Wirkstoff Hohlräume auf. In einer weiteren bevorzugten Ausführungsform der Erfindung weist der partikelförmige, sebumsorbierende Wirkstoff halbkugelförmige Partikel auf, besonders bevorzugt sind mindestens 50 Gew.-% der Partikel halbkugelförmig. In einer weiteren bevorzugten Ausführungsform der Erfindung weist der partikelförmige, sebumsorbierende Wirkstoff halbkugelförmige Partikel mit Hohlräumen auf, besonders bevorzugt sind mindestens 50 Gew.-% der Partikel halbkugelförmig mit Hohlräumen.
Besonders bevorzugte partikelförmige, sebumsorbierende Wirkstoffe sind vernetzte Polymethylmethacrylate mit mittleren Partikeldurchmessern von 5-50 µm, insbesondere die Handelsprodukte Micropearl^{®} M 305 und Micropearl^{®} M 310 von SEPPIC. Außerordentlich bevorzugte partikelförmige, sebumsorbierende Wirkstoffe sind vernetzte Polymethylmethacrylate mit mittleren Partikeldurchmessern von 5 - 50 µm, die halbkugelförmige Partikel mit Hohlräumen umfassen, wobei besonders bevorzugt mindestens 50 Gew.-% der Partikel halbkugelförmig mit Hohlräumen sind, wie insbesondere das Handelsprodukt Micropearl^{®} M 310 von SEPPIC.
Erfindungsgemäß besonders bevorzugte Zusammensetzungen sind dadurch gekennzeichnet, dass sie mindestens einen partikelförmigen sebumsorbierenden Wirkstoff in einer Gesamtmenge von 0,5 bis 10 Gew.-%, bevorzugt 1 bis 5 Gew.-% und besonders bevorzugt 1,5 bis 3 Gew.-%, jeweils bezogen auf die gesamte Zusammensetzung, enthalten.

Zur Unterstützung des erfindungsgemäß verwendeten, die AQP-Synthese-stimulierenden Wirkstoffs, insbesondere Ergothionein, enthalten die erfindungsgemäßen Hautbehandlungsmittel bevorzugt weiterhin mindestens einen adstringierenden Wirkstoff, der zu einer temporären Verengung der Hautporen führt, beispielsweise Tannine bzw. Gerbstoffe bzw. allgemein Polyphenole, weiterhin Aluminiumsalze, wie sie auch als Antitranspirant-Wirkstoffe Verwendung finden. Diese Wirkstoffe regulieren die Sebummenge an der Hautoberfläche durch Verzögerung des Sebumaustritts.

Zur Unterstützung des erfindungsgemäß verwendeten, die AQP-Synthese-stimulierenden Wirkstoffs, insbesondere Ergothionein, enthalten die erfindungsgemäßen Hautbehandlungsmittel bevorzugt weiterhin mindestens einen sebumregulierenden Wirkstoff, der das an der Aktivität der Talgdrüsen beteiligte Enzym 5-alpha-Reduktase inhibiert. Bevorzugte Inhibitoren der 5-alpha-Reduktase sind ausgewählt aus Finasterid^{®}, 4-Androsten-3-on-17β-carbonsäure, (4R)-5,10-seco-19-Norpregna-4,5-dien-3,10,20-trian, (5-alpha,20-R)-4-Diazo-21-hydroxy-20-methylpregnan-3-on, 4-Azasteroide, insbesondere 17β-N,N-diethylcarbamoyl-4-methyl-4-aza-5-alpha-androstan-3-on, 17-alpha-acetoxy-6-methylenpregn-4-en-3,20-dion, 3-Carboxy-androsta-3,5-dien-Steroid-Derivate und 3-Carboxy A Ring-Aryl-Steroide, 3-Androsten-3-carbonsäuren, 3-Carboxy-17β-substituierte Steroide, 17β-(N-t-butylcarboxamid)-5-α-androst-1-en-4-aza-3-on, 17β-(N-t-butylcarboxamid)-androst-3,5-dien-3-carbonsäure, 17β-(N,N-diisopropylcarboxamid)-androst-3,5-dien-3-carbonsäure, 17β-(N,N-diisopropylcarboxamid)-estra-1,3, 5(1 0)-trien-3-carbonsäure, 17β-(N-t-butylcarboxamid)-estra-1,3,5(10)-trien-3-carbonsäure, 17β-(N,N-diisopropylcarboxamid)-estra-1,3,5(10)-trien-3-sulfonsäure, 17β-(N-t-butylcarboxamid)-estra-1,3,5(10)-trien-3-sulfonsäure, 17β-(N,N-diisopropylcarboxamid)-estra-1,3,5(10)-trien-3-phosphonsäure, 17β-(N-t-butylcarboxamid)-estra-1,3,5(10)-trien-3-phosphonsäure, 17β-(N,N-diisopropylcarboxamid)-estra-1,3,5(10)-trien-3-phosphinsäure, 17β-(N-t-butylcarboxamid)-estra-1,3,5(10)-trien-3-phosphinsäure, 17β-(N-t-butylcarboxamid)-androst-3,5-dien-3-phosphinsäure, 17β-(N,N-düsopropylcarboxamid)-androst-3,5-dien-3-phosphinsäure, 17β-(N-t-butylcarboxamid)-androst-3,5-dien-3-phosphonsäure, (Z)-17β-(N,N-diisopropylcarboxamid)-androst-4-en-3-yliden-essigsäure, 17β-(N,N-diisopropylcarboxamid)-5α-androst-2-en-3-essigsäure, (Z)-17β-(N,N-diisopropylcarboxamid)-5α-androst-3-yliden-essigsäure, 17β-(N,N-diisopropylcarboxamid)-5α-androst-3-en-3-essigsäure und 17β-(N-t-butylcarboxamid)-5α-androst-2-en-3-essigsäure sowie den physiologisch verträglichen Salzen der vorgenannten Säuren.
Erfindungsgemäß besonders bevorzugte Zusammensetzungen sind dadurch gekennzeichnet, dass sie mindestens einen Inhibitor der 5-alpha-Reduktase in einer Gesamtmenge von 0,0001 bis 1 Gew.-%, bevorzugt 0,001 bis 0,5 Gew.-% und besonders bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.
Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®} von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft^{®} von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol^{®} von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm^{®} von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia.
Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen organischen hautaufhellenden Wirkstoff. Erfindungsgemäß bevorzugte hautaufhellende Wirkstoffe sind einzeln oder in Mischungen ausgewählt aus Hydrochinon, Kojisäure, Arbutin, Extrakten aus Süßholzwurzel (Glycyrrhiza glabra), Extrakten aus verschiedenen Teilen des Maulbeerbaums (Morus spp., insbesondere aus Morus alba und hier insbesondere Extrakte aus der Baumrinde, dem Stamm, den Blättern und den Früchten), Extrakten aus Scutellaria spp. (Helmkraut), insbesondere aus Scutellaria baicalensis (Baikal-Helmkraut) und hier insbesondere Extrakte aus der Wurzel, Extrakten aus *Waltheria indica* ("Sleepy Morning") und hier insbesondere Extrakte aus den Blättern, Extrakten aus Bärentraube (*Arctostaphylos uva-ursi* (L.), Ericaceae) und hier insbesondere Extrakte aus den Blättern, Extrakten aus Preiselbeere (Blätter und Blüten), Extrakten aus Heidelbeere (Früchte), Extrakten aus Blattknospen von Birnbäumen, Anissamenöl, Extrakten aus Brombeerblättern, weiterhin Extrakten aus *Pyrola rotundifolia* (Rundblättriges Wintergrün), Gurke und/oder Limone, weiterhin Ascorbinsäure, Cumarinsäure ((Z)-2-Hydroxyzimtsäure) und cis-9-Octadecendisäure (andere Nomenklatur: cis-8-Hexadecendicarbonsäure), letztere kommerziell erhältlich z. B. unter der Bezeichnung Arlatone DIOIC DCA von Uniqema, und den Estern und/oder Salzen dieser Säuren.

Erfindungsgemäß besonders bevorzugte kosmetische oder dermatologische Zusammensetzungen sind dadurch gekennzeichnet, dass sie mindestens einen organischen hautaufhellenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Gelpflasters, Kataplasmas, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.
In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als ÖI-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-ÖI-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Mit dem Begriff Parfümöl sind vorzugsweise in sich abgeschlossene Duftstoffkompositionen gemeint, die gemeinhin zur Produktbeduftung eingesetzt werden und insbesondere nach menschlichem Ermessen wohlriechend sind. Dies sei an einem Beispiel erläutert. Will ein Fachmann z.B. ein Deodorant wohlriechend machen, so fügt er ihm für gewöhnlich nicht nur eine (wohl-)riechende Substanz, sondern ein Kollektiv (wohl-)riechender Substanzen bei. Ein solches Kollektiv besteht gewöhnlich aus einer Vielzahl einzelner Riechstoffe, z.B. mehr als 10 oder 15, vorzugsweise bis zu 100 oder mehr. Diese Riechstoffe formen zusammenwirkend ein gewünschtes wohlriechendes, harmonisches Geruchsbild.

Ein erfindungsgemäßes Parfümöl kann einzelne Riechstoffverbindungen, z.B. die synthetischen Produkte vom Typ der Ester, Ether, Aldehyde, Ketone, Alkohole und Kohlenwasserstoffe enthalten. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexyl-propionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote des gebildeten Parfümöl erzeugen.
Die Parfümöle können aber auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.
Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms während des Verdampfens, wobei man die Geruchseindrücke in "Kopfnote" (top note), "Herz- bzw. Mittelnote" (middle note bzw. body) sowie "Basisnote" (end note bzw. dry out) unterteilt.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lemongrasöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-ÖI, Ysop-Öl, Zimtöl, Zimtblätteröl sowie Zypressenöl.
Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische in den Parfümölen eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester.
Zu den leichter flüchtigen Riechstoffen, die im Rahmen der vorliegenden Erfindung in den Parfümöl vorteilhaft einsetzbar sind, zählen insbesondere die niedriger siedenden Riechstoffe natürlichen oder synthetischen Usprung, die allein oder in Mischungen eingesetzt werrden können. Beispiele für leichter flüchtige Riechstoffe sind Alkyisothiocyanate (Alkylsenföle), Butandion, Limonen, Linalool, Linalylacetat und -propionat, Menthol, Menthon, Methyl-n-hep-tenon, Phellandren, Phenylacetaldehyd, Terpinylacetat, Zitral, Zitronellal.

Alle vorgenannten Riechstoffe sind alleine oder in Mischung in den Parfümölen gemäß der vorliegenden Erfindung mit den bereits genannten Vorteilen einsetzbar.

Insbesondere können auch Duftsstoffe aus der Gruppe der Allylalkoholester, Ester sekundärer Alkohole, Ester tertiärer Alkohole, allylische Ketone, Acetale, Ketale, Kondensationsprodukte von Aminen und Aldehyden und/oder deren Mischungen im Parfümöl enthalten sein.

In einer bevorzugten Ausführungsform enthält das erfindungsgemäße Mittel bestimmte Minimalwerte an Parfümöl, nämlich zumindest 0,00001 Gew.-%, vorteilhafterweise zumindest 0,0001 Gew.-%, in beträchtlich vorteilhafter Weise zumindest 0,001 Gew.-%, in vorteilhafterer Weise zumindest 0,01 Gew.-%, in weiter vorteilhafter Weise zumindest 0,1 Gew.-%, in noch weiter vorteilhafter Weise zumindest 0,2 Gew.-%, in sehr vorteilhafter Weise zumindest 0,3 Gew.-%, in besonders vorteilhafter Weise zumindest 0,4 Gew.-%, in ganz besonders vorteilhafter Weise zumindest 0,45 Gew.-%, in erheblich vorteilhafter Weise zumindest 0,5 Gew.-%, in ganz erheblich vorteilhafter Weise zumindest 0,55 Gew.-%, in äußerst vorteilhafter Weise zumindest 0,6 Gew.-%, in höchst vorteilhafterweise zumindest 0,65 Gew.-%, in überaus vorteilhafterweise zumindest 0,7 Gew.-%, in ausnehmend vorteilhafter Weise zumindest 0,75 Gew.-%, in außergewöhnlich vorteilhafter Weise zumindest 0,8 Gew.-%, in außerordentlich vorteilhafter Weise zumindest 0,85 Gew.-%, insbesondere zumindest 0,9 Gew.-% an Parfümöl, bezogen auf das gesamte Mittel.

In einer bevorzugten Ausführungsform enthalten die Parfümöle weniger als 8, vorteilhafterweise weniger als 7, in vorteilhafterer Weise weniger als 6, in wiederum vorteilhafterer Weise weniger als 5, in weiter vorteilhafterweise weniger als 4, noch vorteilhafter weniger als 3, vorzugsweise weniger als 2, insbesondere keine Duftstoffe aus der Liste Amylcinnamal, Amylcinnamylalkohol, Benzylalkohol, Benzylsalicylat, Cinnamylalkohol, Cinnamal, Citral, Cumarin, Eugenol, Geraniol, Hydroxycitronellal, Hydroxymethylpentylcyclohexencarboxaldehyd, Isoeugenol, Anisylalkohol, Benzylbenzoat, Benzylcinnamat, Citronellol, Farnesol, Hexylcinnamaldehyd, Lilial, d-Limonen, Linalool, Methylheptincarbonat, 3-Methyl-4-(2,6,6-trimethyl-2-cyclohexen-1-yl)-3-buten-2-on, Eichenmoosextrakt, Baummoosextrakt.

Nach einer weiteren bevorzugten Ausführungsform ist das erfindungsgemäße Mittel frei von Parfümöl-Komponenten, insbesondere in Produkten für sensitive Haut.

Erfindungsgemäß bsonders bevorzugte Mittel werden als Emulsion bereitgstellt. Emulsionen sind disperse Systeme von zwei oder mehreren miteinander nicht mischbaren Flüssigkeiten. Die eine der flüssigen Phasen bildet dabei das Dispersionsmittel (auch: äußere, kontinuierliche oder zusammenhängende Phase), in dem die andere Phase (auch: innere oder disperse Phase) in Form feiner Tröpfchen verteilt ist. In Abhängigkeit von der Größe der dispergierten Teilchen und von der kinetischen bzw. thermodynamischen Stabilität spricht man von Makro- (auch: grob-dispers) und Mikroemulsion (auch: kolloid-dispers). Der Teilchendurchmesser schwankt dabei zwischen 10⁻⁴ und 10⁻⁸ cm, die meisten Emulsionen zeigen jedoch eine uneinheitliche Teilchengröße und sind polydispers. Je nach Größe der dispergierten Teilchen sind Emulsionen milchig trüb (Makroemulsion) bis klar (Mikroemulsion).

Die meisten natürlichen und technischen Emulsionen bestehen aus Wasser und Öl oder Fett als nicht mischbare Phasen. In Abhängigkeit von Zusammensetzung und Verhältnis der Phasen bestehen zwei Möglichkeiten der Verteilung. Ist Wasser die äußere und ÖI die innere Phase, liegt eine Öl-in-Wasser-Emulsion (O/W-Emulsion) vor, deren Grundcharakter durch das Wasser geprägt ist. Ist Öl die äußere und Wasser die innere Phase, liegt eine Wasser-in-Öl-Emulsion (W/O-Emulsion) vor, wobei hier der Grundcharakter vom Öl bestimmt wird.

Erfindungsgemß besonders bevorzugte Zusammensetzungen liegen in Form von O/W-Emulsionen vor, wobei besonders bevorzugte erfindungsgemäße Emulsionen lamellare Strukturen aufweisen, d.h. Vesikeln enthalten.

Vesikel sind sphärisch-lamellare, polydisperse amphiphile Strukturen unterschiedlicher Geometrie, allgemein für einen durch eine adsorbierte Doppelschicht (eine "Membran") stabilisierten Tropfen. Vesikeln bilden sich zum Beispiel auf dem Weg der Selbstorganisation aus flüssigkristallinen Lα-Phasen, die aus nichtionischen Tensiden und Co-Tensiden bestehen, durch Zusatz von ionischen Tensiden ("Aufladung"). Die Strukturen von Vesikeln lassen sich durch Gefrierbruch-Transmissionselektronenmikroskopie darstellen. Multilamellare Vesikeln können durch Scherung in kleinere und mit zunehmendem Schergradienten in unilamellare Vesikeln umgewandelt werden, die ihrerseits flüssigkristalline (kubische) Phasen mit viskoelastischem Verhalten ausbilden können. Künstlich aus Phospholipiden und anderen grenzflächenaktiven Substanzen hergestellte Vesikeln werden meist Liposomen genannt und finden auch als Vehikel für Wirkstoffe in Medizin und Kosmetik Anwendung.

Zusammenfassend sind erfindungemäße Zusammensetzungen bevorzugt, die in Form einer Emulsion, vorzugsweise in Form einer lamellaren Emulsion, vorliegen.

Die erfindungsgemäß verwendeten Zusammensetzungen können in verschiedenen Darreichungsformen konfektioniert werden, beispielsweise als (ggf. öl- und fettfreies) Gel, als Creme, in Stiftform, als flüssige oder gelförmige Roll-on-Applikation, als getränktes flexibles Substrat (z. B. Pad, Tuch oder Bausch), aber auch als Puder oder Spray.

Erfindungsgemäße Mittel können in fester, halbfester, flüssiger, disperser, emulgierter, suspendierter, oder gelförmiger Form vorliegen. Weiterhin können die erfindungsgemäßen Mittel als Aerosol konfektioniert sein, das heißt, sie sind in einem Druckbehälter verpackt, aus dem sie mit Hilfe eines Treibmittels versprüht werden können. Weiterhin können die erfindungsgemäßen Mittel als treibgasfreies Pumpspray versprüht werden.

In einer besonders bevorzugten Ausführungsform liegen die erfindungsgemäßen Mittel in Form eines Hydrogels vor. Hydrogele sind Wasser enthaltende Gele auf der Basis hydrophiler, aber wasserunlöslicher Polymere, die als dreidimensionale Netzwerke vorliegen. In Wasser quellen diese Netzwerke unter weitgehender Formerhaltung bis zu einem Gleichgewichtsvolumen auf. Die Netzwerk-Bildung erfolgt vorwiegend über chemische Verknüpfung der einzelnen Polymerketten, ist aber auch physikalisch durch elektrostatische, hydrophobe oder Dipol/Dipol-Wechselwirkungen zwischen einzelnen Segmenten der Polymerketten möglich. Über die Wahl der zum Polymeraufbau verwendeten Monomeren, die Art der Vernetzung und die Vernetzungsdichte können gewünschte Eigenschaften der Hydrogele gezielt eingestellt werden. Die notwendige Hydrophilie der Polymeren vermitteln unter anderem Hydroxy-, Carboxylat-, Sulfonat- und/oder Amid-Gruppen. Synthetische Hydrogele basieren unter anderem auf Poly(meth)acrylsäuren, Poly(meth)acrylaten, Polyvinylpyrrolidon oder Polyvinylalkohol. Bevorzugte anionische polymere Hydrogelbildner, die die Wirkung des erfindungsgemäß verwendeten Wirkstoffs unterstützen können, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder TriethanolammoniumSalz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionische Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder TriethanolammoniumSalz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnungen Simulgel^{®} 600, Simulgel^{®} NS, Simulgel^{®} EG, Simulgel^{®} EPG und Sepiplus^{®} 400 als Compound mit einem Öl wie Isohexadecan, Squalan oder hydriertem Polyisobuten, und einem Emulgator, wie Polysorbate, Laureth-7 oder Caprylyl/Capryl Glucoside, vertriebenen Natriumacryloyldimethyltaurat-Copolymere (mit Monomeren, ausgewählt aus Acrylamid, Natriumacrylat, Hydroxyethylacrylat und/oder Acrylsäure) haben sich als erfindungsgemäß besonders wirksam erwiesen. Weitere bevorzugte Hydrogelbildner sind Ammoniumacryloyldimethyltaurat-Vinylpyrrolidon-Copolymere, insbesondere das Handelsprodukt Aristoflex^{®} AVC von Clariant.

Weitere besonders bevorzugte anionische Homopolymer-Hydrogelbildner sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}. Ein besonders bevorzugtes anionisches Copolymer enthält als Monomer zu 80 - 98 % eine ungesättigte C₃₋₆-Carbonsäure oder ihr Anhydrid sowie zu 2 - 20 % Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex Noveon bzw. B.F. Goodrich).

Bevorzugte nichtionische polymere Hydrogelbildner sind beispielsweise Polyvinylalkohole, die teilverseift sein können, z. B. die Handelsprodukte Mowiol^{®} sowie VinylpyrrolidonNinylester-Copolymere und Polyvinylpyrrolidone, die z. B. unter dem Warenzeichen Luviskol^{®} (BASF) vertrieben werden.

Erfindungsgemäß bevorzugte natürliche Hydrogelbildner sind insbesondere ausgewählt aus gewünschtenfalls physikalisch (thermisch) und/oder chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose weiterhin insbesondere Stärken sowie Stärkeabbauprodukte wie Amylose und Amylopektin, chemisch und/oder thermisch modifiziertenStärken, z. B. Hydroxypropylstärkephosphat, Dihydroxypropyldistärkephosphat, insbesondere den anionischen Stärkederivaten Aluminiumstärkeoctenylsuccinat (z.B. die Handelsprodukte Dry Flo^{®}), Natriumstärkeoctenylsuccinat, Calciumstärkeoctenylsuccinat, weiterhin Chitosan und dessen Derivaten, weiterhin Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

In einer weiteren besonders bevorzugten Ausführungsform liegen die erfindungsgemäßen Mittel in Form einer Öl-in-Wasser-Emulsion vor. Derartige Öl-in-Wasser-Emulsionen können, ebenfalls bevorzugt, durch mindestens einen der vorstehend genannten Hydrogelbildner stabilisiert vorliegen.

In einer weiteren besonders bevorzugten Ausführungsform liegen die erfindungsgemäßen Mittel in flüssiger Form vor. Die Applikation kann dabei vorzugsweise mit Sprühvorrichtungen erfolgen. Diese Sprühvorrichtungen enthalten in einem Behälter eine Füllung aus dem erfindungsgemäßen (flüssigen, breiartigen oder pulverförmigen) Hautbehandlungsmittel. Die Füllung kann unter dem Druck eines Treibmittels stehen (Druckgasdosen, Druckgaspackungen, Aerosolpackungen), oder es kann sich um einen mechanisch zu bedienenden Pumpzerstäuber (Pumpsprays) handeln. Die Behälter weisen eine Entnahmevorrichtung auf, vorzugsweise in Gestalt von Ventilen, die die Entnahme des Inhalts als Nebel, Rauch, Schaum, Pulver, Paste oder Flüssigkeitsstrahl ermöglichen. Als Behälter für die Sprühvorrichtungen kommen vor allem zylindrische Gefäße aus Metall (Aluminium, Weißblech, Rauminhalt vorzugsweise <1000 ml), geschütztem bzw. nichtsplitterndem Glas oder Kunststoff (Rauminhalt vorzugsweise <220 ml) bzw. splitterndem Glas oder Kunststoff (Rauminhalt vorzugsweise <150 ml) in Frage.

Cremeförmige, gelförmige, pastose und flüssige Mittel können z.B. in Pump- oder Quetschspendern abgepackt sein, insbesondere auch in Mehrkammer-Pump- oder Quetschspendern.

Unter den Begriff der Flüssigkeit fallen im Sinne der Erfindung auch jegliche Festkörperdispersionen in Flüssigkeiten. Erfindungsgemäße Mittel können auch als Pasten, Salben, Lotionen oder Cremes vorliegen. Feste Mittel können beispielsweise als loser Puder, gepresster Puder oder als Stift vorliegen.

Die Applikation kann z.B. im Falle flüssiger Mittel vorzugsweise auch mit einem Roller-Applikator erfolgen, wie er z.B. aus dem Bereich der Deo-Roller bekannt ist. Solche Roller weisen eine in einem Kugelbett gelagerte Kugel auf, welche durch Bewegung über eine Oberfläche bewegt werden kann. Dabei nimmt die Kugel etwas von dem zu verteilenden Mittel auf und befördert dieses an die zu behandelnde Oberfläche. Mit derartigen Applikatoren lassen sich gezielt und sparsam nur die tatsächlich von Akne und/oder Unreinheiten betroffenen Hautpartien behandeln.

Die Applikation kann z.B. auch mit Substraten erfolgen, die mit einer erfindungsgemäßen Zubereitung beaufschlagt sind. Besonders bevorzugt sind Feuchttücher, d.h. für den Anwender vorgefertigte, vorzugsweise einzeln abgepackte, Feuchttücher, wie sie z. B. aus dem Bereich der Glasreinigung (Brillenputztücher) oder aus dem Bereich der feuchten Toilettenpapiere wohlbekannt sind. Solche Feuchttücher, die vorteilhafterweise auch Konservierungsstoffe enthalten können, sind dann vorteilhafterweise mit einem erfindungsgemäßen Mittel imprägniert oder beaufschlagt, vorteilhafterweise sind sie einzeln verpackt. Sie können z. B. als Reinigungs-Tuch oder Abschminktuch (Abschminkpad) eingesetzt werden, was besonders interessant für den Gebrauch unterwegs ist.

Bevorzugte Substratmaterialien sind bevorzugt ausgewählt aus porösen flächigen Tüchern. Sie können aus einem faserigen oder zellulären flexiblen Material bestehen, das ausreichend mechanische Stabilität und gleichzeitig Weichheit zur Anwendung auf der Haut aufweist. Zu diesen Tüchern gehören Tücher aus gewebtem und ungewebtem synthetischen und natürlichen Fasern, Filz, Papier oder Schaumstoff, wie hydrophilem Polyurethanschaum.

Vorzugsweise werden hier herkömmliche Tücher aus ungewebtem Material (Vliese) verwendet. Vliese sind im allgemeinen als adhäsiv gebondete faserige Produkte definiert, die eine Matte oder geschichtete Faserstruktur aufweisen, oder solche, die Fasermatten umfassen, bei denen die Fasern zufällig oder in statistischer Anordnung verteilt sind. Die Fasern können natürlich sein, wie Wolle, Seide, Jute, Hanf, Baumwolle, Lein, Sisal oder Ramie; oder synthetisch, wie Rayon, Celluloseester, Polyvinylderivate, Polyolefine, Polyamide oder Polyester. Im allgemeinen ist jeder Faserdurchmesser bzw. -titer für die vorliegende Erfindung geeignet. Die hier eingesetzten ungewebten Stoffe neigen aufgrund der zufälligen oder statistischen Anordnung von Fasern in dem ungewebten Material, die ausgezeichnete Festigkeit in allen Richtungen verleihen, nicht zum Zerreißen oder Zerfallen. Beispiele für ungewebte Stoffe, die sich als Substrate in der vorliegenden Erfindung eignen, sind beispielsweise aus WO 98/18441 bekannt. Bevorzugte poröse und flächige Reinigungstücher bestehen aus einem oder verschiedenen Fasermaterialien, insbesondere aus Baumwolle, veredelter Baumwolle, Polyamid, Polyester oder Mischungen aus diesen. Vorzugsweise weisen die Substrate in Tuchform eine Fläche von 10 bis 5000 cm², vorzugsweise von 50 bis 2000 cm², insbesondere von 100 bis 1500 cm² und besonders bevorzugt von 200 bis 1000 cm² auf. Die Grammatur des Materials beträgt dabei üblicherweise zwischen 20 und 1000 g/m², vorzugsweise von 30 bis 500 g/m² und insbesondere von 50 bis 150 g/m². Erfindungsgemäß bevorzugte Hautbehandlungs-Substrate können durch Tränken oder Imprägnierung oder auch durch Aufschmelzen der erfindungsgemäßen Mittel auf ein Substrat erhalten werden.

Die erfindungsgemäßen Mittel, vorzugsweise flüssigen Mittel, können auch mehrphasig sein, die Phasen können z.B. horizontal, also übereinander, oder vertikal, also nebeneinander, angeordnet sein. Es kann sich auch um ein disperses System handeln, bei dem z.B. die festen Bestandteile inhomogen in der flüssigen Matrix verteilt sind, so dass ein solches disperses System vor der Anwendung geschüttelt werden sollte.

Je nach Darreichungsform enthalten die erfindungsgemäßen Zusammensetzungen weitere Wirk- und/oder Hilfsstoffe.

Bezüglich bevorzugter Ausführungsformen der erfindungsgemäßen Verfahren gilt mutatis mutandis das zu den erfindungsgemäßen Verwendungen Gesagte.

### Versuche

An einem Hautmodell, wie in WO 03/053394 A2 beschrieben, wurde der Einfluss folgender Substanzen auf die Aquaporinsynthese überprüft:
1.) Ergothionein (10 mg/ml Testlösung)
2.) Wässriger Extrakt aus Laminaria digitata (mit einem hohen Anteil an Laminarin; verwendet wurde das Handelsprodukt Phycojuvenine in einer solchen Menge, dass die Testlösung eine Konzentration von 10 mg Extrakt-Trockensubstanz pro ml aufwies)
3.) Wässriges Proteinhydrolysat von Hefeproteinen (verwendet wurde das Handelsprodukt Biodynes TRF Powder von Arch Chemicals Inc. in einer von 10 mg Extrakt-Trockensubstanz pro ml Testlösung)

### Versuchsdurchführung:

Die Hautmodelle wurden nach Ankunft in frisches Medium gesetzt. Nach Vorinkubation über Nacht wurden sie alle zwei Tage mit den Testsubstanzen behandelt, indem der Wirkstoff systemisch in das Medium gegeben wurde. Die Kulturen wurden nach 6 Tagen für Cryoschnitte bei -80 °C eingefroren. Zum Zeitpunkt 0 wurden Kontrollproben eingefroren.

### Immunhistologie:

Zum Nachweis von Aquaporinen (AQ3) wurde die indirekte Immunhistologie angewendet. Hierbei bindet ein unkonjungierter Primärantikörper an das Antigen. Anschließend wird ein Sekundärantikörper eingesetzt, der gegen den Primärantikörper gerichtet ist.

### Ergebnis:

Es zeigte sich, dass nur Ergothionein und der wässrige Extrakt aus Laminaria digitata die Aquaporinsynthese stimulierten (=Induktion der Aquaporine (AQ3)). Das aminosäurereiche Hefeproteinhydrolysat hatte keinen stimulierenden Einfluss auf die Aquaporinsynthese.

Die nachfolgenden Beispiele für erfindungsgemäß verwendete Zusammensetzungen sollen den Gegenstand der vorliegenden Anmeldung näher erläutern, ohne ihn hierauf zu beschränken. Alle Mengenangaben beziehen sich, soweit nicht anders angegeben, auf Gew.-%, bezogen auf die gesamte Zusammensetzung.

### 1. ÖI-in-Wasser-Emulsionen

### 1. Beispielserie: Tagescremes mit nachhaltigem Feuchtigkeitseffekt

| | 1 | 2 | 3 |
|---|---|---|---|
| Distelöl | 3,00 | 3,00 | 3,00 |
| Myritol 318 | 5,00 | 5,00 | 5,00 |
| Novata AB | 2,00 | 2,00 | 2,00 |
| Lanette 22 | 1,00 | 1,00 | 1,00 |
| Cutina MD | 2,00 | 2,00 | 2,00 |
| Stenol 1618 | 1,00 | 1,00 | 1, 00 |
| Baysilon M350 | 1,00 | 1,00 | 1,00 |
| Controx KS | 0,05 | 0,05 | 0,05 |
| Propylparaben | 0,20 | 0,20 | 0,20 |
| Dry Flo Plus | 1,00 | 2,00 | 3,00 |
| Propylenglycol | 5,00 | 5,00 | 5,00 |
| Glycerin | 5,00 | 3,00 | 3,00 |
| Methylparaben | 0,20 | 0,20 | 0,20 |
| Phenoxyethanol | 0,9 | 0,5 | 0,9 |
| DSH-C N | 5,0 | 3,0 | |
| Phycojuvenine | 3 | - | 1 |
| Ergothionein | - | 0,5 | 0,1 |
| Parfum | 0,10 | 0,10 | 0,10 |
| Aqua | ad 100 | ad 100 | ad 100 |

### 2. Beispielserie: Tagescremes mit nachhaltigem Feuchtigkeitseffekt

| | 1 | 2 | 3 | 4 |
|---|---|---|---|---|
| Lipoid S75-3 | 0,50 | 0,50 | 0,50 | 0,50 |
| Tocopherylacetat | 0,50 | 0,50 | 0,50 | 0,50 |
| Cutina MD | 1,00 | 1,00 | 1,00 | 1,00 |
| Lanette 22 | 2,00 | 2,00 | 2,00 | 2,00 |
| Baysilone M 350 | 0,50 | 0,50 | 0,50 | 0,50 |
| Propylparaben | 0,20 | 0,20 | 0,20 | 0,20 |
| Dow Corning 9040 | 1,00 | 1,00 | 1,00 | 1,00 |
| Glycerin | 4,50 | 3,00 | 3,00 | 3,00 |
| Hexandiol | 6,00 | 3,00 | | 3,00 |
| Methylparaben | 0,20 | 0,20 | 0,20 | 0,20 |
| Tego Carbomer 140 | 0,30 | 0,30 | 0,30 | 0,30 |
| DSH-CN | 5,00 | 5,00 | 5,00 | 5,00 |
| Simulgel NS | 1,50 | 1,50 | 1,50 | 1,50 |
| TiO₂ | 0,50 | 0,50 | 0,50 | 0,50 |
| Ergothionein | 0,5 | 0,1 | - | - |
| Phycojuvenine | - | - | 3 | 2 |
| Ederline L | - | - | - | 2 |
| Matrixyl 3000 | - | - | - | 3 |
| Wasser | ad 100 | ad 100 | ad 100 | ad 100 |

### 3. Beispielserie: Tagescremes mit nachhaltigem Feuchtigkeitseffekt

| | 1 | 2 | 3 |
|---|---|---|---|
| Sisterna SP30-C | 2,0 | 2,0 | 2,0 |
| Sisterna SP70-C | 0,8 | 0,8 | 0,8 |
| Propylparaben | 0,2 | 0,2 | 0,2 |
| Methylparaben | 0,2 | 0,2 | 0,2 |
| Ethanol | 5,0 | 3,0 | - |
| Dry Flo Plus | 1,0 | 1,0 | 1,0 |
| Parfum | 0,3 | 0,3 | 0,3 |
| Cetearyl Alcohol | 1,0 | 1,0 | 1,0 |
| Fucogel 1000 | 2,0 | 1,0 | 0,5 |
| D-Panthenol | 0,7 | - | - |
| Herbasol Destilat weißer Tee | - | 0,5 | 0,5 |
| Hydroglycolic Extract of Ginseng | - | 0,45 | 0,45 |
| Spirulina Extrakt 3002 | 1,00 | 1,00 | 1,00 |
| DSH-C N | 5,0 | 3,0 | - |
| Keltrol SF | 0,2 | - | 0,2 |
| Aristoflex AVC | - | 0,5 | - |
| Dow Corning 200 Fluid | 7,0 | - | - |
| Dow Corning 245 | - | 5,0 | 5,0 |
| Cetiol 868 | 2,0 | - | 2,0 |
| Natriumascorbylphosphat | 1 | - | 1 |
| Ergothionein | 0,1 | 0,1 | - |
| Phycojuvenine | - | 2 | 3 |
| Parfum | 0,3 | 0,3 | 0,3 |
| Wasser | ad 100 | ad 100 | ad 100 |

### 4. Beispielserie: Nachtcremes mit nachhaltigem Feuchtigkeitseffekt

| | 1 | 2 | 3 |
|---|---|---|---|
| Dow Corning 245 Fluid | 25 | 25 | 25 |
| Abil EM 90 | 2 | 2 | 2 |
| Belsil DM 100 | 2,5 | 2,5 | 2,5 |
| Hydrogenated Castor Oil | 2 | 2,5 | 2 |
| Glycerin | 5 | 5 | 5 |
| NaCl | 2 | 2,1 | 2 |
| Symdiol68 | 0,5 | 0,5 | 0,5 |
| Phenoxyethanol | 0,4 | 0,4 | 0,4 |
| D-Panthenol | 0,45 | 0,45 | 0,45 |
| d,l-alpha-Bisabolol | 0,1 | 0,1 | 0,1 |
| Ergothionein | 0,1 | - | 0,1 |
| Phycojuvenine | - | 3 | 3 |
| Glistin | - | 2 | 1 |
| Parfum | 0,3 | 0,3 | 0,3 |
| Wasser | ad 100 | ad 100 | ad 100 |

### 5. Beispielserie: Tagescremes mit nachhaltigem Feuchtigkeitseffekt

| | 1 | 2 | 3 |
|---|---|---|---|
| Montanov 202 | 5,0 | 5,0 | 5,0 |
| Cutina MD | 2,0 | 2,0 | 2,0 |
| Cetearyl Alcohol | 1,0 | 1,0 | 1,0 |
| Cetiol B | 9,0 | 9,0 | 9,0 |
| Controx KS | 0,05 | 0,05 | 0,05 |
| Dow Corning 245 | | 7,0 | 3,0 |
| Dow Corning 9040 | 1,0 | 1,0 | |
| Fucogel 1000 | | 2,0 | |
| Phycojuvenine | 2 | - | 1,0 |
| Ergothionein | - | 0,1 | 0,1 |
| D-Panthenol | - | - | 0,7 |
| Herbasol Destillat weißer Tee | - | 0,5 | - |
| Hydroglycolic Extract of Ginseng - | | 0,45 | - |
| DSH-C N | 5,0 | 5,0 | - |
| Keltrol SF | - | 0,2 | 0,2 |
| Aristoflex AVC | - | - | 0,5 |
| Dow Corning 200 Fluid | 7,0 | 7,0 | - |
| Dow Corning 245 | - | 5,0 | 3,0 |
| Dow Corning 9040 | 1,0 | - | 1,0 |
| Tego Carbomer | 0,3 | 0,3 | 0,3 |
| Phenonip | 0,5 | 0,5 | 0,5 |
| Dry Flo Plus | 1,0 | 1,0 | 1,0 |
| Parfum | 0,3 | 0,3 | 0,3 |
| Wasser | ad 100 | ad 100 | ad 100 |

### 6. Beispielserie: Tagescremes mit nachhaltigem Feuchtigkeitseffekt

| | 1 | 2 | 3 |
|---|---|---|---|
| Glucamate SSE-20 | 2,0 | 2,0 | 2,0 |
| Sympatens-BS/080 | 2,0 | 2,0 | 2,0 |
| Dry Flo Plus | - | 1,0 | 1,0 |
| Parfum | 0,3 | 0,3 | 0,3 |
| Silikonöl 350 | 0,3 | 0,3 | - |
| Cutina MD | 2,0 | 2,0 | 2,0 |
| Paraffinöl | 2,0 | 1,0 | - |
| Cetearyl Alcohol | 1,0 | 1,5 | 1,25 |
| Capryl-/Caprinsäure-Triglycerid | - | 3,5 | 3,0 |
| Herbasol Destillat Salbei | 1,0 | - | 1,0 |
| Herbasol Destillat Hamamelis | 1,0 | - | 1,0 |
| D-Panthenol | 0,7 | - | 1,0 |
| D,I-Bisabolol | 0,1 | 0,1 | - |
| Phenoxyethanol | 0,98 | 0,5 | 0,9 |
| Symdiol 68 | - | 0,5 | - |
| DSH-C N | - | 5,0 | - |
| Keltrol SF | - | - | 0,2 |
| Aristoflex AVC | - | 0,5 | 0,5 |
| Hexandiol-1,6 | - | 5,0 | 5,0 |
| Glycerin | 2,0 | 5,0 | 10,0 |
| Culminal MHPC 100 | 0,1 | 0,1 | 0,1 |
| Propylenglycol | 2,0 | 5,0 | 3,0 |
| Natriumascorbylphosphat | 1 | - | 1 |
| Phycojuvenine | - | 3 | 3 |
| Ergothionein | 0,1 | 0,2 | - |
| Wasser | ad 100 | ad 100 | ad 100 |

Die Zusammensetzungen aus den Beispielserien 1 - 6 wurden jeweils auf die Gesichtshaut aufgetragen.
Nach regelmäßiger Applikation morgens und abends über einen Zeitraum von 5 Wochen zeigten die Probandinnen eine deutlich straffere und strahlendere Haut mit einem gegenüber der Zeit vor Behandlungsbeginn verbesserten Hautfeuchtegehalt.

### 2. Reinigungszubereitungen

### 1. Beispielserie: Gesichtswässer (Tonics) mit nachhaltigem Feuchtigkeitseffekt

| | 1 | 2 | 3 |
|---|---|---|---|
| Dipropylenglycol | 10,00 | 10,00 | 10,00 |
| Chlorhexidindigluconat | 1,00 | 1,00 | 1,00 |
| Synperonic PE7L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Natriumascorbylphosphat | 1 | - | 1 |
| Ergothionein | 0,1 | 0,1 1 | - |
| Phycojuvenine | - | 2 | 3 |
| Parfume | 0,20 | 0,20 | 0,20 |
| Aqua | ad 100 | ad 100 | ad 100 |

Die erfindungsgemäß verwendeten Tonics wurden jeweils mit Hilfe eines Pads, Wattebausches oder eines Nonwoven-Substrats auf die Gesichtshaut aufgetragen.
Nach regelmäßiger Applikation morgens und abends über einen Zeitraum von 5 Wochen zeigten die Probandinnen eine deutlich straffere und strahlendere Haut mit einem gegenüber der Zeit vor Behandlungsbeginn verbesserten Hautfeuchtegehalt.
Die erfindungsgemäß verwendeten Wirkstoffe lassen sich außer in Gesichtswässer natürlich auch in schäumende Reinigungsmittel mit einem Gehalt an üblichen Reinigungstensiden sowie in milde, gegebenenfalls gering schäumende Reinigungscremes einarbeiten. Derartige Reinigungszusammensetzungen werden typischerweise auf die Haut aufgetragen, wenige Sekunden (z.B. 10 bis 60 Sekunden) bis einige Minuten (z.B. 1, 2, 3, 4 oder 5 Minuten) auf der Haut belassen und anschließend abgespült und/oder abgewischt.
Die erfindungsgemäß verwendeten Wirkstoffe lassen sich auch in Pflaster, insbesondere in Gelpflaster, einarbeiten.

### Liste der verwendeten Rohstoffe

| | INCI | Hersteller/Lieferant |
|---|---|---|
| Acnacidol PG | Sebacic acid, 10-hydroxydecanoic acid, 1,10-decanediol | Vincience |
| Ajidew^{®} NL 50 | Sodium PCA | AJINOMOTO |
| Baysilone-Öl M 350 | Dimethicone | GE Bayer Silicones |
| Biodocarb^{®} | lodopropynyl Butylcarbamate | MILKER & GRÜNING |
| Biopeptide CL | N-Palmitoyl-Gly-His-Lys | Sederma |
| Biopeptide EL | N-Palmitoyl-Val-Gly-Val-Ala-Pro-Gly | Sederma |
| Brij 30 | Laureth-4 | Uniqema |
| Calmosensine | BUTYLENE GLYCOL, WATER, LAURETH-3, HYDROXYETHYLCELLULOSE, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 | Acrylates/C10-30 Alkyl Acrylate Cross-polymer | Noveon |
| Cetiol^{®} HE | PEG-7 Glyceryl Cocoate | Cognis |
| Cetiol^{®} PGL | Hexyldecanol, Hexyldecyl Laurate | Cognis |
| Controx KS | Tocopherol, Hydrogenated Palm Glycerides Citrate | Cognis |
| Cosmedia Guar^{®} C 261 | Guar Hydroxypropyl Trimonium Chloride | Cognis |
| Crodarom Chardonnay L | Propylene Glycol, Water, Vitis Vinifera (Grape) Seed Extract | Croda |
| Cutina CBS | Glyceryl Stearate / Cetearyl Alcohol / Cetyl Palmitate / Cocoglycerides | Cognis |
| Cutina GMS | Glyceryl Stearate (Mschung aus Glycerylmono- und distearat) | Cognis |
| Cutina MD | Glyceryl Stearate | Cognis |
| DC^{®} 190 | Dimethicone | Dow Corning |
| Dehyton K | AQUA (WATER), COCAMIDOPROPYL BETAINE (29 - 32 Gew.-%) | Cognis |
| Deliner | Zea Mays (Corn) Kernel Extract, Butylene Glycol, Xanthan Gum | Coletica |
| DERMOSOFT OCTIOL | 1,2-Octandiol | Dr. Straetmans |
| Dow Corning 1501 Fluid | Cyclomethicone (85 - 86 Gew.-%), Dimethiconol (14-15 Gew.-%) | Dow Corning |
| Dow Corning 200 (0,65 cSt) | Dimethicone | Dow Corning |
| Dow Corning 9040 | Cyclomethicone/ Dimethicone Crosspolymer | Dow Corning |
| Dow Corning^{®}200 Fluid, 5 cSt. | Dimethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline H | PEG-40 Hydrogenated Castor Oil, PPG-2-Ceteareth-9, Pyrus Malus (Apple) Fruit Extract | Seporga |
| Ederline L | Hexyldecanol, Pyrus Malus (Apple) Fruit Extract | Seporga |
| EMULGADE^{®} SE | Glyceryl Stearate, Ceteareth-20, Ceteareth-12, Cetearyl Alcohol, Cetyl Palmitate | Cognis |
| Eumulgin B 2 | Ceteareth-20 | Cognis |
| Eumulgin B 3 | Ceteareth-30 | Cognis |
| Eumulgin 05 | Oleth-5 | Cognis |
| Eusolex^{®} OCR | Octocrylene | Merck KGaA |
| Exsy Algine | ACETYL CITRULL AMIDO ARGININE | Exsymol |
| Fucogel 1000 | Biosaccharide Gum-1 | Solabia |
| Germall^{®} 115 | Imidazolidinyl Urea | Sutton Laboratories |
| Gingkoselect Phytosome | 0,6 to 1,2 Gew.-% Ginsenoside-Aktivsubstanz, Liposomen-verkapselt und unverkapselt | Indena SpA |
| Glistin | AQUA (WATER), GLUTAMYLAMIDOETHYL INDOLE (0,9 - 1,1 Gew.-% Aktivsubstanz), Methylparaben (0,12 - 0,15 Gew.-%) | Exsymol |
| Glucamate^{®}DOE 120 | PEG-120 Methyl Glucose Dioleate | AMERCHOL |
| Hibiscin^{®} HP-LS-9198 | Water, Hibiscus esculentus Seed Extract, Phenoxyethanol | Laboratoires Serobiologiques |
| Honeyquat^{®} 50 | Hydroxypropyltrimonium Honey | BROOKS |
| Hydagen CMF | Chitosan Glycolate | Cognis |
| Hydrovance | Bis-N, N'-(2-Hydroxyethyl)harnstoff (ca. 50 Gew.-%), Wasser, Harnstoff, Ammoniumlactat | National Starch |
| Jaguar HP 105 | HYDROXYPROPYL GUAR | Rhodia |
| Keltrol SF | Xanthan Gum | Kelco |
| Keratec Pep | Water, Hydrolyzed Keratin | Croda |
| Kombuchka | AQUA ( WATER), SACCHAROMYCES/ XYLINUM BLACK TEA FERMENT, GLYCERIN, HYDROXYETHYLCELLULOSE | Sederma |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} 22 | Behenyl Alcohol | Cognis |
| LANETTE^{®} O | Cetearyl Alcohol | Cognis |
| Lipobelle Soyaglycone | Alcohol, Polysorbate 80, Soy Isoflavones | Mibelle AG Cosmetics |
| Lipoid S 75-3 | Aqua, Lecithine | Lipoid GmbH |
| Lotus Germ Extract | Water, Butylene Glycol, Nelumbo Nucifera Germ Extract | Maruzen |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| MYRITOL^{®} 312 | Caprylic/Capric Triglyceride | Cognis |
| MYRITOL^{®} PC | PROPYLENE GLYCOL DICAPRYLATE/DICAPRATE | Cognis |
| Natrulon RC 50 DG | WATER, CARNITINE, POLYGLYCERIN-10 | Lonza |
| Neutrol TE | TETRAHYDROXYPROPYL ETHYLENEDIAMINE | BASF |
| Novata AB | Cocoglycerides | Cognis |
| Olea europ. Fol extr. S. sicc. | Olea Europea (Olive) Leaf Extract | Fruitarom |
| Omega-CH-Aktivator | Tripeptide-1 | GfN |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Phenonip^{®} | Phenoxyethanol, Methylparaben, Ethylparaben, Propylparaben, Butylparaben, ca. 28 % Aktivsubstanz | NIPA |
| Photosomes | Aqua, Lecithine, Plancton Extract | AGI Dermatics |
| Phycojuvenine | AQUA (WATER), LAMINARIA DIGITATA EXTRACT (1,5 - 4 Gew.-% Trockensubstanz-Gehalt) | Codif International |
| Phytodermin | SOYBEAN PROTEIN GLYCINE SOJA (Linne) | CLR Chem. Laboratorium Kurt Richter |
| Phytokine | HYDROLYZED SOY PROTEIN | Coletica |
| Plantacare^{®} 818 UP | Coco Glucoside, ca. 50 % Aktivsubstanz | Cognis |
| Plantaren^{®} 1200 | Lauryl Glucoside, ca. 50 % Aktivsubstanz | Cognis |
| Plantaren^{®} 2000 | Decyl Glucoside, ca. 50 % Aktivsubstanz | Cognis |
| Polymer JR 400^{®} | Polyquaternium-10 | UNION CARBIDE |
| Ridulisse C | HYDROLYZED SOY PROTEIN | Silab |
| Sambucus AO | Water Glycerin, Sambucus Nigra Flower Extract | Alpaflor/Centerche m |
| Sensiva^{®} SC 50 | 2-Ethylhexylglycerinether | Schülke & Mayr |
| Sepigel^{®} 305 | Polyacrylamide, C₁₃-C₁₄ Isoparaffin, Laureth-7 | SEPPIC |
| Sepilift DPHP | Dipalmitoyl Hydroxyproline | Seppic |
| Sepivinol R | Wine Extract (Polyphenolreicher Extrakt aus Rotwein) | Seppic |
| Silymarin Phytosome | Silybum Marianum Extract and Phospholipids | Indena SpA |
| Simulgel NS | Hydroxyethyl Acrylate / Sodium Acryloyldimethyl Taurate Copolymer / Squalane / Polysorbate 60 | Seppic |
| Stenol 16/18 | Cetearylalkohol | Cognis |
| Symdiol 68 | 1,2-Octandiol, 1,2-Hexandiol | Symrise |
| SYN^{®}-COLL | Water, Palmitoyl-Lys-Val-Lys | Pentapharm |
| Texapon^{®} NSO | Sodium Laureth Sulfate, Aqua (ca. 28 % Aktivsubstanz) | Cognis |
| Texapon^{®} SB 3 | Aqua, Disodium Laureth Sulfosuccinate, (ca. 40 % Aktivsubstanz), Citric Acid | Cognis |
| Tioveil^{®}-AQ-N | Cl 77891 (Titanium Dioxide),Alumina, Silica, Sodium Polyacrylate | Uniqema (Tioxide Specialties) |
| Tween^{®}-80 | Polysorbate-80 | |
| Ultrahold 8 | ACRYLATES/t-BUTYLACRYLAMIDE COPOLYMER | BASF |
| Ultrasomes | Aqua, Lecithine, Micrococcus Luteus Extract | AGI Dermatics |
| Uvinul MS 40 | BENZOPHENONE-4 | BASF |
| Vegetal Filling Spheres | CAPRYLIC/CAPRIC TRIGLYCERIDE, SILICA DIMETHYL SILYLATE, HYDROLYZED WHEAT PROTEIN, BUTYLENE GLYCOL, PHENOXYETHANOL, METHYL-PARABEN, ETHYLPARABEN, PROPYL-PARABEN, BUTYLPARABEN, ISOBUTYL-PARABEN | Coletica |

## Patentansprüche

1. Verwendung mindestens eines 2-Mercapto-N^{α}N^{α}N^{α}-trialkyl-L-histidinium-Betains der Formel (I), 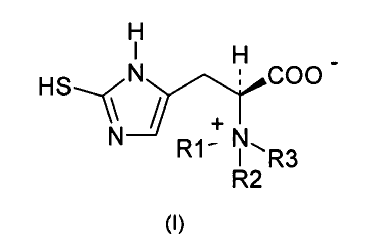 in der R1, R2 und R3 unabhängig voneinander ausgewählt sind aus -CH₃; -CH₂CH₃, -CH₂CH₂CH₃, -CH(CH₃)₂, -(CH₂)₃CH₃, -CH₂-CH(CH₃)₂, -CH(CH₃)CH₂CH₃, -C(CH₃)₃, wobei bevorzugt 2-Mercapto-N^{α}N^{α}N^{α}-trimethyl-L-histidinium-Betain der Formel (la) 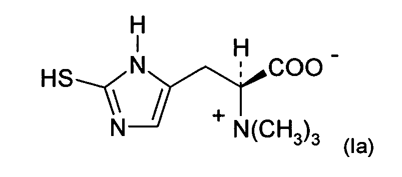 verwendet wird,
und/oder mindestens eines Extraktes aus Laminaria Digitata
zur Regulierung und/oder Verbesserung des Wasserhaushalts der Haut.

2. Verwendung mindestens eines 2-Mercapto-N^{α}N^{α}N^{α}-trialkyl-L-histidinium-Betains der Formel (I), 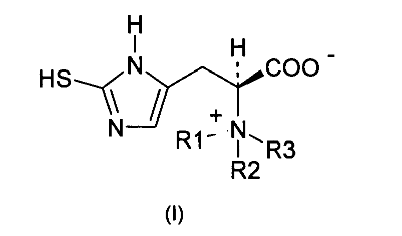 in der R1, R2 und R3 unabhängig voneinander ausgewählt sind aus -CH₃; -CH₂CH₃, -CH₂CH₂CH₃, -CH(CH₃)₂, -(CH₂)₃CH₃, -CH₂-CH(CH₃)₂, -CH(CH₃)CH₂CH₃, -C(CH₃)₃,
und/oder mindestens eines Extraktes aus Laminaria Digitata zur Stimulierung der Aquaporinsynthese.

3. Verwendung von 2-Mercapto-N^{α}N^{α}N^{α}-trialkyl-L-histidinium-Betainen der Formel (I), 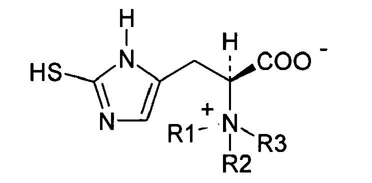 in der R1, R2 und R3 unabhängig voneinander ausgewählt sind aus -CH₃; -CH₂CH₃, -CH₂CH₂CH₃, -CH(CH₃)₂, wobei bevorzugt 2-Mercapto-N^{α}N^{α}N^{α}-trimethyl-L-histidinium-Betain der Formel (la) 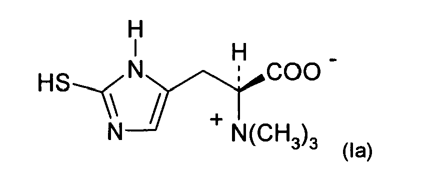 verwendet wird,
zur Stimulierung der Aquaporinsynthese.

4. Verwendung von mindestens einem Laminarin der Formel (II), 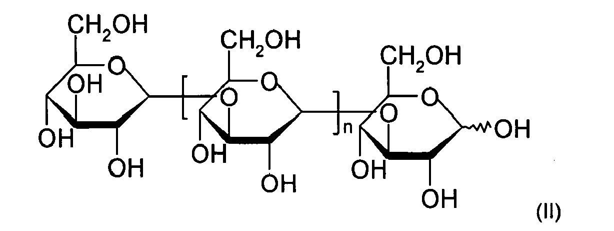 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
zur Regulierung und/oder Verbesserung des Wasserhaushalts der Haut.

5. Verwendung von mindestens einem Laminarin der Formel (II), 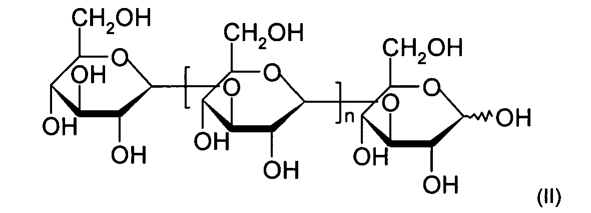 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
zur Stimulierung der Aquaporinsynthese.

6. Verwendung gemäß einem der Ansprüche 1 - 5 zur nicht-therapeutischen, kosmetischen Behandlung trockener Haut.

7. Verwendung gemäß einem der Ansprüche 1 - 6, **dadurch gekennzeichnet, dass** das mindestens eine 2-Mercapto-N^{α}N^{α}N^{α}-trialkyl-L-histidinium-Betain der Formel (I), 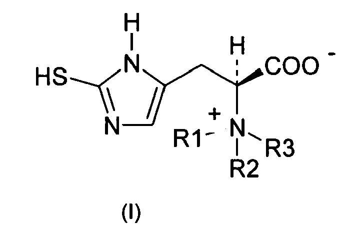 in der R1, R2 und R3 unabhängig voneinander ausgewählt sind aus -CH₃; -CH₂CH₃, -CH₂CH₂CH₃, -CH(CH₃)₂, -(CH₂)₃CH₃, -CH₂-CH(CH₃)₂, -CH(CH₃)CH₂CH₃, -C(CH₃)₃, bevorzugt 2-Mercapto-N^{α}N^{α}N^{α}-trimethyl-L-histidinium-Betain der Formel (la), 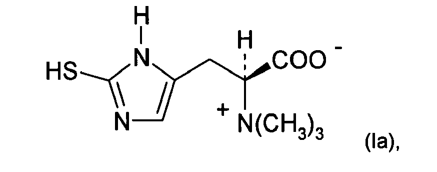 und/oder der mindestens eine Extrakt aus Laminaria Digitata
jeweils in einer Gesamtmenge von 0,001 bis 10 Gew.-%, bezogen auf das Gewicht der gesamten Zusammensetzung, in einem geeigneten kosmetischen oder dermatologischen Träger enthalten ist/sind.

8. Verwendung gemäß einem der Ansprüche 1 - 7, **dadurch gekennzeichnet, dass** das mindestens eine Laminarin der Formel (II), 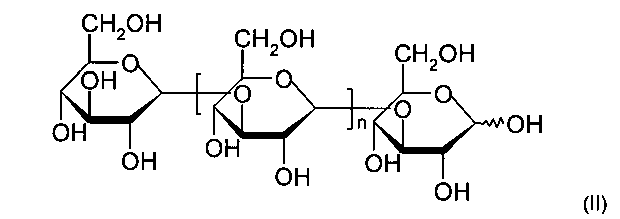 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
in einer Gesamtmenge von 0,00005 - 0,5 Gew.-%, vorzugsweise 0,0005 - 0,25 Gew.-% und insbesondere 0,005 - 0,1 Gew.-%, jeweils bezogen auf das Gewicht der gesamten Zusammensetzung, in einem geeigneten kosmetischen oder dermatologischen Träger enthalten ist.

9. Nicht-therapeutisches Verfahren zur Regulierung und/oder zur Verbesserung des Wasserhaushalts der Haut und/oder zur Stimulierung der Aquaporinsynthese, **dadurch gekennzeichnet, dass** mindestens ein 2-Mercapto-N^{α}N^{α}N^{α}-trialkyl-L-histidinium-Betain der Formel (I), 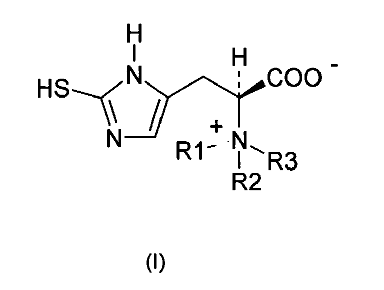 in der R1, R2 und R3 unabhängig voneinander ausgewählt sind aus -CH₃; -CH₂CH₃, -CH₂CH₂CH₃, -CH(CH₃)₂, -(CH₂)₃CH₃, -CH₂-CH(CH₃)₂, -CH(CH₃)CH₂CH₃, -C(CH₃)₃, bevorzugt 2-Mercapto-N^{α}N^{α}N^{α}-trimethyl-L-histidinium-Betain der Formel (la), 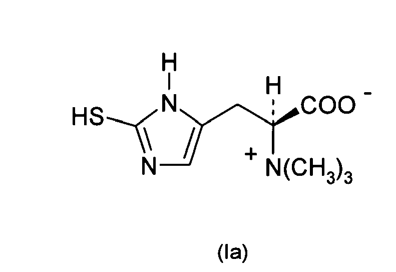 auf die Haut aufgetragen wird.

10. Nicht-therapeutisches Verfahren zur Regulierung und/oder zur Verbesserung des Wasserhaushalts der Haut und/oder zur Stimulierung der Aquaporinsynthese, **dadurch gekennzeichnet, dass** mindestens ein Extrakt aus Laminaria digitata auf die Haut aufgetragen wird.

11. Nicht-therapeutisches Verfahren zur Regulierung und/oder zur Verbesserung des Wasserhaushalts der Haut und/oder zur Stimulierung der Aquaporinsynthese, **dadurch gekennzeichnet, dass** mindestens ein Laminarin der Formel (II), 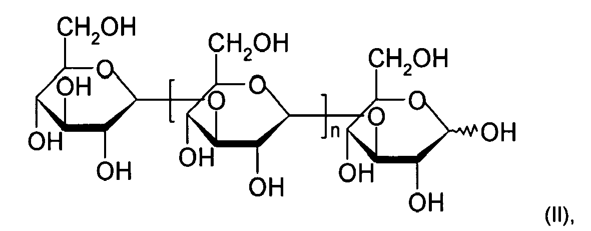 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
auf die Haut aufgetragen wird.

12. Nicht-therapeutisches Verfahren gemäß einem der Ansprüche 9, 10 oder 11 zur nicht-therapeutischen, kosmetischen Behandlung trockener Haut.

13. Nicht-therapeutisches Verfahren gemäß einem der Ansprüche 9, 10, 11 oder 12, **dadurch gekennzeichnet, dass** das mindestens eine 2-Mercapto-N^{α}N^{α}N^{α}-trialkyl-L-histidinium-Betain der Formel (I), 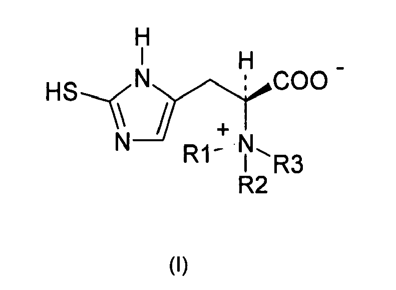 in der R1, R2 und R3 unabhängig voneinander ausgewählt sind aus -CH₃; -CH₂CH₃, -CH₂CH₂CH₃, -CH(CH₃)₂, -(CH₂)₃CH₃, -CH₂-CH(CH₃)₂, -CH(CH₃)CH₂CH₃, -C(CH₃)₃, bevorzugt 2-Mercapto-N^{α}N^{α}N^{α}-trimethyl-L-histidinium-Betain der Formel (la), 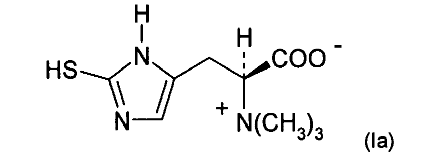 und/oder der mindestens eine Extrakt aus Laminaria Digitata
jeweils in einer Gesamtmenge von 0,001 bis 10 Gew.-%, bezogen auf das Gewicht der gesamten Zusammensetzung, in einem geeigneten kosmetischen oder dermatologischen Träger enthalten ist/sind.

14. Nicht-therapeutisches Verfahren gemäß einem der Ansprüche 9, 10, 11, 12 oder 13, **dadurch gekennzeichnet, dass** das mindestens eine Laminarin der Formel (II), 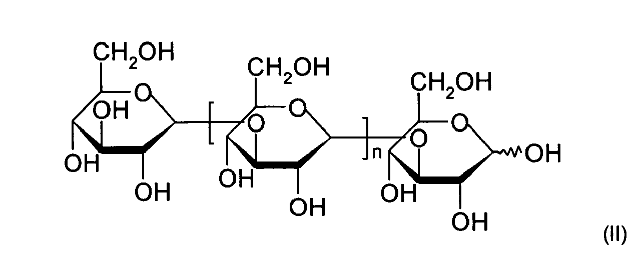 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind,
in einer Gesamtmenge von 00005 bis 0,5 Gew.-%, vorzugsweise 0,0005 bis 0,25 Gew.-% und insbesondere 0,005 bis 0,1 Gew.-%, jeweils bezogen auf das Gewicht der gesamten Zusammensetzung, in einem geeigneten kosmetischen oder dermatologischen Träger enthalten ist.

15. Kosmetische oder dermatologische topische Zusammensetzungen, enthaltend in einem geeigneten kosmetischen oder dermatologischen Träger - jeweils bezogen auf das Gewicht der gesamten Zusammensetzung - eine Kombination der Komponenten a) und b) enthält, wobei a) und b) folgendermaßen definiert sind:
a)
i) 0,001 bis 10 Gew.-% mindestens eines Extraktes aus Laminaria Digitata und/oder
ii) 0,00005 bis 0,5 Gew.-%, vorzugsweise 0,0005 bis 0,25 Gew.-% und insbesondere 0,005 bis 0,1 Gew.-% Laminarin der Formel (II) 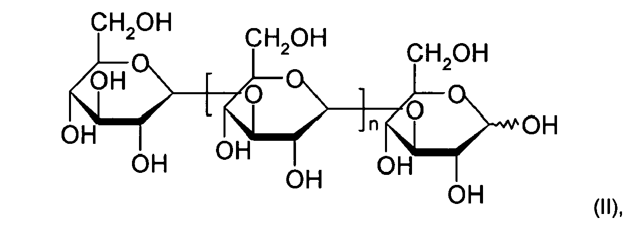
in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind;
b) 0,001 bis 10 Gew.-% mindestens eines 2-Mercapto-N^{α}N^{α}N^{α}-trialkyl-L-histidinium-Betains der Formel (I) 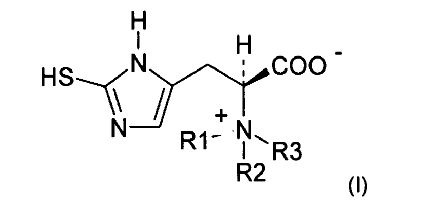 in der R1, R2 und R3 unabhängig voneinander ausgewählt sind aus -CH₃; -CH₂CH₃, - CH₂CH₂CH₃, -CH(CH₃)₂, -(CH₂)₃CH₃, -CH₂-CH(CH₃)₂, -CH(CH₃)CH₂CH₃, -C(CH₃)₃.

16. Zusammensetzung nach Anspruch 15, **dadurch gekennzeichnet, dass** sie 0,001 bis 10 Gew.-%, vorzugsweise 0,01 bis 5 Gew.-% und insbesondere 0,1 bis 2 Gew.-% mindestens eines 2-Mercapto-N^{α}N^{α}N^{α}-trialkyl-L-histidinium-Betains der Formel (I) enthält, 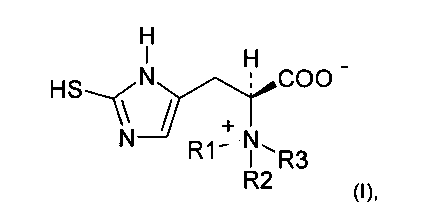 in der R1, R2 und R3 unabhängig voneinander ausgewählt sind aus -CH₃; -CH₂CH₃, -CH₂CH₂CH₃, -CH(CH₃)₂, wobei bevorzugte Zusammensetzungen 2-Mercapto-N^{α}N^{α}N^{α}-trimethyl-L-histidinium-Betain der Formel (la) 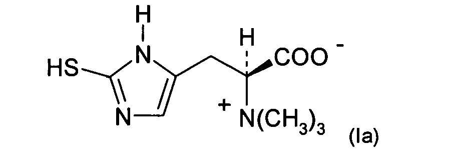 enthalten.

17. Zusammensetzung nach einem der Ansprüche 15 oder 16, **dadurch gekennzeichnet, dass** sie weiterhin mindestens einen Wirkstoff enthalten, der ausgewählt ist aus:
a) Monomeren, Oligomeren und Polymeren von Aminosäuren, N-C₂-C₂₄-Acylaminosäuren, den Estern und/oder den physiologisch verträglichen Metallsalzen dieser Substanzen,
b) DNA- oder RNA-Oligonucleotiden,
c) natürlichen Betainverbindungen,
d) Vitaminen, Provitaminen und Vitaminvorstufen der Gruppen A, B, C, E, H und K und den Estern der vorgenannten Substanzen,
e) α-Hydroxycarbonsäuren, α-Ketocarbonsäuren, β-Hydroxycarbonsäuren und deren Ester-, Lacton- oder Salzform,
f) den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
g) Isoflavonoiden und Isoflavonoid-reichen Pflanzenextrakten,
h) Ubichinon und Ubichinol sowie deren Derivaten,
i) Silymarin,
j) natürlich vorkommenden Xanthin-Derivaten, ausgewählt aus Coffein, Theophyllin, Theobromin und Aminophyllin,
k) Ectoin,
l) Kreatin,
m) Olivenblattextrakten, Ursolsäure, Oleanol und/oder Oleanolsäure,
n) Mono- und Polyhydroxystilbenen und deren Estern,
o) Derivaten von methyliertem Silanol,
p) Phytinsäure,
q) Extrakten aus Maiskörnern (Zea Mays (Corn) Kernel Extract),
r) Extrakten aus Haferkörnern (Avena Sativa (Oat) Kernel Extract),
s) Saccharomyces/Xylinum/Black Tea Ferment,
t) Apfelkernextrakten (Pyrus Malus (Apple) Fruit Extract),
u) Lotuskeim-Extrakten (Nelumbo Nucifera Germ Extract),
v) Rotweinextrakten,
w) Traubenkernextrakten (Vitis Vinifera (Grape) Seed Extract), die bevorzugt aus der Chardonnay-Traube stammen,
x) Extrakten aus Schwarzen Holunderblüten (Sambucus Nigra Flower Extract),
y) Wirkstoffen, die die beta-Endorphinsynthese in Keratinozyten stimulieren,
z) anorganischen und organischen UV-Filtersubstanzen,
aa) selbstbräunenden Wirkstoffen,
bb) hautaufhellenden Wirkstoffen,
cc) Wirkstoffen, die die Prostaglandinsynthese und/oder die Leukotrien-Synthese inhibieren,
dd) sebumregulierenden Wirkstoffen,
ee) sowie Desoxyzuckern oder Desoxyzucker-Bausteine enthaltenden Polysacchariden.

18. Zusammensetzung nach einem der Ansprüche 15 bis 17, **dadurch gekennzeichnet, dass** sie in Form einer Emulsion, vorzugsweise in Form einer lamellaren Emulsion, vorliegt.
